# EUROPEAN PATENT SPECIFICATION

(11) **EP 2 379 586 B1**
(45) Date of publication and mention of the grant of the patent: **02.11.2016**
(21) Application number: 09796016.5
(22) Date of filing: 21.12.2009
(51) Int. Cl.: C07K 14/535, C12N 15/861, A61K 35/76

(54) **ONCOLYTIC ADENOVIRAL VECTORS AND METHODS AND USES RELATED THERETO**
ONCOLYTISCHE ADENOVIRALE VEKTOREN UND VERFAHREN UND VERWENDUNGEN
VECTEURS ADÉNOVIRAUX ONCOLYTIQUES, LEURS PROCÉDÉS ET LEURS UTILISATIONS

(30) Priority: 22.12.2008 FI 20080671; 27.04.2009 FI 20095466; 29.09.2009 US 585971
(43) Date of publication of application: 26.10.2011
(73) Proprietor: Targovax Oy, 00180 Helsinki (FI)
(72) Inventor: HEMMINKI, Akseli, FI-00260 Helsinki (FI); KANERVA, Anna, FI-00260 Helsinki (FI); CERULLO, Vincenzo, FI-00330 Helsinki (FI); PESONEN, Sari, FI-00570 Helsinki (FI)
(74) Representative: Boco IP Oy Ab
(86) International application number: PCT/FI2009/051025
(87) International publication number: WO 2010/072900

(56) References cited:
- WO-A1-2005/030261
- WO-A1-2007/068772
- WO-A2-2007/103825
- LUO JINGJING ET AL: "Treatment of cancer with a novel dual-targeted conditionally replicative adenovirus armed with mda-7/IL-24 gene." CLINICAL CANCER RESEARCH : AN OFFICIAL JOURNAL OF THE AMERICAN ASSOCIATION FOR CANCER RESEARCH, vol. 14, no. 8, 15 April 2008 (2008-04-15) , pages 2450-2457, XP002571566 ISSN: 1078-0432
- LEI N ET AL: "An oncolytic adenovirus expressing granulocyte macrophage colony-stimulating factor shows improved specificity and efficacy for treating human solid tumors." CANCER GENE THERAPY, vol. 16, no. 1, 1 August 2008 (2008-08-01) , pages 33-43, XP002571569 ISSN: 1476-5500
- RAKI M ET AL: "Utility of TK/GCV in the context of highly effective oncolysis mediated by a serotype 3 receptor targeted oncolytic adenovirus." GENE THERAPY OCT 2007, vol. 14, no. 19, October 2007 (2007-10), pages 1380-1388, XP002571570 ISSN: 0969-7128
- ZHOU JIANFENG ET AL: "Novel oncolytic adenovirus selectively targets tumor-associated polo-like kinase 1 and tumor cell viability." CLINICAL CANCER RESEARCH : AN OFFICIAL JOURNAL OF THE AMERICAN ASSOCIATION FOR CANCER RESEARCH, vol. 11, no. 23, 1 December 2005 (2005-12-01), pages 8431-8440, XP002571571 ISSN: 1078-0432
- BRISTOL J ANDREW ET AL: "In vitro and in vivo activities of an oncolytic adenoviral vector designed to express GM-CSF" MOLECULAR THERAPY, ACADEMIC PRESS, SAN DIEGO, CA, US, vol. 7, no. 6, 1 June 2003 (2003-06-01), pages 755-764, XP009091891 ISSN: 1525-0016
- BAUERSCHMITZ GERD J ET AL: "Tissue-specific promoters active in CD44+CD24-/low breast cancer cells." CANCER RESEARCH, vol. 68, no. 14, 15 July 2008 (2008-07-15) , pages 5533-5539, XP002571568 ISSN: 1538-7445
- CHOI K-J ET AL: "Concurrent delivery of GM-CSF and B7-1 using an oncolytic adenovirus elicits potent antitumor effect." GENE THERAPY, vol. 13, no. 13, July 2006 (2006-07), pages 1010-1020, XP002571567 ISSN: 0969-7128

## Description

### Field of the invention

The present invention relates to the fields of life sciences and medicine. Specifically, the invention relates to cancer therapies. More specifically, the present invention relates to oncolytic adenoviral vectors and cells and pharmaceutical compositions comprising said vectors. The present description also relates to a use of said vectors in the manufacture of a medicament for treating cancer in a subject and a method of treating cancer in a subject. Furthermore, the present description relates to methods of producing GM-CSF in a cell and increasing tumor specific immune response in a subject, as well as uses of the oncolytic adenoviral vector of the invention for producing GM-CSF in a cell and increasing tumor specific immune response in a subject.

### Background of the invention

Cancer can be treated with surgery, hormonal therapies, chemotherapies and/or radiotherapies but in many cases, cancers, which are often characterized by an advanced stage, cannot be cured with present therapeutics. Therefore, novel cancer cell targeted approaches such as gene therapies are needed.

During the last twenty years gene transfer technology has been under intensive examination. The aim of cancer gene therapies is to introduce a therapeutic gene into a tumor cell. These therapeutic genes introduced to a target cell may for example correct mutated genes, suppress active oncogenes or generate additional properties to the cell. Suitable exogenous therapeutic genes include but are not limited to immunotherapeutic, anti-angiogenic, chemoprotective and "suicide" genes, and they can be introduced to a cell by utilizing modified virus vectors or non-viral methods including electroporation, gene gun and lipid or polymer coatings.

Requirements of optimal viral vectors include an efficient capability to find specific target cells and express the viral genome in the target cells. Furthermore, optimal vectors have to stay active in the target tissues or cells. All these properties of viral vectors have been developed during the last decades and for example retroviral, adenoviral and adeno-associated viral vectors have been widely studied in biomedicine.

To further improve tumor penetration and local amplification of the anti-tumor effect, selectively oncolytic agents, e.g. conditionally replicating adenoviruses, have been constructed. Oncolytic adenoviruses are a promising tool for treatment of cancers. Tumor cells are killed by oncolytic adenoviruses due to a replication of the virus in a tumor cell, the last phase of the replication resulting in a release of thousands of virions into the surrounding tumor tissues for effective tumor penetration and vascular re-infection. Tumor cells allow replication of the virus while normal cells are spared due to engineered changes in the virus genome, which prevent replication in non-tumor cells.

In addition to replication mediated cell killing, oncolytic adenoviruses can also be armed with different therapeutic transgenes. This approach combines the advantages of conventional gene delivery with the potency of replication competent agents. One goal of arming viruses is induction of an immune reaction towards the cells that allow virus replication. Virus replication alone, although immunogenic, is normally not enough to induce effective anti-tumor immunity. To strengthen induction of therapeutic immunity, viruses can be armed with stimulatory proteins such as cytokines for facilitation of the introduction of tumor antigens to antigen presenting cells such as dendritic cells, and their stimulation and/or maturation. Introduction of immunotherapeutic genes into tumor cells and furthermore, translation of the proteins, leads to activation of the immune response and efficient destruction of tumor cells. The most relevant immune cells in this regard are natural killer cells (NK) and cytotoxic CD8+ T-cells.

Adenoviruses are medium-sized (90-100nm), nonenveloped icosahedral viruses, which have double stranded linear DNA of about 36 kilo base pairs in a protein capsid. The viral capsid has fiber structures, which participate in attachment of the virus to the target cell. First, the knob domain of the fiber protein binds to the receptor of the target cell (e.g. CD46 or cox-sackievirus adenovirus receptor (CAR)), secondly, the virus interacts with an integrin molecule and thirdly, the virus is endocytosed into the target cell. Next, the viral genome is transported from endosomes into the nucleus and the replication machinery of the target cell is utilized also for viral purposes (Russell W.C. 2000, J General Virol 81, 2573-2604).

The adenoviral genome has early (E1-E4), intermediate (IX and IVa2) and late genes (L1-L5), which are transcribed in sequential order. Early gene products affect defense mechanisms, cell cycle and cellular metabolism of the host cell. Intermediate and late genes encode structural viral proteins for production of new virions (Wu and Nemerow, 2004, Trends Microbiol 12: 162-168; Russell W.C. 2000, J General Virol 81, 2573-2604; Volpers C. and Kochanek S. 2004, J Gene Med 6 suppl 1, S164-71; Kootstra N.A. and Verma I.M. 2003, Annu Rev Pharmacol Toxicol 43, 413-439).

More than 50 different serotypes of adenoviruses have been found in humans. Serotypes are classified into six subgroups A-F and different serotypes are known to be associated with different conditions i.e. respiratory diseases, conjunctivitis and gastroenteritis. Adenovirus serotype 5 (Ad5) is known to cause respiratory diseases and it is the most common serotype studied in the field of gene therapy. In the first Ad5 vectors E1 and/or E3 regions were deleted enabling insertion of foreign DNA to the vectors (Danthinne and Imperiale 2000). Furthermore, deletions of other regions as well as further mutations have provided extra properties to viral vectors. Indeed, various modifications of adenoviruses have been suggested for achieving efficient anti-tumor effects.

For example, patent EP1377671 B1 (Cell Genesys, Inc.) and application US2003/0104625 A1 (Cheng C. et al.) describe an oncolytic adenoviral vector encoding an immunotherapeutic protein granulocyte-macrophage colony-stimulating factor (GM-CSF). Also, publication EP1767642 A1 (Chengdu Kanghong Biotechnologies Co., Ltd.) points out oncolytic adenoviral vectors having improved effects on human immune response. Further, Luo Jingjing et al. (2008) discloses an oncolytic adenoviral vector comprising an Ad5 nucleic acid backbone, a 24 bp deletion (D24) in the RB binding constant region 2 of E1 and an antitumor transgene in place of the deleted gp19k/6.7k in the E3 region.

Still, more efficient and accurate gene transfer as well as increased specificity and sufficient tumor killing ability of gene therapies are warranted. Safety records of therapeutic vectors must also be excellent. The present invention provides a cancer therapeutic tool with these aforementioned properties by utilizing both oncolytic and immunotherapeutic properties of adenoviruses in a novel and inventive way.

### Brief description of the invention

The object of the invention is to provide novel methods and means for achieving the above-mentioned properties of adenoviruses and thus, solving the problems of conventional cancer therapies. More specifically, the invention provides novel methods and means for gene therapy.

The present application describes construction of recombinant viral vectors, methods related to the vectors, and their use in tumor cells lines, animal models and cancer patients.

The present invention relates to an oncolytic adenoviral vector comprising an adenovirus serotype 5 (Ad5) nucleic acid backbone, a 24 bp deletion (D24) in the Rb binding constant region 2 of adenoviral E1 and a nucleic acid sequence encoding a granulocyte-macrophage colony-stimulating factor (GM-CSF) in the place of the deleted gp19k/6.7K in the adenoviral E3 region.

The present invention further relates to a cell comprising the adenoviral vector of the invention.

The present invention also relates to a pharmaceutical composition comprising the adenoviral vector of the invention.

The present invention also relates to a use of the adenoviral vector of the invention in the manufacture of a medicament for treating cancer in a subject.

The present description also relates to a method of treating cancer in a subject, wherein the method comprises administration of the vector or pharmaceutical composition of the invention to a subject.

Furthermore, the present invention also relates to a method of producing GM-CSF in a cell, wherein the method comprises:
a) carrying a vehicle comprising an oncolytic adenoviral vector of the invention to a cell, and
b) expressing GM-CSF of said vector in the cell.

Furthermore, the present description also relates to a method of increasing tumor specific immune response in a subject, wherein the method comprises:
a) carrying a vehicle comprising an oncolytic adenoviral vector of the invention to a target cell or tissue,
b) expressing GM-CSF of said vector in the cell, and
c) increasing amount of cytotoxic T cells and/or natural killer cells in said target cell or tissue.

Still, the present description also relates to a use of the oncolytic adenoviral vector of the invention for producing GM-CSF in a cell.

Still, the present description relates to the oncolytic adenoviral vector of the description for producing GM-CSF in a cell.

Still, the present description also relates to a use of the oncolytic adenoviral vector of the invention for increasing tumor specific immune response in a subject.

Still, the present description relates to the oncolytic adenoviral vector of the invention for increasing tumor specific immune response in a subject.

The present invention provides a tool for treatment of cancers, which are refractory to current approaches. Also, restrictions regarding tumor types suitable for treatment remain few compared to many other treatments. In fact all solid tumors may be treated with the proposed description. Larger tumors by mass and more complex tumors can be cured by the presentdescription. The treatment can be given intratumorally, intracavitary, intravenously and in a combination of these. The approach can give systemic efficacy despite local injection. The approach can also eradicate cells proposed as tumor initiating ("cancer stem cells").

Besides enabling the transport of the vector to the site of interest the vector of the description also assures the expression and persistence of the transgene. Furthermore, immune response against the vector as well as the transgene is minimized.

The present invention solves a problem related to therapeutic resistance of conventional treatments. Furthermore, the present invention provides tools and methods for selective treatments, without toxicity or damages in healthy tissues. Advantages of the present invention include also different and reduced side effects in comparison to other therapeutics. Importantly, the approach is synergistic with many other forms of therapy including chemotherapy and radiation therapy, and can therefore be used in combination regimens.

Induction of an immune reaction towards cells that allow replication of unarmed viruses is normally not strong enough to lead to development of therapeutic tumor immunity. In order to overcome this weakness, the present invention provides armed viruses with a potent inducer of anti-tumor immunity. The present description discloses cancer therapy, wherein tumor cells are destroyed by virion caused oncolysis. In addition, various different mechanisms activating human immune response, including activation of natural killer cells (NK) and dendritic cells (DC) are influenced.

Compared to adenoviral tools of the prior art, the present invention provides a more simple, more effective, inexpensive, non-toxic and/or safer tool for cancer therapy. Furthermore, helper viruses are not needed.

The novel products of the invention enable further improvements in cancer therapy.

### Brief description of the figures

Figure 1 shows a schematic of pAd5-D24-GMCSF. The virus bears a 24 base pair deletion in the constant region 2 of E1 A. gp19k and 6.7K in E3 have been replaced with the cDNA of human GM-CSF. ADP refers to the adenovirus death protein.
Figure 2 shows a schematic of the first step of cloning to generate shuttle plasmid (pTHSN) bearing GM-CSF.
Figure 3 shows a schematic of the second step of cloning to generate the plasmid containing all the adenoviral genes with the 24 base pair deletion in E1 region (which mediates selective replication in cancer cells).
Figure 4 shows a schematic of the third step of cloning to generate the plasmid containing all the adenoviral genes except for gp19k and 6.7K which have been replaced by GM-CSF (pAd5D24.GM-CSF (SEQ ID NO 8)). Ad5-RGD-D24-GMCSF (SEQ ID NO 9), Ad5/3-D24-GMCSF (SEQ ID NO 7) and Ad5-pK7-D24-GMCSF (SEQ ID NO 10) were created similarly.

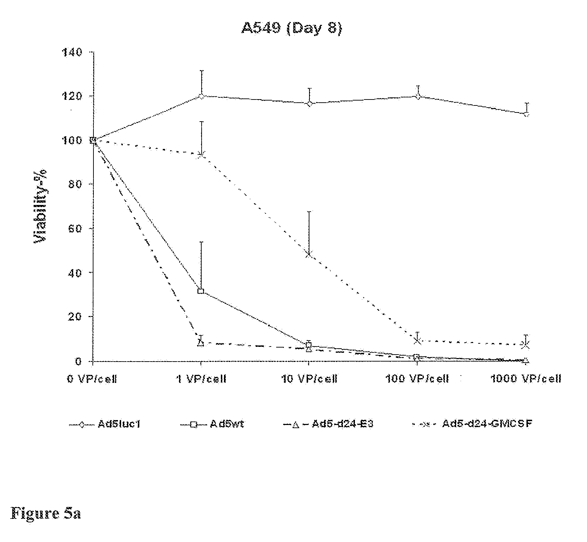

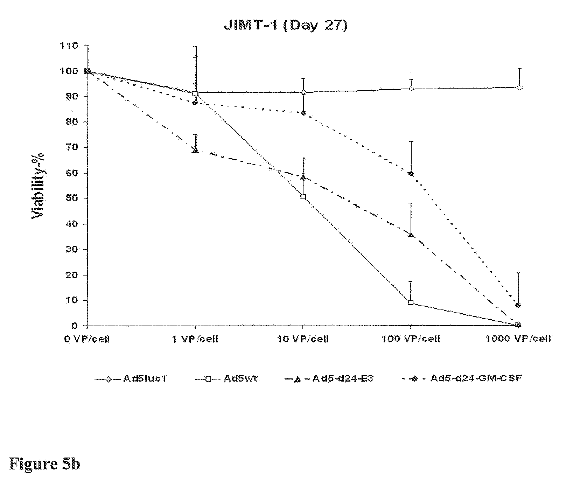

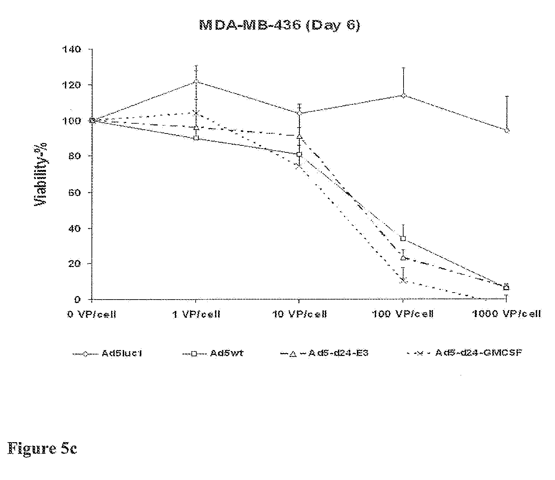

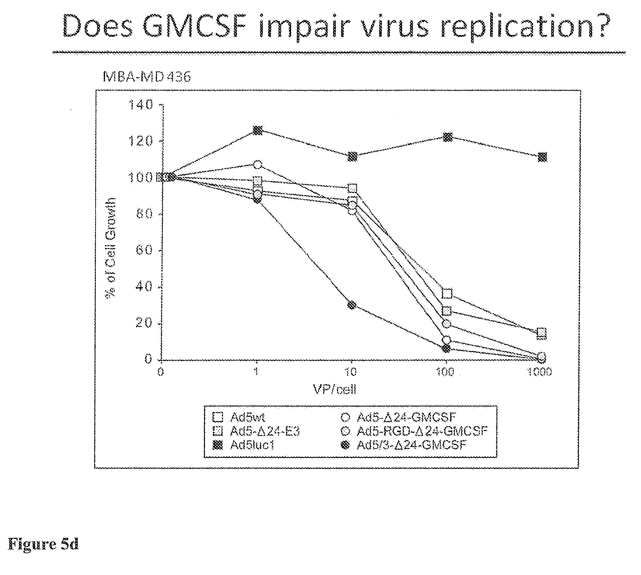

Figures 5a-d show that GMCSF expression does not impair virus replication and cell killing effect. Figure 5a represents results of MTS assay showing lung cancer derived (A549) cell killing efficiency of the new generated virus Ad5-D24-GMCSF. Figure 5b represents results of MTS assay showing killing of JIMT-1 cancer initiating cells ("cancer stem cells") with Ad5-D24-GMCSF. Figure 5c represents results of MTS assay showing breast cancer cells (MDA-MB-436) killing efficiency of the new generated virus Ad5-D24-GMCSF. Figure 5d represents results of MTS assay showing MDA-MB-436 killing efficiency of the new generated viruses Ad5-D24-GMCSF, Ad5-RGD-D24-GMCSF and Ad5/3-D24-GMCSF.
Figure 6a shows adenovirus-coupled expression of human GMCSF. A549 cell line was infected with Ad5D24 or Ad5D24-GMCSF, media was collected over time and analyzed for expression of GMCSF by FACSARRAY.
Figure 6b shows that adenovirus-expressed GMCSF retains its biological activity in human lymphocytes. TF1 cells, which require human GMCSF for staying alive, were cultured in the presence of human recombinant GMCSF (E. coli-produced, purchased from Sigma) or supernatant from Ad5-D24-GMCSF infected cells.
Figures 7a and 7b show *in vitro* analysis of transduction of a patient tumor to test the infectivity of an Ad5-based virus.
Figure 7c shows a prediction of tumor transduction with Ad5-D25-GMCSF by staining its receptor CAR (coxsackie-adenovirus receptor) in archival tumor specimens.
Figure 8a shows *in vivo* efficiency of Ad5-D24-GMCSF in Syrian Hamsters (permissive for human adenovirus replication) bearing pancreatic cancer tumors. Both Ad5D24 and Ad5-D24-GMCSF eradicate the tumors within 16 days following the treatments. 1x10⁹ VP of virus were administered on days 0, 2 and 4.
Figure 8b shows that intratumoral injection of Ad5-D24-GMCSF resulted in high levels of hGMCSF in serum of Syrian Hamsters. Animals treated with Ad5D24E3 or Ad5-D24-GMCSF were sampled on day 4 and the concentration of human GMCSF in serum was assessed by FACSARRAY.
Figure 8c shows that curing of HapT1 tumors with Ad5-D24-GMCSF (but not with Ad5D24) protected Syrian hamsters from subsequent rechallenge with HapT1. This demonstrates that Ad5-D24-GMCSF can induce a tumor specific immune response. Animals previously treated with Ad5D24 or Ad5D24-GMCSF (Figure 8a) were re-challenged with the same tumor and tumor growth was measured over time.
Figure 8d shows induction of a tumor specific immune response by Ad5-D24-GMCSF, curation of HapT1 tumors with Ad5-D24-GMCSF did not protect Syrian hamsters from Hak tumors. Animals with HapT1 tumors previously treated with Ad5D24, or Ad5D24-GMCSF (Figure 8a) were re-challenged with a different tumor and tumor growth was measured over time.
Figure 9a shows efficacy of Ad5-D24-GMCSF in combination with metronomic oral cyclophosphamide. 88% tumor reduction was observed by CT scan 71 days following the beginning of the treatment.
Figure 9b shows efficacy of the treatment with Ad5D24-GMCSF in an ovarian cancer patient treated with Ad5-D24-GMCSF. CT scan showed complete disappearance of all measurable tumors as indicated by the arrow.
Figures 10a-d show that Ad5-D24-GMCSF elicited a T cell-response against both tumor epitopes and adenovirus (present in tumor cells). T cells harvested from patients treated with Ad5-D24-GMCSF were analyzed by IFN-gamma ELISPOT upon stimulation with a mix of peptide from Adenovirus 5 and a mix of peptide from the tumor antigen survivin.
Figure 11 shows induction of Adenovirus Hexon-specific T cells. Leukocytes harvested from patients treated with Ad5-D24-GMCSF were stained with a CD3, CD8 and hexon-specific tetramer antibodies and analyzed by flow cytometry before and after the treatment. Treatment increased hexon specific cytotoxic T-cells from 0.21 to 2.72%.
Figure 12 shows a reduction of circulating T regulatory cells in patient R73. PBMCs which are positive for CD4, negative for CD127 but high in Foxp3 are considered effective T regulatory cells.
Figures 13a-i show schematics of cloning to generate shuttle plasmid (pTHSN) bearing GM-CSF, to generate the plasmid containing all the adenoviral genes with the 24 base pair deletion in E1 region (which mediates selective replication in cancer cells) and to generate the Ad5/3-D24-GMCSF plasmid containing all the adenoviral genes except for gp19k and 6.7K which have been replaced by GM-CSF (pAd5D24.GM-CSF).
Figure 14 shows a nucleotide sequence of Ad5/3-D24-GMCSF. Bolded region indicates the D24 deleted E1 A region (nucleotides 563-1694). Underlined region indicates GMCSF (nucleotides 28380-28814). Region in italics indicates the Ad3 knob region (nucleotides 31701-32272). The sequence of the figure corresponds to sequence SEQ ID NO 7.

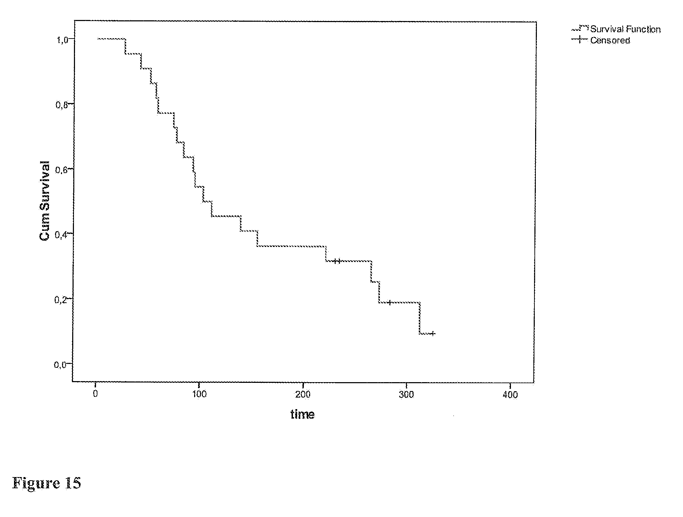

Figure 15 shows survival plot for Ad5/3-D24-GMCSF treated patients.

### Detailed description of the invention

### Adenoviral vector

In Ad5, as well as in other adenoviruses, an icosahedral capsid consists of three major proteins: hexon (II), penton base (III), and a knobbed fiber (IV), along with minor proteins: VI, VIII, IX, IIIa, and IVa2 (Russell W.C. 2000, J General Virol 81, 2573-2604). Proteins VII, small peptide *mu,* and a terminal protein (TP) are associated with DNA. Protein V provides a structural link to the capsid via protein VI. Virus encoded protease is needed for processing some structural proteins.

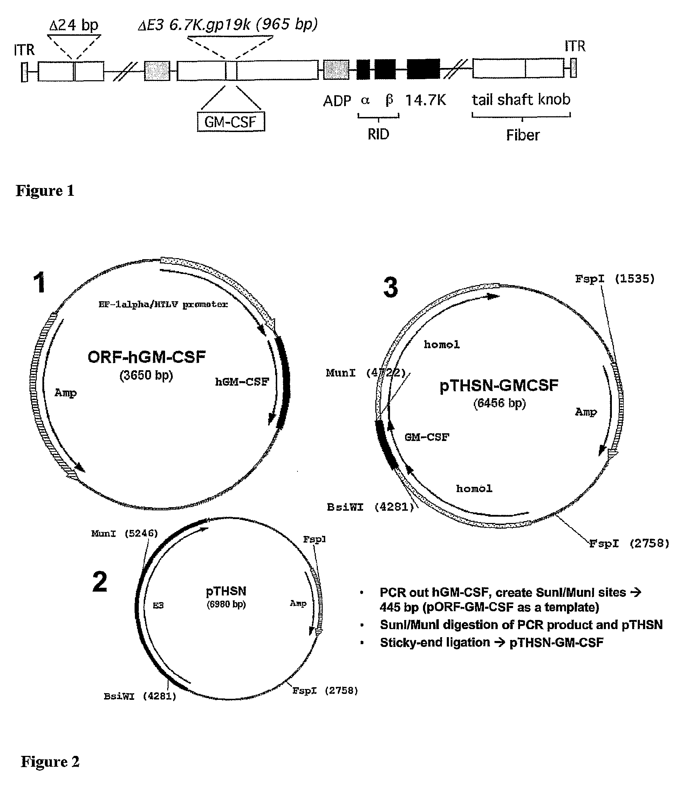

The oncolytic adenoviral vector of the present invention is based on an adenovirus serotype 5 (Ad5) nucleic acid backbone, a 24 bp deletion (D24) in the Rb binding constant region 2 (CR2) of adenoviral E1 and a nucleic acid sequence encoding a granulocyte-macrophage colony-stimulating factor (GM-CSF) in the place of the deleted gp19k/6.7K in the adenoviral E3 region (Figure 1). In a preferred embodiment, the adenoviral vector is based on a human adenovirus.

Ad5 genome contains early (E1-4), intermediate (IX and IVa2) and late (L1-5) genes flanked by left and right inverted terminal repeats (LITR and RITR, respectively), which contain the sequences required for the DNA replication. The genome contains also packaging signal (ψ) and major late promoter (MLP).

Transcription of the early gene E1 A starts the replication cycle followed by expression of E1B, E2A, E2B, E3 and E4. E1 proteins modulate cellular metabolism in a way that makes a cell more susceptible to virus replication. For example they interfere with NF-κB, p53, and pRb-proteins. E1A and E1 B function together in inhibiting apoptosis. E2 (E2A and E2B) and E4 gene products mediate DNA replication and E4 products also effect virus RNA metabolism and prevent host protein synthesis. E3 gene products are responsible for defending against the host immune system, enhancing cell lysis, and releasing of virus progeny (Russell W.C. 2000, J General Virol 81, 2573-2604).

Intermediate genes IX and IVa2 encode minor proteins of the viral capsid. Expression of the late genes L1-5, which lead to production of the virus structural components, the encapsidation and maturation of virus particles in the nucleus, is influenced by MLP (Russell W.C. 2000, J General Virol 81, 2573-2604).

Compared to a wild type adenovirus genome, the adenoviral vector of the invention lacks 24 base pairs from CR2 in E1 region, specifically in E1 A region, and gp19k and 6.7K in E3 region.It is also disclosed that, in addition to partial regions E1 and E3, the oncolytic adenoviral vector can further comprise one or more regions selected from a group consisting of E2, E4, and late regions.The oncolytic adenoviral vector comprising the following regions: a left ITR, partial E1, pIX, pIVa2, E2, VA1, VA2, L1, L2, L3, L4, partial E3, L5, E4, and a right ITR is also disclosed. The regions may be in any order in the vector, but in a preferred embodiment, the regions are in a sequential order in the 5' to 3' direction. Open reading frames (ORFs) may be in the same DNA strand or in different DNA strands. In a preferred embodiment, the E1 region comprises a viral packaging signal.

As used herein, expression "adenovirus serotype 5 (Ad5) nucleic acid backbone" refers to the genome or partial genome of Ad5, which comprises one or several regions selected from a group consisting of partial E1, pIX, pIVa2, E2, VA1, VA2, L1, L2, L3, L4, partial E3, L5 and E4 of Ad5 origin. One preferred vector of the invention comprises nucleic acid backbone of Ad5. In another preferred vector, the adenoviral nucleic acid backbone is mostly derived from Ad5 and combined with a portion (e.g. a part of the capsid structure) of Ad3.

As used herein, expression "partial" region refers to a region, which lacks any part compared to a corresponding wild type region. "Partial E1 refers to E1 region with D24 and "partial E3" refers to E3 region lacking gp19k/6.7K.

As used herein, expressions "VA1" and "VA2" refer to virus associated RNAs 1 and 2, which are transcribed by the adenovirus but are not translated. VA1 and VA2 have a role in combating cellular defence mechanisms.

As used herein, expression "a viral packaging signal" refers to a part of virus DNA, which consists of a series of AT-rich sequences and governs the encapsidation process.

24 base pair deletion (D24) of E1 affects CR2 domain, which is responsible for binding the Rb tumor suppressor/cell cycle regulator protein and thus, allows the induction of the synthesis (S) phase i.e. DNA synthesis or replication phase. pRb and E1 A interaction requires eight amino acids 121 to 127 of the E1 A protein conserved region (Heise C. et al. 2000, Nature Med 6, 1134-1139), which are deleted in the present invention. The vector of the present invention comprises a deletion of nucleotides corresponding to amino acids 122-129 of the vector according to Heise C. et al. (2000, Nature Med 6, 1134-1139). Viruses with the D24 are known to have a reduced ability to overcome the G1-S checkpoint and replicate efficiently only in cells where this interaction is not necessary, e.g. in tumor cells defective in the Rb-p16 pathway (Heise C. et al. 2000, Nature Med 6, 1134-1139; Fueyo J et al. 2000, Oncogene 19, 2-12).

The E3 region is nonessential for viral replication *in vitro,* but the E3 proteins have an important role in the regulation of host immune response i.e. in the inhibition of both innate and specific immune responses. The gp19k/6.7K deletion in E3 refers to a deletion of 965 base pairs from the adenoviral E3A region. In a resulting adenoviral construct, both gp19k and 6.7K genes are deleted (Kanerva A et al. 2005, Gene Therapy 12, 87-94). The gp19k gene product is known to bind and sequester major histocompatibility complex I (MHC1) molecules in the endoplasmic reticulum, and to prevent the recognition of infected cells by cytotoxic T-lymphocytes. Since many tumors are deficient in MHC1, deletion of gp19k increases tumor selectivity of viruses (virus is cleared faster than wild type virus from normal cells but there is no difference in tumor cells). 6.7K proteins are expressed on cellular surfaces and they take part in downregulating TNF-related apoptosis inducing ligand (TRAIL) receptor 2.

In the present invention, the GM-CSF transgene is placed into a gp19k/6.7k deleted E3 region, under the E3 promoter. This restricts transgene expression to tumor cells that allow replication of the virus and subsequent activation of the E3 promoter. E3 promoter may be any exogenous or endogenous promoter known in the art, preferably endogenous promoter. In a preferred embodiment, a nucleic acid sequence encoding GM-CSF is under the control of the viral E3 promoter.

GM-CSF participates in immune response by acting through various mechanisms including recruitment of natural killer (NK) cell and stimulation of antigen presenting cells (APC). APC can then recruit, activate and target T-cells towards the tumor. The nucleotide sequence encoding GM-CSF may be from any animal such as a human, ape, rat, mouse, hamster, dog or cat, but preferably GM-CSF is encoded by a human sequence. The nucleotide sequence encoding GM-CSF may be modified in order to improve the effects of GM-CSF, or unmodified i.e. of a wild type. In a preferred embodiment, a nucleic acid sequence encoding GM-CSF is of a wild type.

The vector as disclosed may also comprise other modifications than partial deletions of CR2 and E3 and insertion of GM-CSF sequence as mentioned above. In a preferred embodiment of the invention, all the other regions of the Ad5 vector are of a wild type. In another preferred embodiment of the invention, the E4 region is of a wild type. In a preferred embodiment of the invention, a wild type region is located upstream of the E1 region. "Upstream" refers to immediately before the E1 region in the direction of expression. E1 B region may also be modified in the vectoras disclosed.

Insertion of exogenous elements may enhance effects of vectors in target cells. The use of exogenous tissue or tumor-specific promoters is common in recombinant adenoviral vectors and they are also described. For example, viral replication can be restricted to target cells for example by promoters, which include but are not limited to CEA, SLP, Cox-2, Midkine, hTERT, variants of hTERT, E2F, variants of E2F, CXCR4, SCCA2 and TTS. They are usually added to control E1 A region, but in addition to or alternatively, other genes such as E1 B or E4 can also be regulated. Exogenous insulators i.e. blocking elements against unspecific enhancers, the left ITR, the native E1A promoter or chromatin proteins may also be included in recombinant adenoviral vectors. Any additional components or modifications may optionally be used but are not obligatory in the vectors as disclosed.

Most adults have been exposed to the most widely used adenovirus serotype Ad5 and therefore, the immune system can rapidly produce neutralizing antibodies (NAb) against them. In fact, the prevalence of anti-Ad5 NAb may be up to 50%. It has been shown that NAb can be induced against most of the multiple immunogenic proteins of the adenoviral capsid, and on the other hand it has been shown that even small changes in the Ad5 fiber knob can allow escape from capsid-specific NAb. Modification of the knob is therefore important for retaining or increasing gene delivery in the contact of adenoviral use in humans.

Furthermore, Ad5 is known to bind to the receptor called CAR via the knob portion of the fiber, and modifications of this knob portion or fiber may improve the entry to the target cell and cause enhanced oncolysis in some cancers (Ranki T. et al. 2007, Int J Cancer 121, 165-174). Indeed, capsid-modified adenoviruses are advantageous tools for improved gene delivery to cancer cells.

In one embodiment of the invention, the oncolytic adenoviral vector comprises a capsid modification. As used herein "capsid" refers to the protein shell of the virus, which includes hexon, fiber and penton base proteins. Any capsid modification i.e. modification of hexon, fibre and/or penton base proteins known in the art, which improves delivery of the virus to the tumor cell, may be utilizedaccording to the present description. Modifications may be genetic and/or physical modifications and include but are not limited to modifications for incorporating ligands, which recognize specific cellular receptors and/or block native receptor binding, for replacing the fiber or knob domain of an adenoviral vector with a knob of other adenovirus (chimerism) and for adding specific molecules (e.g. FGF2) to adenoviruses. Therefore, capsid modifications include but are not limited to incorporation of small peptide motif(s), peptide(s), chimerism(s) or mutation(s) into the fiber (e.g. into the knob, tail or shaft part), hexon and/or penton base. In a preferred embodiment of the invention, the capsid modification is Ad5/3 chimerism, insertion of an integrin binding (RGD) region and/or heparin sulphate binding polylysine modification into the fiber. In a specific embodiment of the invention, the capsid modification is Ad5/3 chimerism.

As used herein, "Ad5/3 chimerism" of the capsid refers to a chimerism, wherein the knob part of the fiber is from Ad serotype 3, and the rest of the fiber is from Ad serotype 5.

As used herein, "RGD region" refers to the arginine-glycine-aspartic acid (RGD) motif, which is exposed on the penton base and interacts with cellular αv integrins supporting adenovirus internalization. In a preferred embodiment of the invention, the capsid modification is a RGD-4C modification. "RGD-4C modification" refers to an insertion of an integrin binding RGD-4C motif in the HI loop of the fiber knob domain. 4C refers to the four cysteins, which form sulphur bridges in RGD-4C. Construction of recombinant Ad5 fiber gene encoding the fiber with the RGD-4C peptide is described in detail for example in the article of Dmitriev I. et al. (1998, Journal of Virology, 72, 9706-9713).

As used herein, "heparan sulphate binding polylysine modification" refers to addition of a stretch of seven lysines to the fiber knob c-terminus.

Expression cassettes are used for expressing transgenes in a target, such as a cell, by utilizing vectors. As used herein, the expression "expression cassette" refers to a DNA vector or a part thereof comprising nucleotide sequences, which encode cDNAs or genes, and nucleotide sequences, which control and/or regulate the expression of said cDNAs or genes. Similar or different expression cassettes may be inserted to one vector or to several different vectors. Ad5 vectors of the present invention may comprise either several or one expression cassettes. However, only one expression cassette is adequate. In a preferred embodiment of the invention, the oncolytic adenoviral vector comprises at least one expression cassette. In a preferred embodiment of the invention, the oncolytic adenoviral vector comprises only one expression cassette.

A cell comprising the adenoviral vector of the invention may be any cell such as a eukaryotic cell, bacterial cell, animal cell, human cell, mouse cell etc. A cell may be an *in vitro, ex vivo* or *in vivo* cell. For example, the cell may be used for producing the adenoviral vector *in vitro, ex vivo* or *in vivo,* or the cell may be a target, such as a tumor cell, which has been infected with the adenoviral vector.

In a method of producing GM-CSF in a cell, a vehicle comprising the vector of the invention is carried into a cell and furthermore, GM-CSF gene is expressed and the protein is translated and secreted in a paracrine manner. "A vehicle" may be any viral vector, plasmid or other tool, such as a particle, which is able to deliver the vector of the invention to a target cell. Any conventional method known in the art can be used for delivering the vector to the cell.

Tumor specific immune response may be increased in a subject according to the present description. Cytotoxic T cells and/or natural killer cells are stimulated, produced and targeted as a consequence of GM-CSF expression. In a preferred embodiment, amount of natural killer and/or cytotoxic T cells is increased in a target cell or tissue. In order to follow or study the effects, various markers of immune response (e.g. inflammatory markers) may be determined. The most common markers include but are not limited to increase in pro-inflammatory cytokines, tumor or adenovirus specific cytotoxic T-cells, recruitment and activation of antigen presenting cells or increase in size of local lymph nodes. The levels of these markers may be studied according to any conventional methods known in the art, including but not limited to those utilizing antibodies, probes, primers etc. such as ELISPOT assay, tetramer analysis, pentamer analysis and analysis of different cell types in blood or in tumors.

### Cancer

The recombinant Ad5 vectors of the invention have been constructed for replication competence in cells, which have defects in the Rb-pathway, specifically Rb-p16 pathway. These defective cells include all tumor cells in animals and humans (Sherr C.J. 1996, Science 274, 1672-1677). In a preferred embodiment of the invention, the vector is capable of selectively replicating in cells having defects in the Rb-pathway. As used herein "defects in the Rb-pathway" refers to mutations and/or epigenetic changes in any genes or proteins of the pathway. Due to these defects, tumor cells overexpress E2F and thus, binding of Rb by E1 A CR2, that is normally needed for effective replication, is unnecessary.

Any cancers or tumors, including both malignant and benign tumors as well as primary tumors and metastasis may be targets of gene therapies. In a specific embodiment the cancer is any solid tumor. In a preferred embodiment of the invention, the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer, tonsil cancer.

### Pharmaceutical composition

A pharmaceutical composition of the invention comprises at least one type of the vectors of the invention. Furthermore, the composition may comprise at least two, three or four different vectors of the invention. In addition to the vector of the invention, a pharmaceutical composition may also comprise any other vectors, such as other adenoviral vectors, other therapeutically effective agents, any other agents such as pharmaceutically acceptable carriers, buffers, excipients, adjuvants, antiseptics, filling, stabilising or thickening agents, and/or any components normally found in corresponding products.

The pharmaceutical composition may be in any form, such as solid, semisolid or liquid form, suitable for administration. A formulation can be selected from a group consisting of, but not limited to, solutions, emulsions, suspensions, tablets, pellets and capsules.

In a preferred embodiment of the invention, the oncolytic adenoviral vector or pharmaceutical composition acts as an *in situ* cancer vaccine. As used herein *"in situ* cancer vaccine" refers to a cancer vaccine, which both kills tumor cells and also increases the immune response against tumor cells. Virus replication is a strong danger signal to the immune system (=needed for a TH1 type response), and thus acts as a powerful costimulatory phenomenon to GM-CSF mediated maturation and activation of APCs, and recruitment of NK cells. Tumor cell lysis also helps to present tumor fragments and epitopes to APCs and furthermore, costimulation is produced by inflammation. Thus, an epitope independent (i.e. not HLA restricted) response is produced in the context of each tumor and therefore takes place *in situ.* Tumor specific immune response is activated in the target cell as well as the surrounding cells, e.g. in the target tissue.

The effective dose of vectors depends on at least the subject in need of the treatment, tumor type, location of the tumor and stage of the tumor. The dose may vary for example from about 10e8 viral particles (VP) to about 10e14 VP, preferably from about 5x10e9 VP to about 10e13 VP and more preferably from about 8x10e9 VP to about 10e12 VP. In one specific embodiment the dose is in the range of about 5x10e10 - 5x10e11 VP.

The pharmaceutical compositions may be produced by any conventional processes known in the art, for example by utilizing any one of the following: batch, fed-batch and perfusion culture modes, column-chromatography purification, CsCl gradient purification and perfusion modes with low-shear cell retention devices.

### Administration

The vector or pharmaceutical composition of the description may be administered to any eukaryotic subject selected from a group consisting of plants, animals and human beings. In a preferred embodiment of the invention, the subject is a human or an animal. An animal may be selected from a group consisting of pets, domestic animals and production animals.

Any conventional method may be used for administration of the vector or composition to a subject. The route of administration depends on the formulation or form of the composition, the disease, location of tumors, the patient, comorbidities and other factors. In a preferred embodiment of the invention, the administration is conducted through an intratumoral, intramuscular, intra-arterial, intravenous, intrapleural, intravesicular, intracavitary or peritoneal injection, or an oral administration.

Only one administration of oncolytic adenoviral vectors of the invention may have therapeutic effects. However, in a preferred embodiment of the invention, oncolytic adenoviral vectors or pharmaceutical compositions are administered several times during the treatment period. Oncolytic adenoviral vectors or pharmaceutical compositions may be administered for example from 1 to 10 times in the first 2 weeks, 4 weeks, monthly or during the treatment period. In one embodiment, administration is done three to seven times in the first 2 weeks, then at 4 weeks and then monthly. In a specific embodiment, administration is done four times in the first 2 weeks, then at 4 weeks and then monthly. The length of the treatment period may vary, and for example may last from two to 12 months or more.

In order to avoid neutralizing antibodies in a subject, the vectors of the invention may vary between treatments. In a preferred embodiment of the invention, the oncolytic adenoviral vector having a different fiber knob of the capsid compared to the vector of the earlier treatment is administered to a subject. As used herein "fiber knob of the capsid" refers to the knob part of the fiber protein (Figure 1).

The gene therapy of the invention is effective alone, but combination of adenoviral gene therapy with any other therapies, such as traditional therapy, may be more effective than either one alone. For example, each agent of the combination therapy may work independently in the tumor tissue, the adenoviral vectors may sensitize cells to chemotherapy or radiotherapy and/or chemotherapeutic agents may enhance the level of virus replication or effect the receptor status of the target cells. The agents of combination therapy may be administered simultaneously or sequentially.

In a preferred embodiment of the invention, the method or use further comprises administration of concurrent radiotherapy to a subject. In another preferred embodiment of the invention, the method or use further comprises administration of concurrent chemotherapy to a subject. As used herein "concurrent" refers to a therapy, which has been administered before, after or simultaneously with the gene therapy of the invention. The period for a concurrent therapy may vary from minutes to several weeks. Preferably the concurrent therapy lasts for some hours.

Agents suitable for combination therapy include but are not limited to All-trans retinoic acid, Azacitidine, Azathioprine, Bleomycin, Carboplatin, Capecitabine, Cisplatin, Chlorambucil, Cyclophosphamide, Cytarabine, Daunorubicin, Docetaxel, Doxifluridine, Doxorubicin, Epirubicin, Epothilone, Etoposide, Fluorouracil, Gemcitabine, Hydroxyurea, Idarubicin, Imatinib, Mechlorethamine, Mercaptopurine, Methotrexate, Mitoxantrone, Oxaliplatin, Paclitaxel, Pemetrexed, Temozolomide, Teniposide, Tioguanine, Valrubicin, Vinblastine, Vincristine, Vindesine and Vinorelbine.

In a preferred embodiment of the invention, the method or use further comprises administration of verapamil or another calcium channel blocker to a subject. "Calcium channel blocker" refers to a class of drugs and natural substances which disrupt the conduction of calcium channels, and it may be selected from a group consisting of verapamil, dihydropyridines, gallopamil, diltiazem, mibefradil, bepridil, fluspirilene and fendiline.

In a preferred embodiment of the invention, the method or use further comprises administration of autophagy inducing agents to a subject. Autophagy refers to a catabolic process involving the degradation of a cell's own components through the lysosomal machinery. "Autophagy inducing agents" refer to agents capable of inducing autophagy and may be selected from a group consisting of, but not limited to, mTOR inhibitors, PI3K inhibitors, lithium, tamoxifen, chloroquine, bafilomycin, temsirolimus, sirolimus and temozolomide. In a specific embodiment of the invention, the method further comprises administration of temozolomide to a subject. Temozolomide may be either oral or intravenous temozolomide.

In one embodiment of the invention, the method or use further comprises administration of chemotherapy or anti-CD20 therapy or other approaches for blocking of neutralizing antibodies. "Anti-CD20 therapy" refers to agents capable of killing CD20 positive cells, and may be selected from a group consisting of rituximab and other anti-CD20 monoclonal antibodies. "Approaches for blocking of neutralizing antibodies" refers to agents capable of inhibiting the generation of anti-viral antibodies that normally result from infection and may be selected from a group consisting of different chemotherapeutics, immunomodulatory substances, corticoids and other drugs. These substances may be selected from a group consisting of, but not limited to, cyclophosphamide, cyclosporin, azathioprine, methylprenisolone, etoposide, CD40L, CTLA4Ig4, FK506 (tacrolismus), IL-12, IFN-gamma, interleukin 10, anti-CD8, anti-CD4 antibodies, myeloablation and oral adenoviral proteins.

The oncolytic adenoviral vector as described induces virion mediated oncolysis of tumor cells and activates human immune response against tumor cells. In a preferred embodiment, the method or use further comprises administration of substances capable to downregulating regulatory T-cells in a subject. "Substances capable to downregulating regulatory T-cells" refers to agents that reduce the amount of cells identified as T-suppressor or Regulatory T-cells. These cells have been identified as consisting one or many of the following immunophenotypic markers: CD4+, CD25+, FoxP3+, CD127- and GITR+. Such agents reducing T-suppressor or Regulatory T-cells may be selected from a group consisting of anti-CD25 antibodies or chemotherapeutics.

In a preferred embodiment of the invention, the method or use further comprises administration of cyclophosphamide to a subject. Cyclophosphamide is a common chemotherapeutic agent, which has also been used in some autoimmune disorders. In the present invention, cyclophosphamide can be used to enhance viral replication and the effects of GM-CSF induced stimulation of NK and cytotoxic T-cells for enhanced immune response against the tumor. It can be used as intravenous bolus doses or low-dose oral metronomic administration.

Any method or use of the invention may be either *in vivo, ex vivo* or *in vitro* method or use.

The present invention is illustrated by the following examples, which are not intended to be limiting in any way.

### Examples

### Example 1. Cloning of three D24-GM-CSF type viruses

- PCR out hGM-CSF,
- create Sunl/Munl sites => 445 bp (pORF-GM-CSF as a template)
- Sunl/Munl digestion of PCR product and pTHSN
- Sticky-end ligation =>
- *Pme*I linearized pShuttle-D24 + pTG3602 => pAd5-D24
- Ad5-D24-GM-CSF (SEQ ID NO 8: E1A region with D24 deletion in nucleotide positions 563-1524 and a fiber region in nucleotide positions 30490-32236) Homol recomb: *Srf*I linearized pAd5-D24 + *Fsp*I linearized pTHSN-GM-CSF => pAd5-D24-GM-CSF Pacl linearization & transfection => Ad5-D24-GM-CSF

All phases of the cloning were confirmed with PCR and multiple restriction digestions. The shuttle plasmid pTHSN-GMCSF was sequenced. Absence of wild type E1 was confirmed with PCR. The E1 region, transgene and fiber were checked in the final virus with sequencing and PCR, which was then taken to the clean lab for production. To this end, viral DNA was extracted by over night (ON) incubation with appropriate buffer solution and after PCR and sequence was performed to analyze the integrity of the fiber as well as the GMCSF cDNA. All phases of the virus production, including transfection, were done on A549 cells to avoid risk of wild type recombination as described before (Kanerva A et al. 2003, Mol Ther 8, 449-58; Baeurschmitz GJ et al. 2006, Mol Ther 14, 164-74). GM-CSF is under the E3 promoter (specifically under endogenous viral E3A gene expression control elements), which results in replication associate transgene expression, which starts about 8h after infection. E3 is intact except for deletion of *6.7K*/*gp19K.*

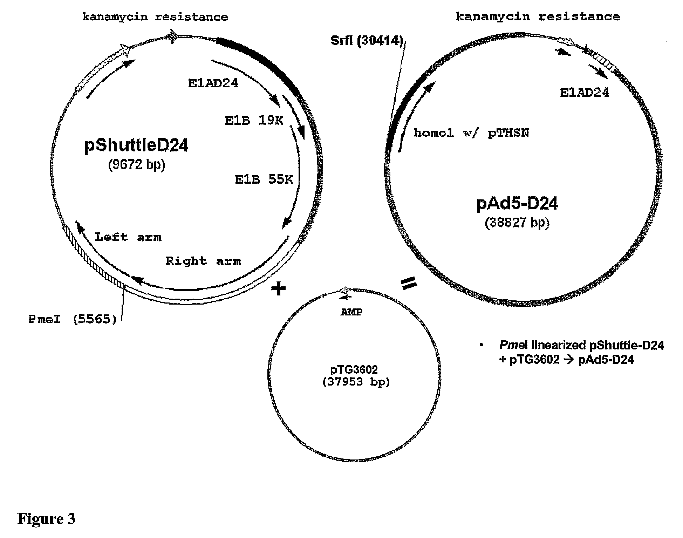

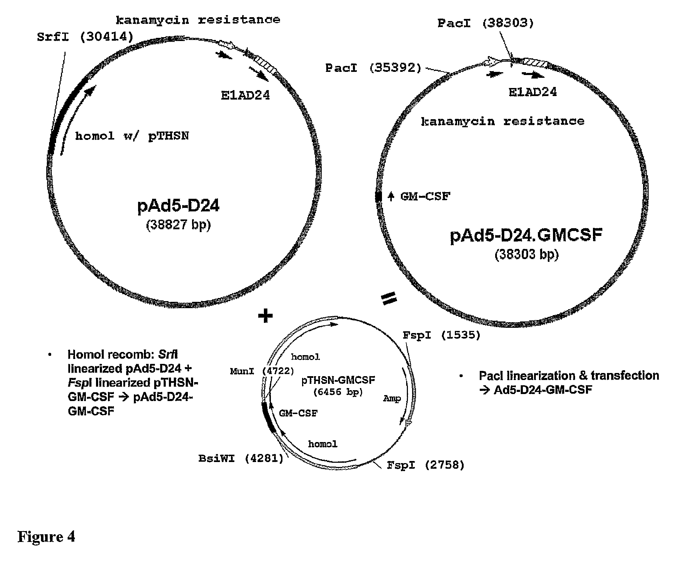

Ad5/3-D24-GM-CSF (SEQ ID NO 7) and Ad5-RGD-D24-GM-CSF (SEQ ID NO 9: E1A region with D24 deletion in nucleotide positions 580-1541, a fiber region in nucleotide positions 30514-32286 and RGD-modification in nucleotide positions 32128-32183) were constructed identically, except a rescue plasmid featuring either a knob from serotype 3, or RGD-4C in the Ad5 fiber HI-loop were used. Ad5-pK7-D24-GMCSF (SEQ ID NO 10: E1A region with D24 deletion in nucleotide positions 561-1526, a fiber region in nucleotide positions 30499-32255 and pK7-modification in nucleotide positions 32247-32378) was also created similarly. (Figures 2-4)

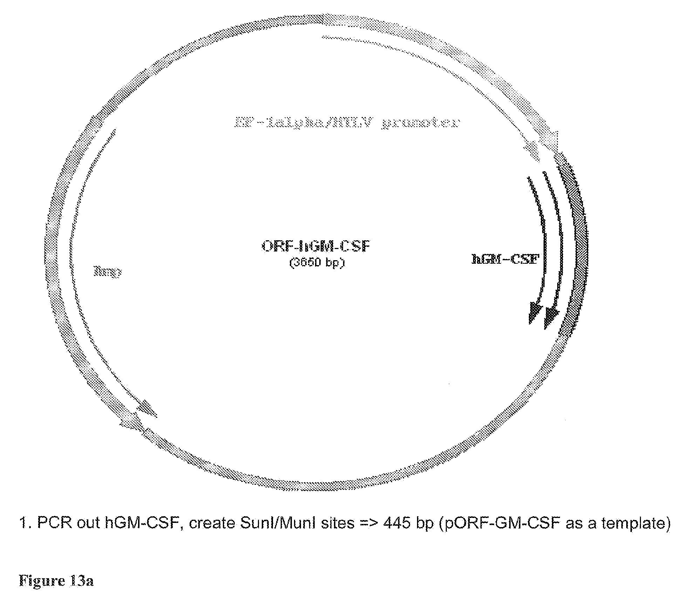

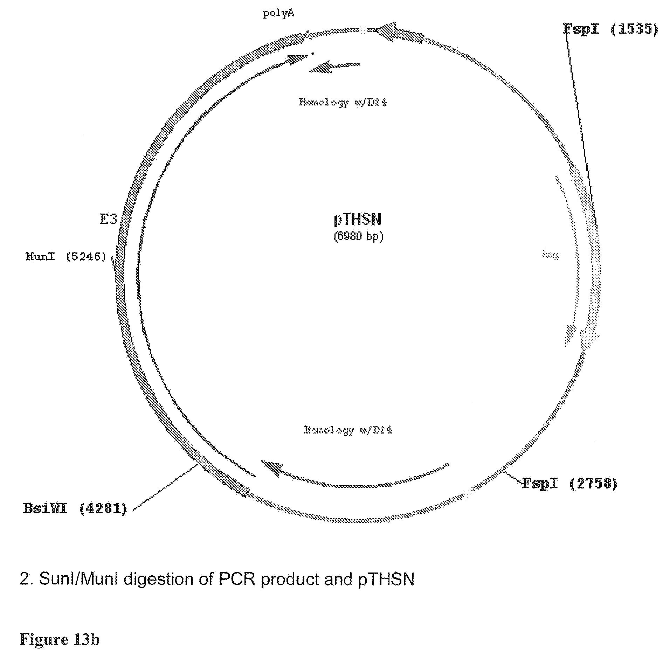

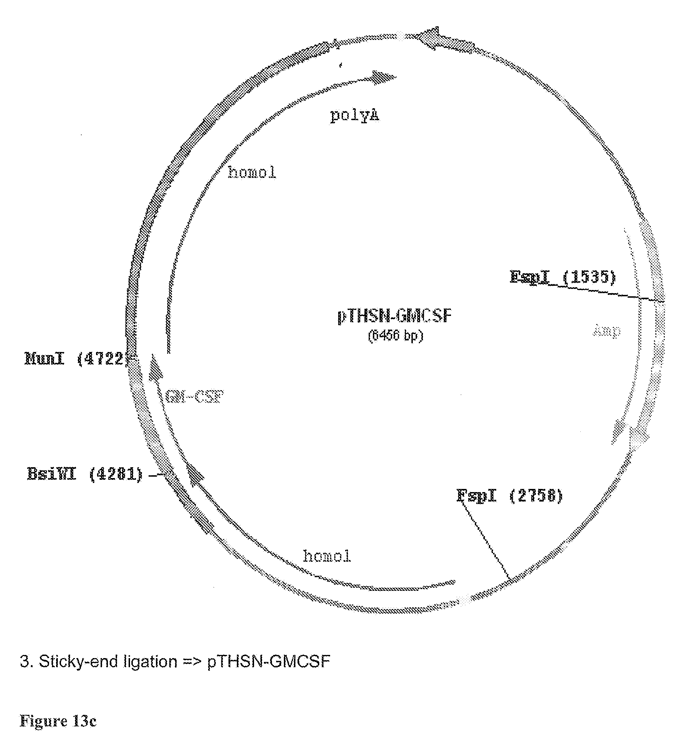

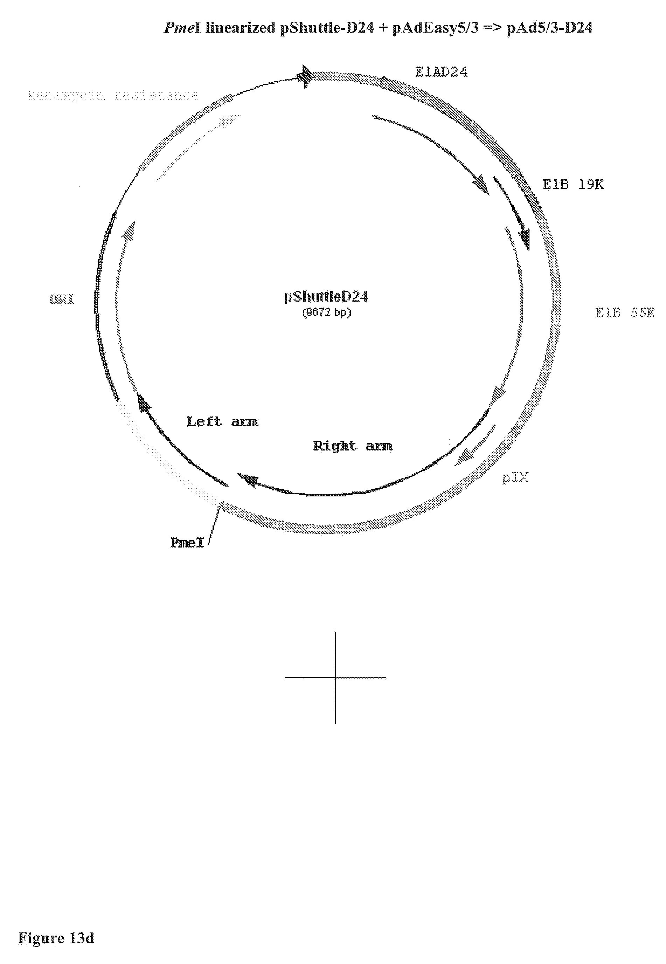

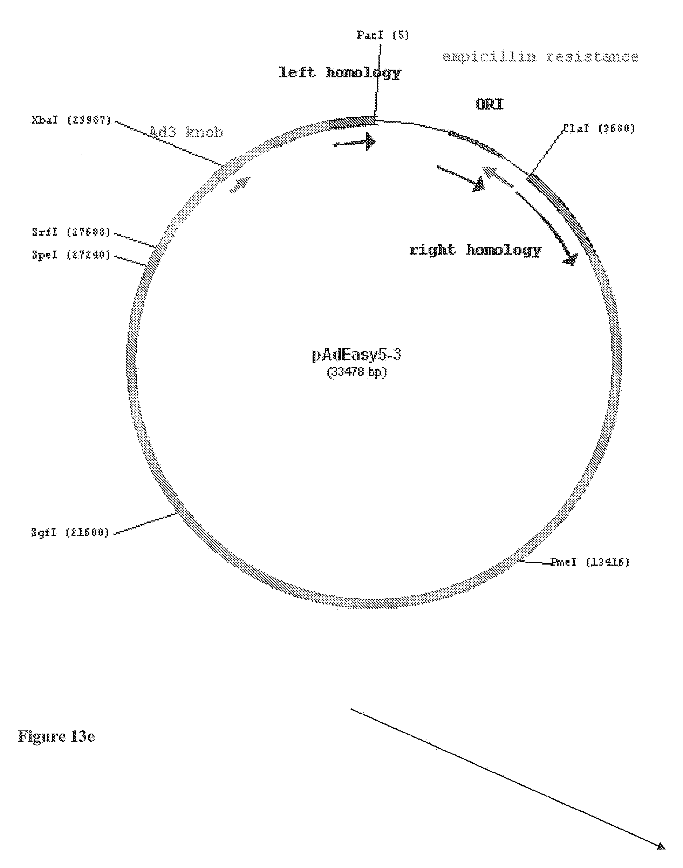

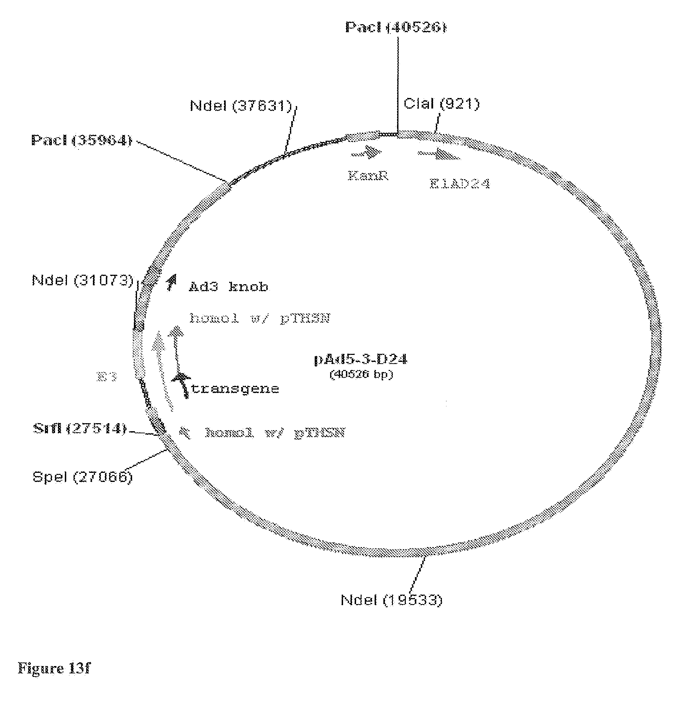

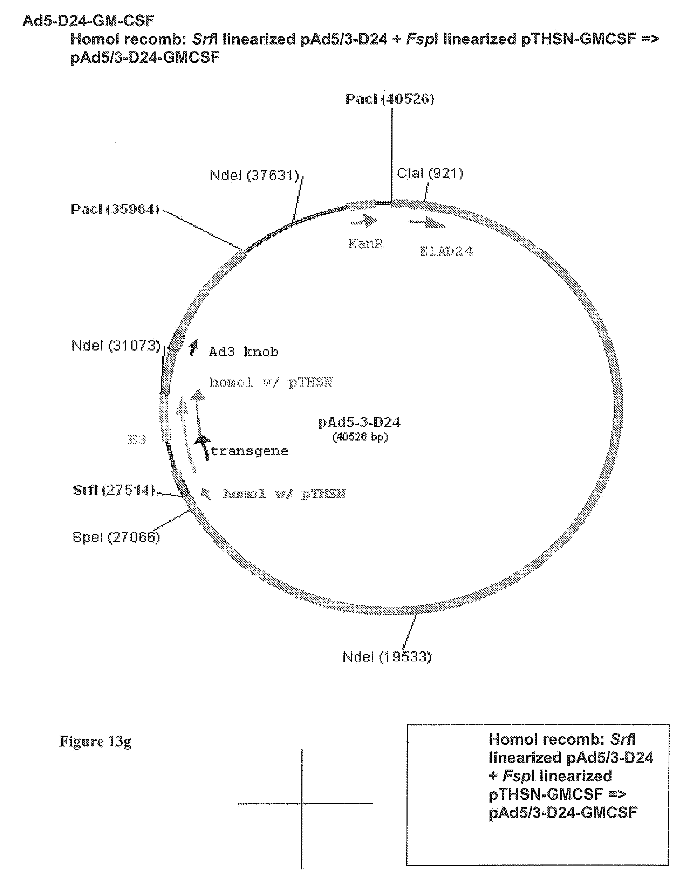

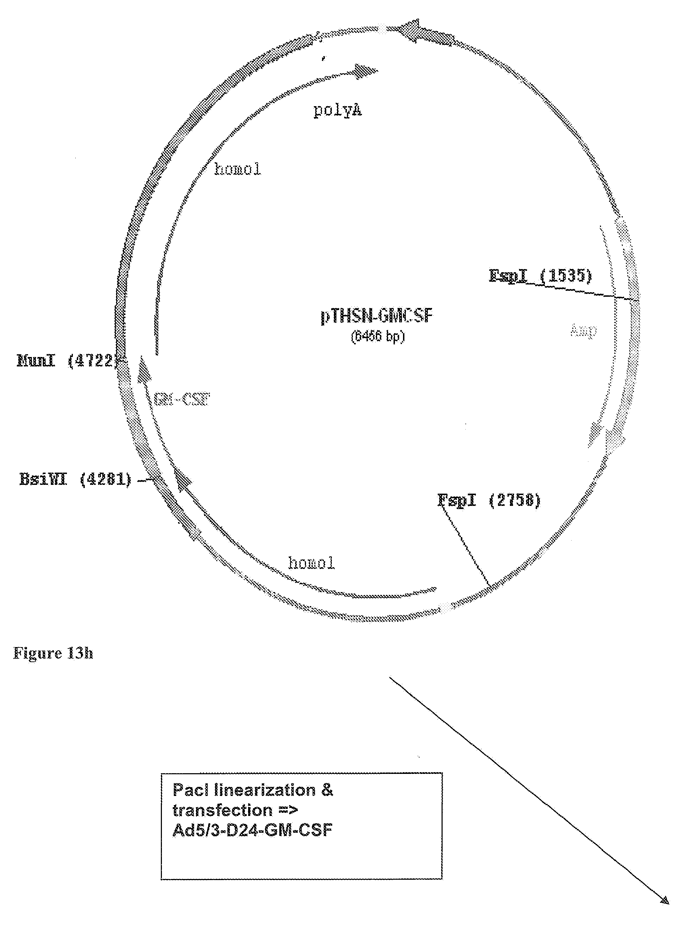

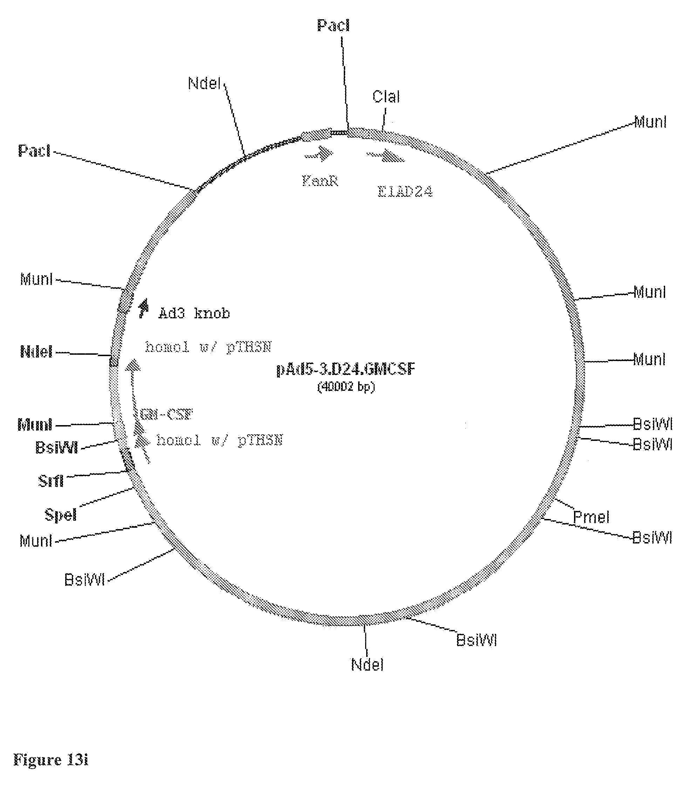

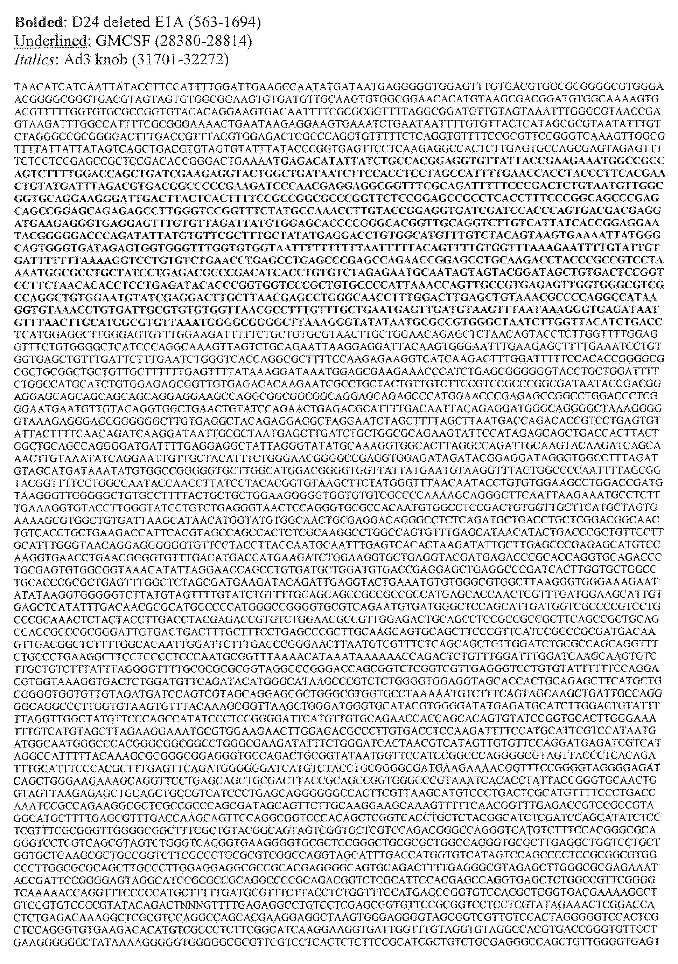

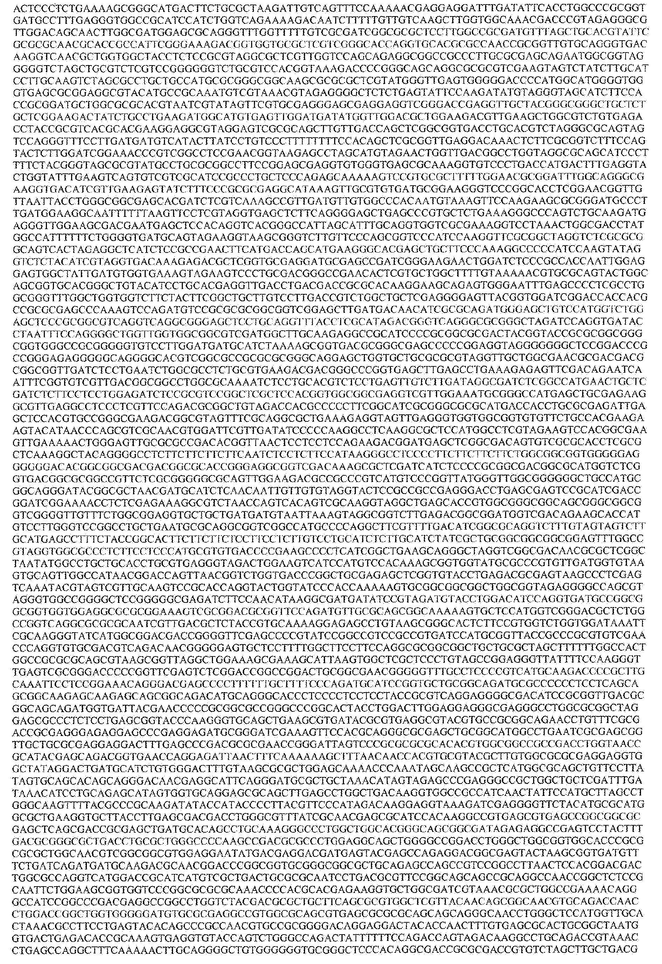

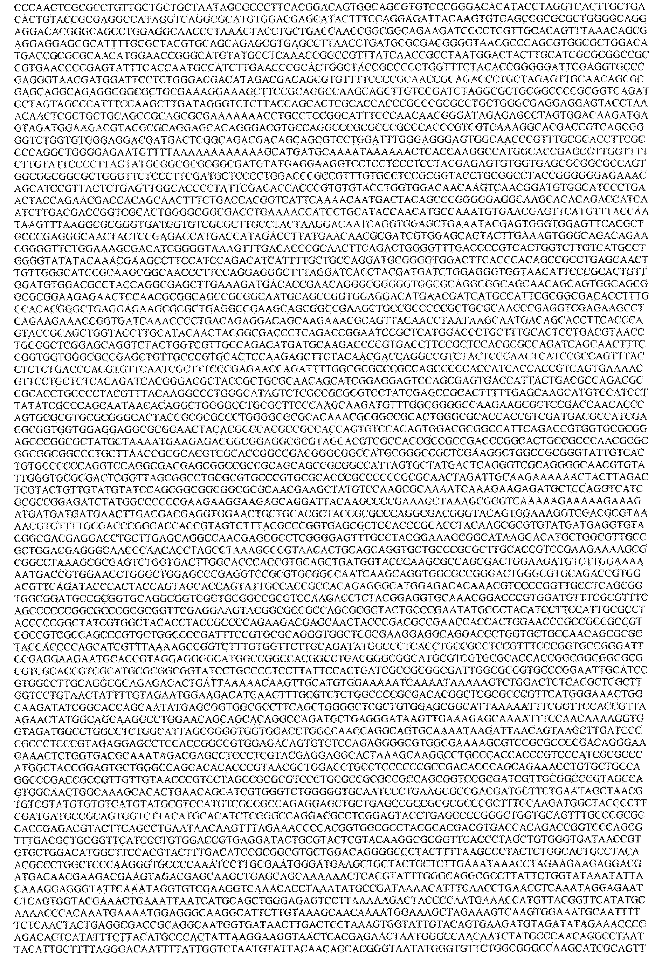

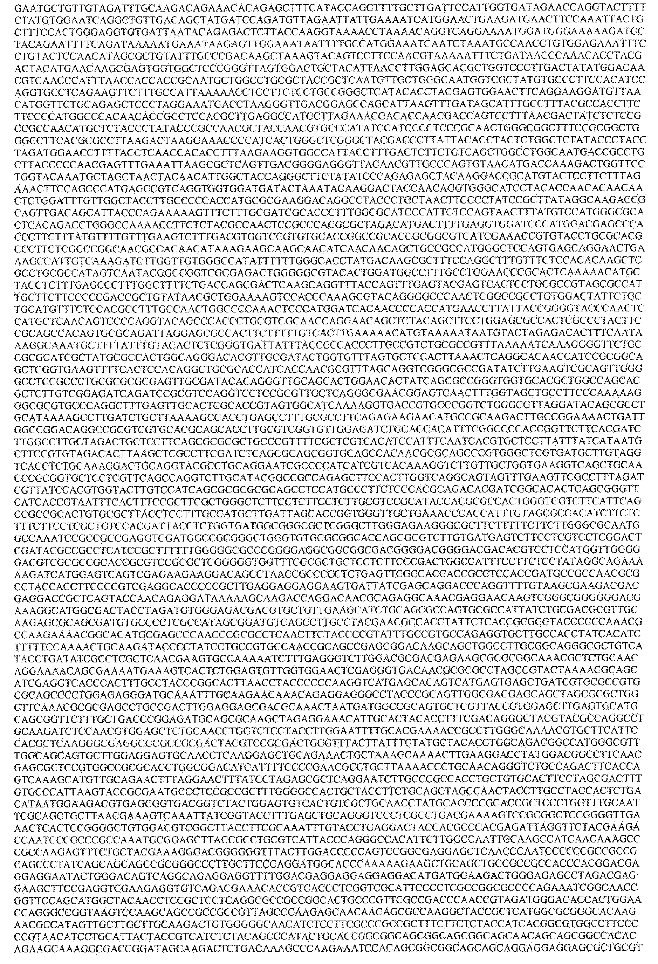

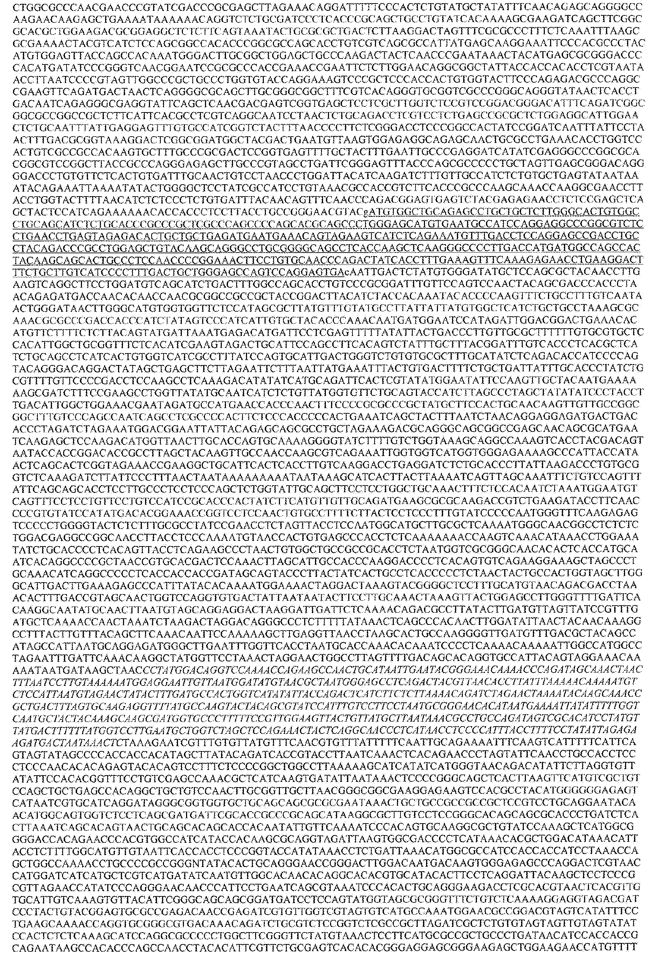

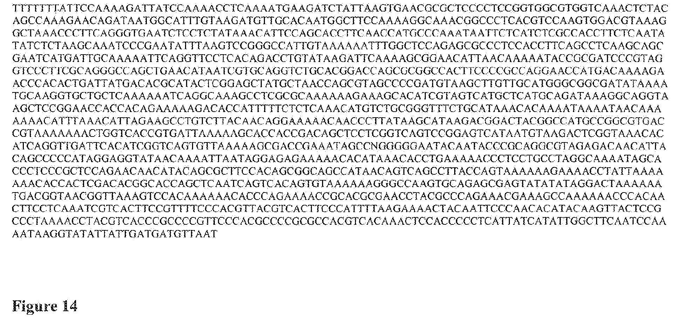

Ad5/3-D24-GM-CSF was constructed as follows. A pAdEasy-1-derived plasmid containing a chimeric 5/3 fiber, pAdEasy5/3, was created by homologous recombination in *E.coli* of Ad5/3l1uc1 viral genome and *BstX*I-digested 8.9 kb fragment of pAdEasy-1. Next, a shuttle vector containing a 24-bp deletion in *E1A* (pShuttleD24) was linearized with *Pme*I and recombined with pAdEasy5/3 resulting in pAd5/3-D24. In order to insert human GMCSF gene into *E3* region, an *E*3-cloning vector pTHSN was created by inserting *Spe*I to *Nde*I fragment from Ad5 genome into the multi-cloning site of pGEMSZf+ (Promega, Madison, WI). pTHSN was further digested with *Sun*I/*Mun*I creating a 965-bp deletion in *E3* region (*6.7K* and *gp19K* deleted). The 432bp cDNA encoding human GMCSF (Invitrogen, Carlsbad CA) was amplified with primers featuring specific restriction sites *Sun*I/*Mun*I flanking the gene and then inserted into *Sun*I/*Mun*I-digested pTHSN (pTHSN-GMCSF). pAd5/3-D24-GMCSF was generated by homologous recombination in *E.coli* between Fspl-linearized pTHSN-GMCSF and Srfl-linearized pAd5/3-D24. Ad5/3-D24-GMCSF virus genome was released by *Pac*I digestion and transfection to A549 cells for amplification and rescue (Figures 13 and 14, SEQ ID NO 7).

### Example 2. In vitro analysis of D24-GM-CSF type viruses

In vitro efficacy of D24-GM-CSF type viruses was studied in lung cancer cells (A549), breast cancer stem cell derived explant cells (JIMT-1) and breast cancer cells (MDA-MB-436) by utilizing MTS cell killing assays. MTS assay is currently the standard method to assess cell viability in cancer gene therapy publications. Ad5Luc1 is a replication deficient virus and acts as a negative control. Ad5wt is a wild type Ad5 virus (strain Ad300wt) and was used as a positive control. Ad5-d24-E3 harbors an isogenic 24bp deletion in E1 but is intact in E3. VP indicates virus particles.

In summary, Ad5-D24-GMCSF had oncolytic activity similar to positive controls in vitro, and therefore transgene production did not compromise the oncolytic potency of the virus (Figures 5a-c). Similar data was shown for Ad5/3-D24-GM-CSF and Ad5-RGD-D24-GM-CSF (Figure 5d).

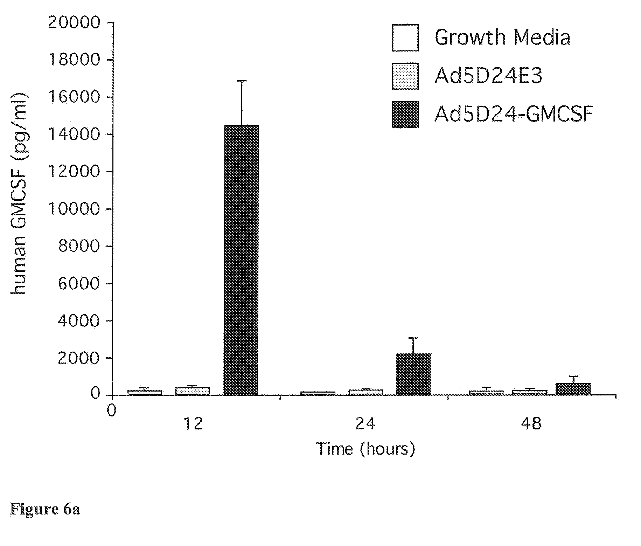

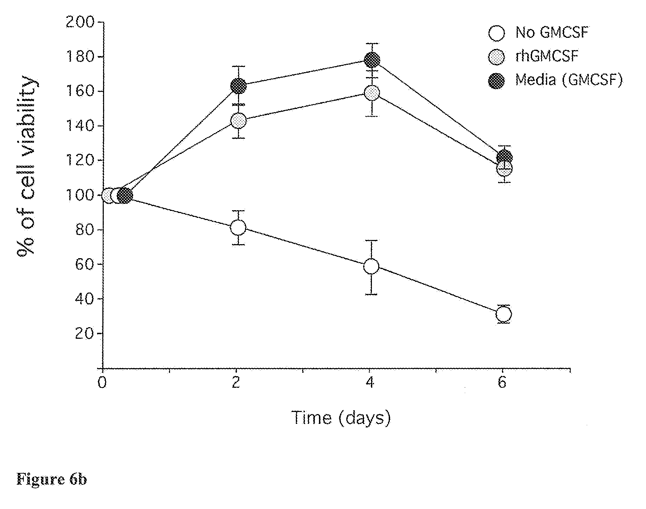

To test whether Ad5D24-GMCSF was able to express the transgene, A549 cell line was infected with 1000 VP/cell and media was collected over time. Concentration of GMCSF (Figure 6a) in the media was measured by FACSARRAY (BD Biosciences, San Diego, CA USA) according to manufacturer's instructions. In addition to that, we also analyzed whether the virus-expressed GMCSF retained its biological function. To this end TF1 cell line, whose growth and survival is strictly dependent on human GMCSF were treated with media collected from A549 cell line previously infected with Ad5D24-GMCSF. TF1 viability was assessed over time by MTS assay. The result of this experiment was that the virus expressed-GMCSF was able to stimulate growth of such cell line, and no difference was found with the same cell line treated with human recombinant GMCSF (Sigma) (Figure 6b).

### Example 3. Pretreatment analysis of transduction

### I. Infection of tumor cells with Ad5Luc1

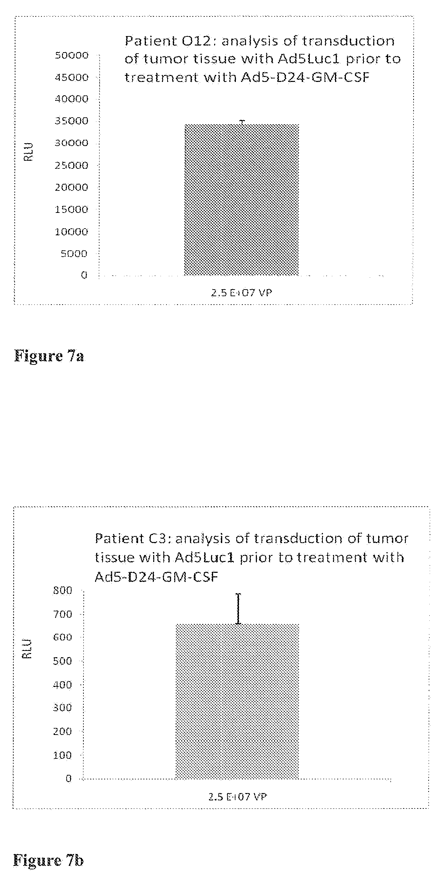

To check that a tumor could be infected with Ad5 based viruses, biopsies taken from tissues were homogenized, and infected with luciferase coding Ad5Luc1 according to standard protocol of infection. Briefly, cells seeded in wells were washed twice with PBS, virus was thawed and resuspended in a minimun amount of growth media and gently poured on the cells. The infection proceeded for 30 minutes and afterwards the cells were washed again in PBS and appropriate amount of complete growth media was added. Luciferase quantification was assessed 24 hours later. Please note that only a minute amount of tissue was obtained and therefore the number of cells could not be calculated, nor the amount of virus normalized to amount of tissue. Thus, no quantitative analyses were made, but qualitative data showed successful gene transfer in patients 012 and C3 (Figures 7a-b). Background luciferase values (circa 200 RLU) were subtracted.

### II. Immunohistochemical staining of CAR

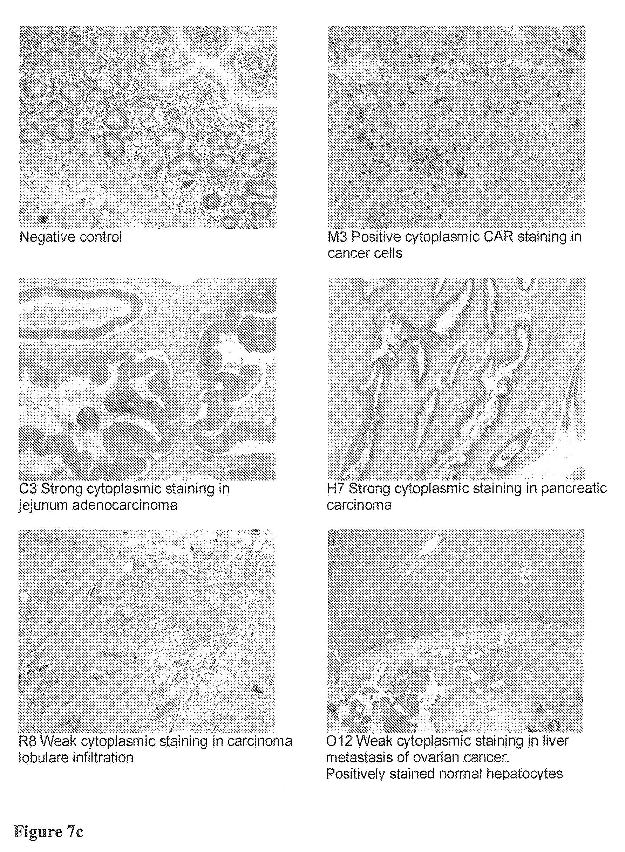

Available archival specimens of patient's tumors (patients for Ad5-D24-GM-CSF treatment) were collected and analyzed for CAR (the adenovirus serotype 5 receptor) expression by immunohistochemistry. Adenovirus receptor CAR stainings of cancer cell cytoplasms (M3), jejunum adeno-carsinoma (C3), pancreatic carcinoma (H7), carcinoma lobulare infiltration (R8), liver metastasis of ovarian cancer (012) and lung metastasis of synovialsarcoma (S23) are shown in Figure 7c.

### III. Neutralizing antibody titers against the Ad5/3 capsid

293 cells were seeded on 96-well plates at 1 x 10⁴ cells/well and cultured overnight. Next day, cells were washed with DMEM without FCS. To inactivate complement, human serum samples were incubated at 56 °C for 90 min. A four-fold dilution series (1:1 to 1:16 384) was prepared in serum-free DMEM (Sarkioja M et al. 2008, Gene Ther 15(12): 921-9). Ad5/3l1uc1 was mixed with serum dilutions and incubated at room temperature for 30 min. Next, cells in triplicates were infected with 100 VP/cell in 50 µl of mix, and 1 h later 100 µl of growth medium with 10% FCS was added. 24h post-infection, cells were lysed and luciferase activity was measured with Luciferase Assay System (Promega, Madison, WI) utilizing TopCount luminometer (PerkinElmer, Waltham, MA). Luciferase readings were plotted relative to gene transfer achieved with Ad5/3l1uc1 alone in order to evaluate the effect of neutralizing antibodies in the serum of patients treated with Ad5/3-d24-GMCSF. The neutralizing antibody titer was determined as the lowest degree of dilution that blocked gene transfer more than 80%.

### Example 4. Ex vivo analysis of Ad5/3-D24-GMCSF efficacy in ascites and pleural samples

Fresh ascites/pleural effusion sample was stored at +4ºC over night. The sample was divided into 50ml falcon tubes and the cells were isolated by centrifugation 900rpm, 8min, +4ºC. To lyse red blood cells the sample was incubated 5-10min in room temperature with 25ml ACK Lysis Buffer (Invitrogen, Carlsbad, CA). The falcons were filled up with 2%DMEM and the cells were centrifugated (900rpm, 8min, +4ºC). Cell suspension of 100000 cells/ml in 2%DMEM-fungizone was prepared (50ml 2%DMEM + 200µl Fungizone (BMS, Espoo, Finland)).

For luciferase-assay, to test the effect of capsid modification on transductional efficacy, cells were seeded into two 24-well plates, 50 000 cells/well. 24h later the cells in triplicates were infected with Ad5luc1 or Ad5/3l1uc1 in concentrations of 500vp/cell and 5000/vp/cell in 2% DMEM. Luciferase expression was analyzed similarly as in Example 3 III (determining neutralizing antibody titers).

Fresh pre-treatment samples of ascites and pleural effusion for patients K75 and V136, respectively, were analyzed, and in both samples high transduction with Ad5/3 was seen.

For MTS-assay, to test the potency of Ad5/3-d24-GMCSF on clinical samples, cells were seeded into two 96-well plates, 10 000 cells/well. The cells were infected after 24h incubation. Infections were carried out in 2%DMEM. Next day 10%DMEM was added. The cells were checked daily and the culture medium was changed every other day. Before measuring, the medium was aspirated and 100µl of fresh 10% DMEM was pipetted to the wells. 20µl of MTS- Assay Buffer (Promega, Madison, WI) was added and the cells were incubated for 1.5 - 4 hours. The absorbance was measured with Multi-scan Ascent and Ascent Software v2.6 (Thermo Labsystems, Helsinki, Finland) at 490 nm and background absorbance was subtracted from the absorbance of the samples.

Pre-treatment samples of pleural effusion from V136 and M137 were assessed for oncolytic potency of Ad5/3-d24-GMCSF. Six days after infection there was 62% and 29% less viable cells (p<0.001), respectively, than in an uninfected control sample suggesting that Ad5/3-d24-GMCSF was able to kill tumor cells present in the effusion.

For assessing the presence of virus in samples obtained after treatment, cells were resuspended in 3ml 2%DMEM after lysing red blood cells and freeze-thawed four times in -80ºC. The 293 cells were seeded in 96-well plates 10 000cells/well and incubated for 24h hours. The sample was centrifugated 15min, 4000rpm, +4ºC and the supernatant was collected. 293 cells were infected with 100µl/well of the supernatant. After 10 days of incubation the wells were assessed for the presence of cytopathic effect.

To assess replication of Ad5/3-d24GMCSF at the tumor, we also analyzed an ascites sample taken from patient 082 7 days after treatment. This resulted in 70% of the cells showing cytopathic effect, while uninfected control cells did not show similar effects.

### Example 5. In vivo analysis of D24-GM-CSF type viruses in animals

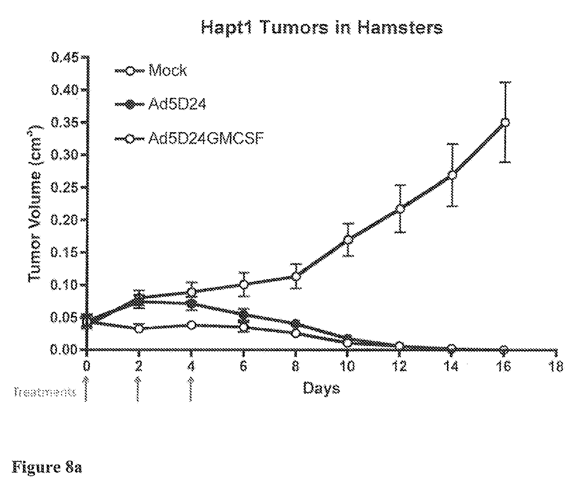

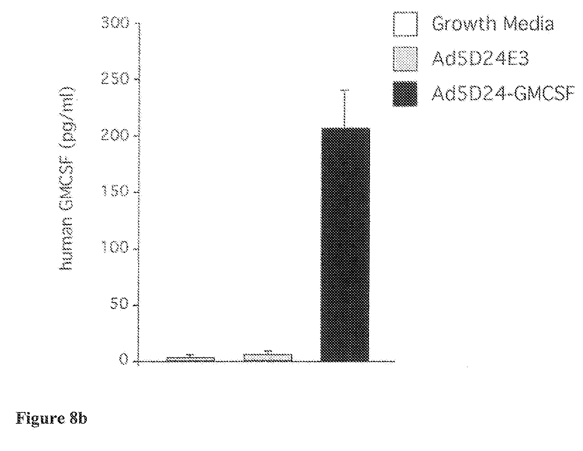

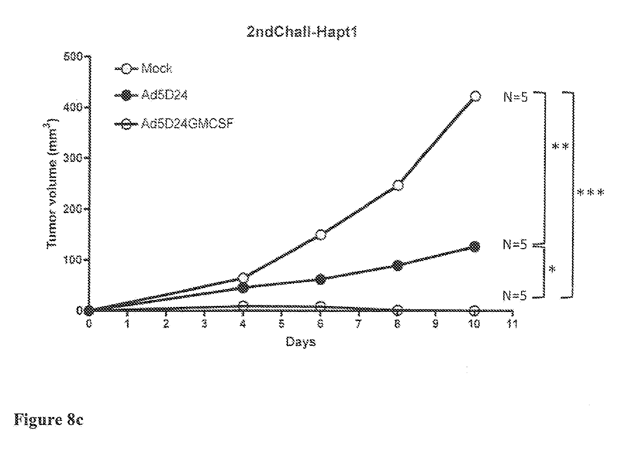

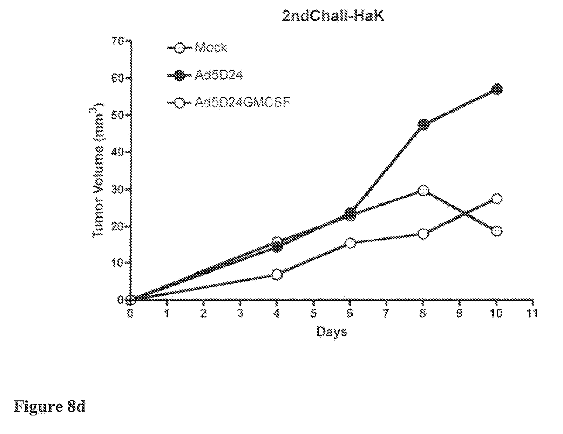

In vivo efficacy of Ad5-D24-GM-CSF was tested in immune competent Syrian hamsters, which are semipermissive for human adenovirus replication (mice are non-permissive) (Ying B. et al. 2009, Cancer Gene Ther doi:10.1038/cgt.2009.6.). 7*10⁶ HapT1 pancreatic cancer cells were injected subcutaneously and 1*10⁹ virus particles (VP) of Ad5-D24-GM-CSF or Ad5D24E3 (which does not express GM-CSF) were injected intratumorally on day 0, 2 and 4. The mock group was injected with the same volume of growth media at the same indicated time points. Figure 8b shows that intratumoral injections of Ad5-D24-GMCSF resulted in high levels of hGM-CSF in serum of Syrian hamsters. Human GM-CSF is known to be active in Syrian hamsters (Cho, Exp Toxicol Pathol 2006 vol. 57 (4) pp. 321-8). Interestingly, all animals were tumor-free by day sixteen except for mock group (Figure 8a). Tumor scars were still analyzed for two additional weeks to assess whether reappearance of the tumor might have occurred. However, on day 32 there was still no sign of tumor in these animals so that the first part of the experiment was terminated and animals of the Mock group were euthanized. The remaining treated animals were at this point challenged with the same tumor in the right side of the upper body by subcutaneous injection of 7^{*}10⁶ HapT1 cells while on the left side were challenged with a different tumor (1*10⁶ of HaK tumor) for which the animals were naïve. Tumor growth was measured over time and is reported in Figures 8c-d. Interestingly the animals that were previously treated with Ad5D24GMCSF completely rejected the HapT1 tumor challenge while Hak tumors grew normally, while the animals that were previously treated with Ad5D24E3 grew independently HapT1 and HaK tumors (Figures 8c-d).

In summary, the data indicates that Ad5-D24-GM-CSF has antitumor activity in immune competent tumor bearing animals, and it is able to elicit tumor-specific immunity to the degree of rejecting subsequent challenge of the same tumor.

### Example 6. In vivo analysis of D24-GM-CSF type viruses in human patients

### I. Patients

Patients with advanced and treatment refractory solid tumors were enrolled in a government-approved compassionate treatment protocol. Information of patients receiving Ad5-D24-GM-CSF is listed in Table 1.

22 patients with advanced solid tumors refractory to standard therapies (Table 6) were treated with a single round of Ad5/3-d24-GMCSF intravenously and intratumorally (Table 7). Intratumoral injection was performed intraperitoneally or intrapleurally in the case of carcinomatosis or pleural metastases, respectively. Inclusion criteria were solid tumors refractory to conventional therapies, WHO performance score 3 or less and no major organ function deficiencies. Exclusion criteria were organ transplant, HIV, severe cardiovascular, metabolic or pulmonary disease or other symptoms, findings or diseases preventing oncolytic virus treatment. Written informed consent was obtained and treatments were administered according to Good Clinical Practice and the Declaration of Helsinki.

### II. Treatments with adenoviral vector encoding GM-CSF

### a) Ad5-D24-GM-CSF, Ad5/3-D24-GM-CSF or Ad5-RGD-D24-GM-CSF treatments

Ad5-D24-GM-CSF, Ad5/3-D24-GM-CSF and Ad5-RGD-D24-GM-CSF were then produced according to clinical grade and treatment of patients was initiated. This "phase 0" compassionate use program has been discussed at the HUCH Surgical Ethics committee. The program has also been discussed at FinOHTA (national evaluation of medical technologies) and Ethics Negotiation Board of the Finnish Medical Association. Legal aspects have been checked with Ministry of Social Affairs and Health, the National Agency of Medicines, the legal council at the Finnish Medical Association, the National authority for Medicolegal affairs and the Parliamentary Board of Social Affairs and Health. The treatments have been approved by the Finnish Gene Technology Board.

Patients received a single round of treatment on day 0. Virus administration was performed by ultrasound-guided intratumoral injection and circa one fifth of the dose was given intravenously. The starting dose of 8x10¹⁰ VP was chosen based on safety results published by others.

Virus was diluted in sterile saline solution at the time of administration under appropriate condition. Following virus administration all patients were monitored overnight at the hospital and subsequently for the following 4 weeks as outpatients. Physical assessment and medical history were done at each visit and clinically relevant laboratory values were followed. Side effects of treatment were recorded and scored according to Common Terminology for Adverse Events v3.0 (CTCAE).

Because many cancer patients have symptoms due to disease, pre-existing symptoms were not scored if they did not become worse. However, if the symptom became more severe, e.g. pre-treatment grade 1 changed to grade 2 after treatment, it was scored as grade 2. Serum levels of GMCSF and four other cytokines (IL-6, IL-8, IL-10 and TNF-alpha) were analyzed by BD Cytometric Bead Array (CBA) Human Soluble Protein Flex Set (Becton Dickinson, Franklin Lakes, NJ, US). Tumor size was assessed by contrast-enhanced computer tomography (CT) scanning. Maximum tumor diameters were obtained. Response Evaluation Criteria in Solid Tumors (RECIST1.1) criteria were applied to overall disease, including injected and non-injected lesions. These criteria are: partial response PR (>30% reduction in the sum of tumor diameters), stable disease SD (no reduction/increase), progressive disease PD (>20% increase). Clear tumor decreases not fulfilling PR were scored as minor responses (MR). Serum tumor markers were also evaluated when elevated at baseline, and the same percentages were used.

Blood samples were collected before and after the treatment for analyses. In Table 1 is summarized the viral load in the serum of patients treated with Ad5-D24-GMCSF. Quantitative PCR (qPCR) was used for that analysis (see section III for description of the methods).

Tables 2, 3 and 4 summarize all the adverse events that were recorded during and after the Ad5-D24-GM-CSF treatment. All the adverse events have been graded according to Common Terminology for Adverse Events v3.0 (CTCAE). Of note is that all the patients manifested grade 1 or/and 2 flu-like symptoms, but only two grade 3 symptoms were observed, one case of constipation in an ovarian cancer patients who had suffered of constipation before and one grade 3 hyponatremia.

In Table 5 there is reported the efficacy evaluation of Ad5-D24-GM-CSF according to RECIST criteria (Therasse P et al. 2000, J Natl Cancer Inst 92, 205-16). Interestingly two complete response (CR) and five stable diseases (SD) were observed in 14 analyzable patients for a 50% clinical benefit rate.

### b) Safety of Ad5/3-d24-GMCSF in Cancer Patients

Treatments were well tolerated up to the highest used: 4x10¹¹ VP/patient. No grade 4-5 adverse events were seen. Grade 1-2 flu-like symptoms were common with 19/22, 17/22 and 8/22 patients experiencing fever, fatigue or upper respiratory symptoms, respectively. Pain in the injection site (6 patients), abdominal pain (10 patients) and nausea (9 patients) were also common grade 1-2 adverse events as well as (Table 8). Asymptomatic and self-limiting grade 3 hematological side effects were seen in 4 patients: anemia (grade 2 at baseline), neutropenia, aspartate aminotrasferase elevation and hyponatremia. The only non-hematological grade 3 side effect was cholecystitis seen three week after treatment in pancreatic cancer patient H83. He also had grade 3 alanine aminotrasferase and bilirubin elevations. Taken together, these symptoms suggest tumor mediated biliary duct compression. It is unclear if this was treatment mediated inflammatory swelling or tumor growth caused by disease progression.

### III. Detection of the virus from blood

Serum samples were collected from patients treated with Ad5-D24-GM-CSF or Ad5/3-D24-GM-CSF (see example 6, *I.)* and conventional PCR was carried out with primers and conditions according to Takayama et al. 2007, J. Med. Virol. 79:278-284. Briefly, total DNA was extracted by adding 3 µg of carrier DNA (polydeoxyadenylic acid; Roche, Mannheim, Germany) to 400 µl of serum and using the QIAamp DNA mini kit. Extracted DNA was eluted in 60 µl nuclease-free water and DNA concentration was measured by spectrophotometry. PCR amplification was based on primers and probe targeting the E1 A region flanking the 24-bp deletion (forward primer 5'-TCCGGTTTCTATGCCAAACCT-3' (SEQ ID NO 1), reverse primer 5'-TCCTCCGGTGATAATGACAAGA-3' (SEQ ID NO 2) and probe onco 5^{'FAM}-TGATCGATCCACCCAGTGA-3^{'MGBNFQ} (SEQ ID NO 3)). In addition, a probe complementary to a sequence included in the 24-bp region targeted for deletion was used to test the samples for the presence of wild-type adenovirus infection (probe wt 5^{'VIC}-TACCTGCCACGAGGCT-3^{'MGBNFQ} (SEQ ID NO 4)).

The real-time PCR conditions for each 25 µl reaction were as follows: 2X LightCycler480 Probes Master Mix (Roche, Mannheim, Germany), 800 nM each forward and reverse primer, 200 nM each probe and 250 ng extracted DNA. PCR reactions were carried out in a LightCycler (Roche, Mannheim, Germany) under the following cycling conditions: 10 min at 95°C, 50 cycles of 10 s at 95°C, 30 s at 62°C and 20 sec at 72°C and 10 min at 40°C. All samples were tested in duplicate. TaqMan exogenous internal positive control reagents (Applied Biosystems) were used in the same PCR runs to test each sample for the presence of PCR inhibitors.

A regression standard curve was generated using DNA extracted from serial dilutions of Ad5/3-D24-Cox2L (1 X 10⁸-10 vp/ml) in normal human serum. The limit of detection and limit of quantification for the assay were 500 vp/ml of serum.

Positive samples were confirmed by real-time PCR using LightCycler480 SYBR Green I Master mix (Roche, Mannheim, Germany) and primers specific for adenovirus and GM-CSF sequences (forward primer 5'-AAACACCACCCTCCTTACCTG -3' (SEQ ID NO 5) and reverse primer 5'-TCATTCATCTCAGCAGCAGTG -3' (SEQ ID NO 6)).

### IV. Presence of Ad5/3-d24-GMCSF in Serum after Treatment

All patients were negative for Ad5/3-d24-GMCSF prior to the treatment with Ad5/3-d24-GMCSF. On day 1 17/19 patients had measurable levels of virus genomes in the serum, with the highest titer being 2061 VP/ml serum. From samples taken during days 3-7 12/15 patients were positive, with the highest titer of 3,36 x10⁵ vp/ml serum. Positive samples were seen up to day 58 after treatment. (Table 9)

### V. GMCSF and neutralizing antibody titers in serum after treatment

There was no significant change in the systemic levels of GMCSF after Ad5/3-d24-GMCSF treatment, which correlated well with no significant effects seen in the levels of total white blood cell counts. This suggests a general restriction of GMCSF production to the local sites of virus replication in the tumors. In one patient, S70, a transient increase in serum GMCSF was seen on day 4 accompanied by a transient elevation of the leukocyte count. This might have been related to effective virus replication as the patient simultaneously had fever and 3.36x10⁵vp/ml of virus in serum (Table 9). This patient did not experience any serious adverse events during follow-up. However, a post treatment CT scan suggested anti-tumor activity (SD) and for 4 weeks after treatment she was feeling better and her persistent chest pains were gone. The highest GMCSF concentration measured in this patient's blood was 115pg/ml, which is approximately 10-fold lower than the toxic levels of GMCSF in humans.

At baseline 4/15 patients were completely negative for neutralizing antibodies against Ad5/3. Another 2 patients had barely detectable titers (1-4) while 8 patients had low neutralizing titer (16-64). No patients had medium or high neutralizing titers against Ad5/3 at baseline. After treatment the titer increased in all patients (p<0.005) (Table 9). No clear correlation was seen between neutralizing antibody titers and viral dose, antitumor activity or toxicity. Intriguingly, with regard to virus load in serum two patients, Y62 and 079, were positive for neutralizing antibodies at baseline and had high titers during weeks 2-4 but still had measureable loads of virus present in their serum at least 28 and 58 days, respectively (Table 9). This indicates that even high antibody titers cannot hinder virus replication in the tumors. Interestingly, the antibody titer did not reach maximum in all patients. For example, S70, X122 and H83 had large amounts of virus circulating during week 1 and their antibody titer rose slowly.

### VI. Killing of differentiated tumor cells

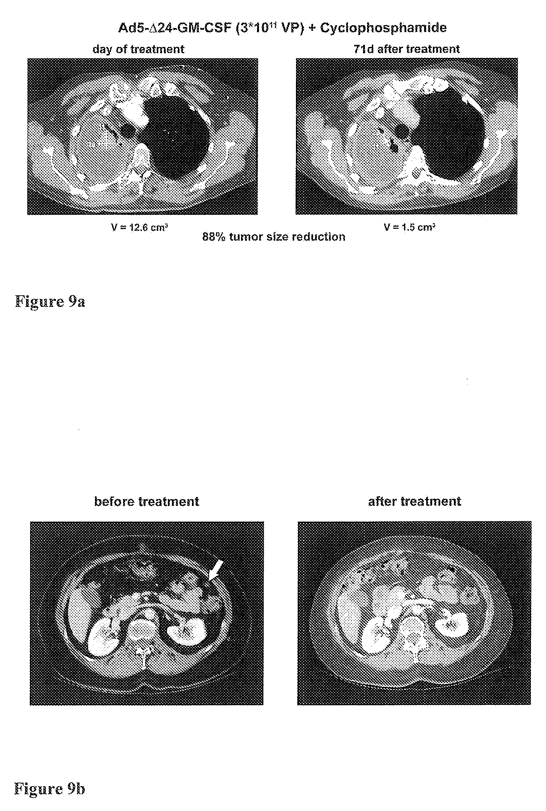

CT scans of a patient suffering from ovarian cancer and a patient suffering from mesothelioma (see Table 1) before and after Ad5-D24-GM-CSF treatment are shown in Figures 9a-b.

### VII. Efficacy of Ad5/3-D24-GMCSF

All patients had progressing tumors prior to treatment. 12 patients could be assessed for radiological benefit according to RECIST1.1 (Table 9). 2 patients had a minor response, 6 patients had stable disease, and 4 patients had progressive disease (PD). Therefore, the radiological clinical benefit rate was 67%. Of note, the rapidly growing pancreatic tumor in H96 stopped growing but a metastatic lesion appeared in the lungs and was thus scored PD. Similarly, patient 0129 had a 6% reduction of the injected tumor but had a new metastasis. In patient V136, who had two metastatic cancers, a non-injected liver lesion disappeared, while the other tumors remained SD.

With regard to tumor markers, assessed for patients who had elevated markers at baseline, 2/6 had some lowering of marker levels and 4/6 had elevation of marker levels (Table 9).

Overall survival of patients after treatment is shown in Figure 14.

In addition to objective measurements of anti-tumor activity we also saw clinical and/or subjective benefit in several cases (Table 9). These included the patients overall wellbeing being improved, a palpable tumor feeling softer and/or smaller and symptoms caused by the tumor being relieved. Of importance is that two patients, previously suffering from rapid accumulation of ascites and/or pleural effusion, had a clear reduction of their accumulation after the virus treatment, this effect lasted for several months in both cases.

Overall, signs of antitumor efficacy were seen in 13/21 patients (62%).

### VIII. Hematological effects of the treatment

Levels of leucocytes, erythrocytes, Hb, thrombocytes, bilirubin, INR, ALT, AST, ALP, creatinine, K, Na, CRP, CA19-9, GT, Fibrin D-dimers and CEA were studied after Ad5/3-D24-GM-CSF treatment (Tables 3-4).

### IX. Immune response to virus

### a) IL-6, IL-10, TNF-α and IL-8

One potential drawback of adenovirus gene therapy is its early toxicity due to viral components which may be immunogenic and can lead to a sepsis-like shock and even death (Brunetti-Pierri et al. Gene Ther 2004; Raper et al 2002). It is, therefore, extremely important to monitor signs for a possible cytokines storm which may later evolve in organ failure. To this end, soon after the treatment and at indicated time point blood was drawn from patients and pro-inflammatory cytokines were analyzed by FACSARRAY as described in the article of Cerullo V et al. (2007, Mol Ther 15, 378-85). No significant changes were seen in the patients treated with Ad5-D24-GM-CSF indicating lack of early innate toxicity.

For the results of lack of early innate toxicity related to Ad5/3-D24-GM-CSF see Table 10.

### b) Induction of cytotoxic T-cells against tumors and specific immunity against tumor epitope

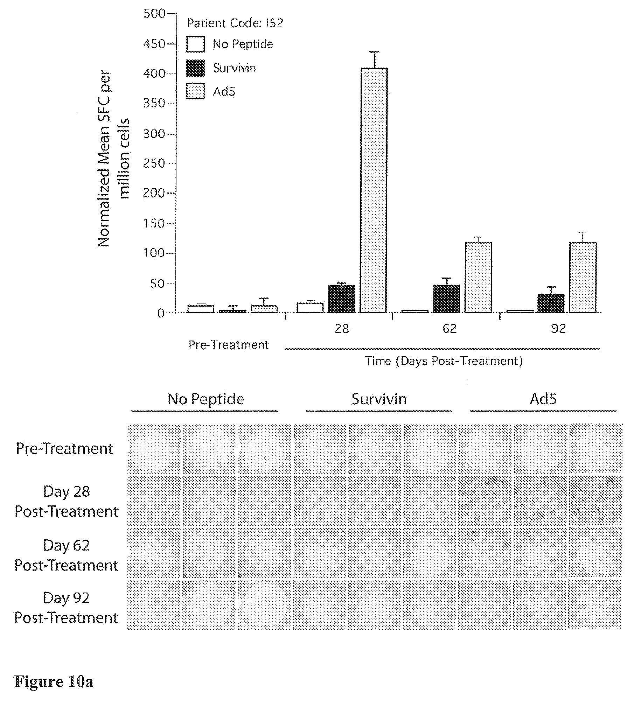

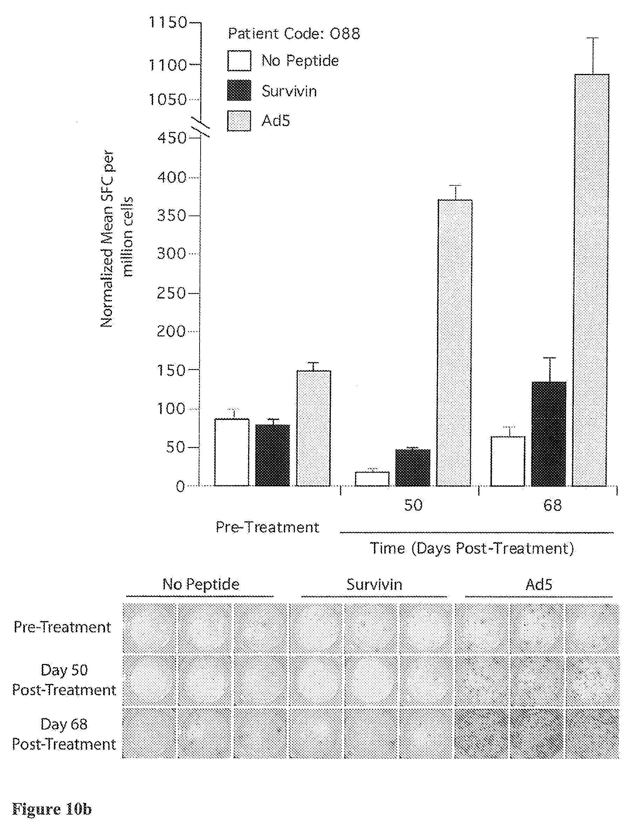

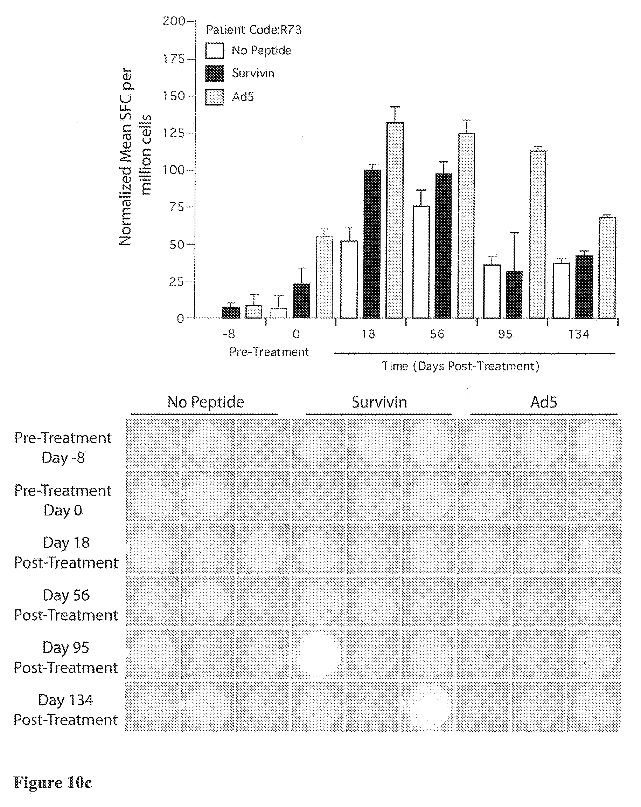

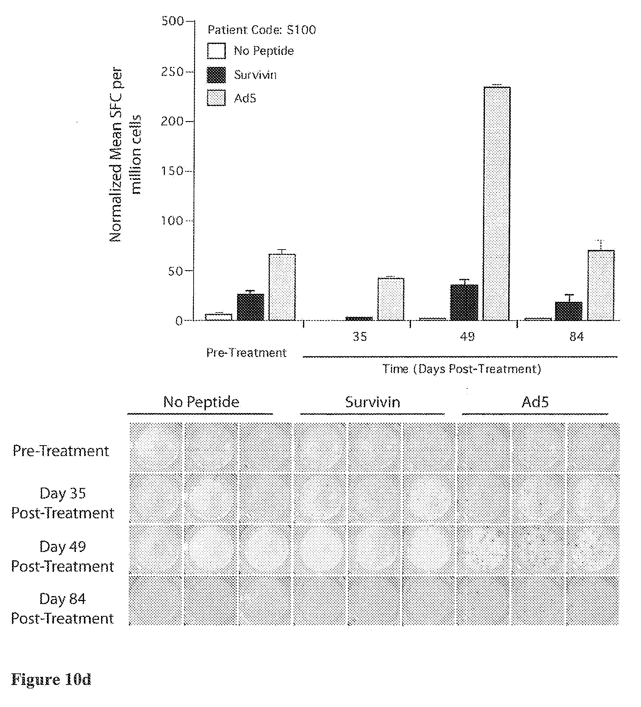

Oncolytic cell death allows the immune system to gain the capacity for recognizing and killing tumor cells. This is potentially beneficial for tumor eradication and may facilitate cures. Adenovirus is cleared out from the body in a relative short time following the administration; hence it becomes of key importance to stimulate the immune system to be able to recognize specific tumor-antigen so that the treatment can result in a sustained beneficial effect for the patient. In addition, in the presence of antibody, the virus is neutralized so that it can lose its efficacy of infecting metastasis. However, effector T or NK cells induced against the tumor are free to circulate and eventually kill metastasis far from the injected tumor. In order to demonstrate that the GMCSF-expressing adenovirus is able to elicit adenovirus- and tumor specific immunity, PBMCs collected from treated patients were analyzed by INF-gamma ELISPOT. The ELISPOT was performed in a blinded fashion way by an external company which was not provided of information on any kind of treatment the patients underwent through (Proimmune). In figures 10a-d are illustrated the results from such analysis. It is clear that in same patients, when the T cells were stimulated with a pool of peptide derived from either tumor antigen (survivin) or adenovirus (penton) these cells were activated hence produce INF-gamma (IFN-gamma is a specific activation marker of stimulated T cells).

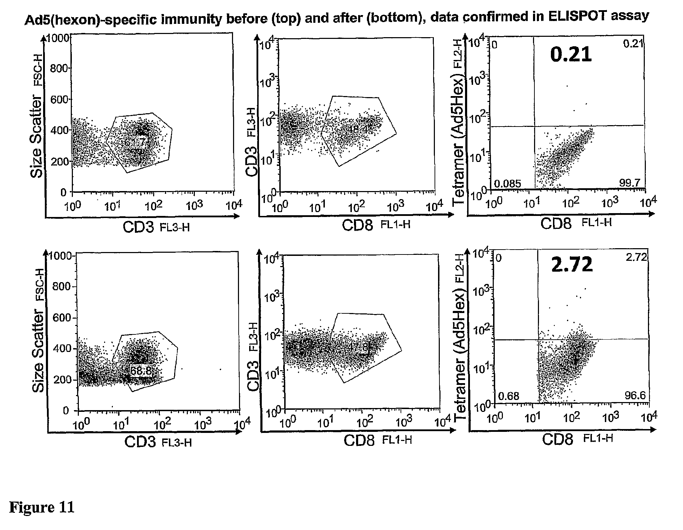

Figure 11 shows induction of Adenovirus Hexon-specific T cells. Leukocytes harvested from patients treated with Ad5-D24-GMCSF were stained with a CD3, CD8 and hexon-specific tetramer antibodies and analyzed by flow cytometry before and after the treatment. Treatment increased hexon specific cytotoxic T-cells from 0.21 to 2.72%.

### c) Reduction of regulatory T-cells

Previous data has demonstrated that metronomic administration of cyclophosphamide reduces regulatory T cells (T-Reg) in laboratory animals.

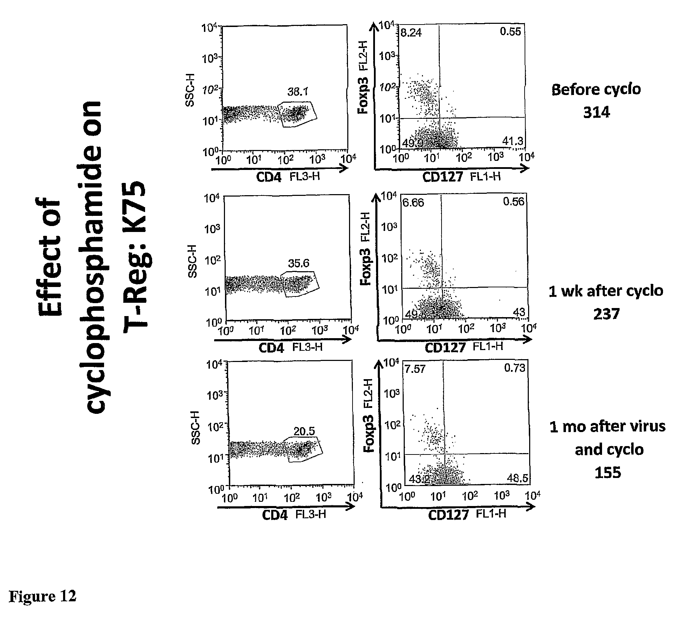

This approach was utilized in patients who received metronomic administration of cyclophoshamide before and after the Ad5-D24-GM-CSF treatment and T reg analysis was performed on PBMCs harvested from these patients. In the example illustrated in Figure 12 it is shown one example from patient R73 and it shows a reduction in circulating T reg. Total PBMCs were harvested from the patients and frozen in appropriate media. At the time of analysis all the samples were thawed and stained first with CD4 and CD127 antibodies, following the cells were permeabilized and stained for the transcription factor Foxp3. Cells that resulted positive for CD4, negative for CD127 but high in Foxp3 are considered effective T regulatory cells (T reg) (Figure 12).

### Example 7. Statistical analysis

Two tailed Student's t-test was used to compare luciferase activity and pre- and post-treatment neutralizing antibody titers, cytokine levels and GM-CSF concentrations. Survival data was processed with Kaplan-Meier analysis.

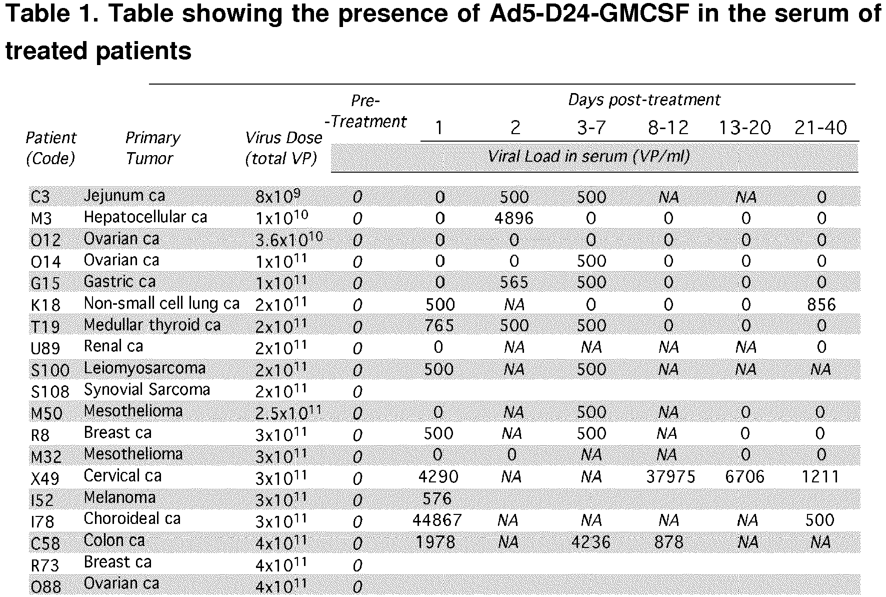

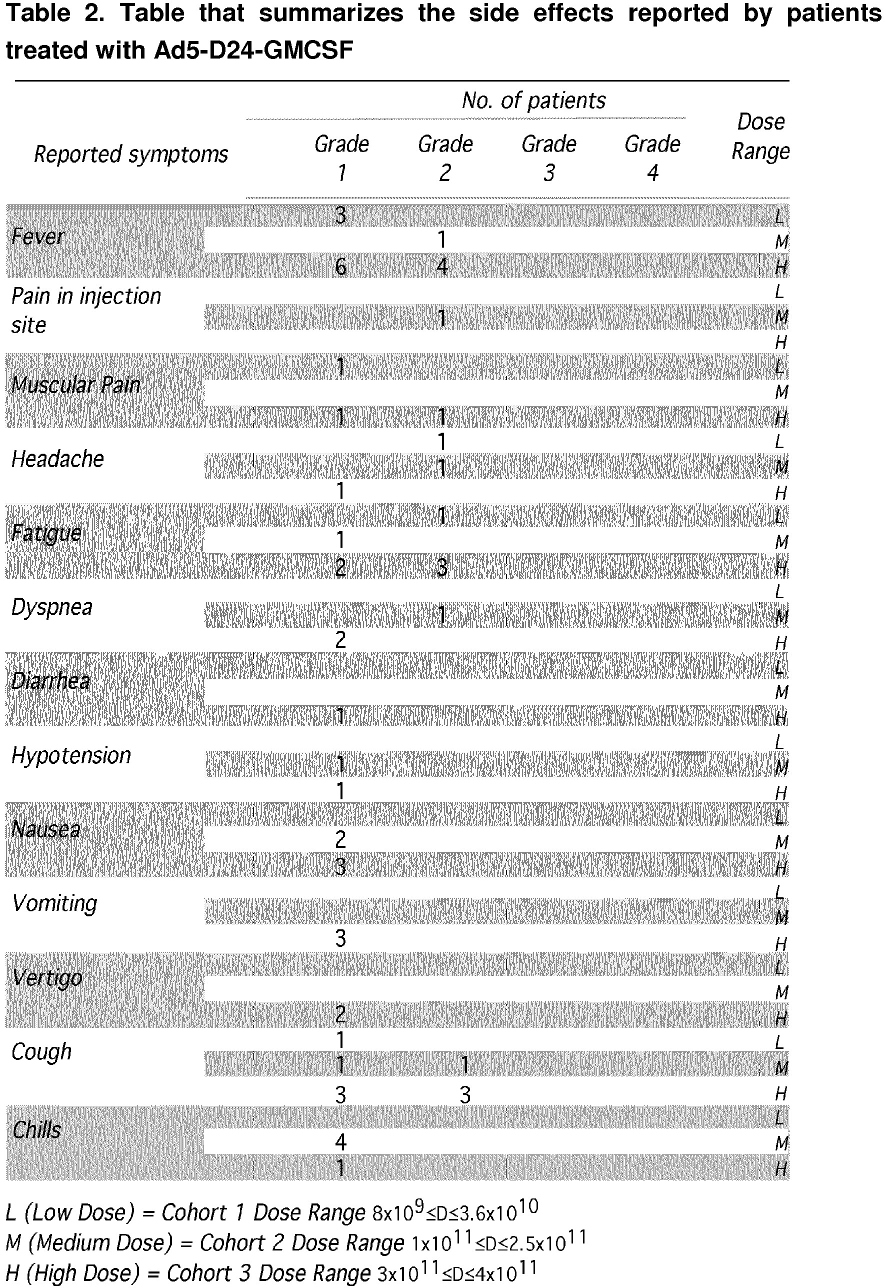

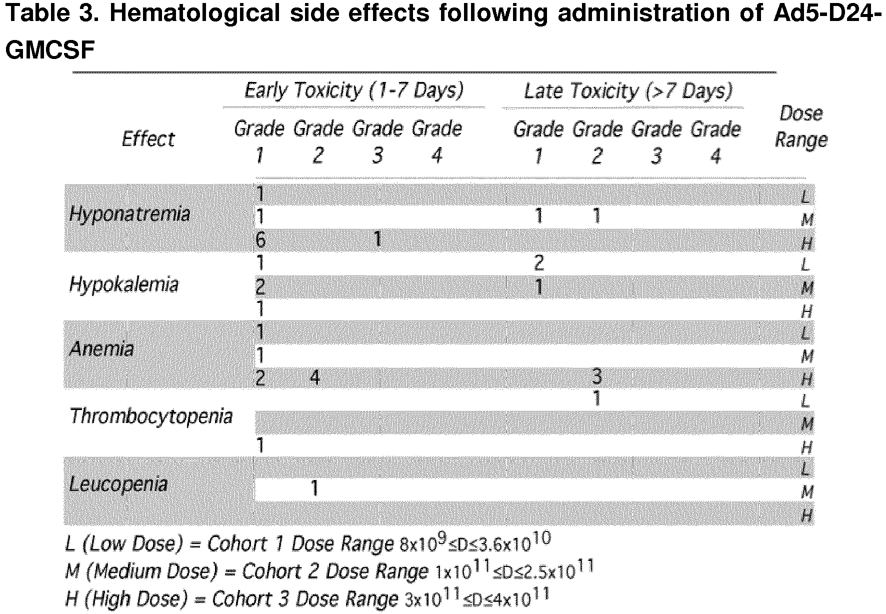

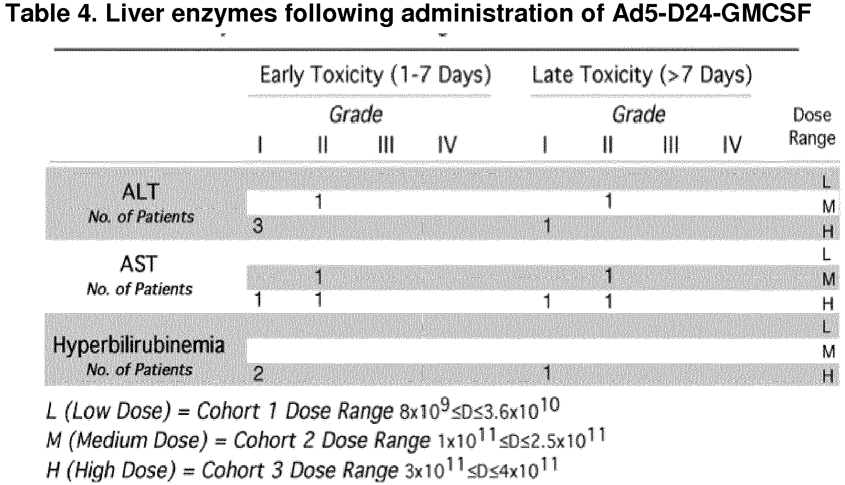

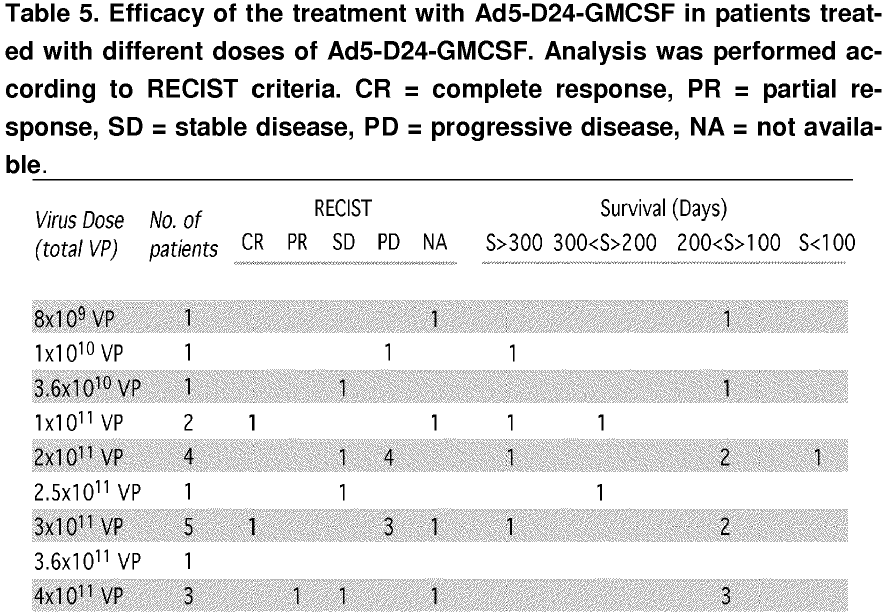

**Table 6. Summary of characteristics of patients for Ad5/3-D24-GM-CSF treatment at baseline. The numbers indicate number of patients out of 22.**

| **Sex** | ***N. of Patients*** |
|---|---|
| Male | 11 |
| Female | 11 |

| **WHO performance score** | |
|---|---|
| Median | 1 |
| Range | 0-3 |

| **Tumor Type** | |
|---|---|
| Cholangio carcinoma | 1 |
| Pancreatic cancer | 3 |
| Colorectal cancer | 2 |
| Prostatic cancer | 2 |
| Lung cancer | 1 |
| Ovarian cancer | 4 |
| Melanoma (choroidea or skin) | 3 |
| Head and neck squamous cell cancer | 1 |
| Sarcoma (bone, synovia or chondro) | 3 |
| Uterus cancer | 1 |
| Bladder cancer | 1 |
| Mesothelioma | 1 |

| **Previous treatments** | |
|---|---|
| Chemotherapy | 22 |
| Median chemo regimens per patient | |
| Radiotherapy | 6 |
| Hormone treatments | 1 |
| Surgical treatments | 15 |

| **Ad5/3 Neutralizing antibodies (baseline)** | |
|---|---|
| Positive | 11 |
| Negative | 4 |

| **Age** | **Years** |
|---|---|
| Median | 61 |
| Mean | 56 |
| Range | 17-78 |

**Table 7. Characteristcs of patients for Ad5/3-D24-GM-CSF treatment at baseline and description of treatment**

| **ID** | **Age Sex** | **Diagnosis** | **Prior therapies** | **WHO^{a}** | **Cyclo-fosfamide^{d}** | **Dose (VP)^{b}** | **Route^{c}** |
|---|---|---|---|---|---|---|---|
| Y62 | 55 M | Cholangiocarcinoma and clear cell hypernefroma. Metastases to liver and neck. | Gemcitabine | 2 | + | 1 x 10¹¹ | ¾ i.t. liver |
| | | | | | | | met., neck, kidney |
| | | | | | | | ¼ i.v. |
| H64 | 54 F | Pancreatic carcinoma. Metastases to liver. | Operation, gemcitabine. | 0 | - | 8 x 10¹⁰ | ¾ i.t. liver met. |
| | | | | | | | ¼ i.v. |
| C66 | 63 F | Colon carcinoma. Metastases to liver, lungs, lymph nodes. | Operation, leucovorin, oxaliplatin, 5-fluorouracil and bevacizumab | 0 | + | 2 x 10¹¹ | 2/3 i.t. liver |
| | | | | | | | met. |
| | | | | | | | 1/3 i.v. |
| S67 | 55 M | Chondrosarcoma of hip. | Operation, irradiation, cisplatin. | 1 | + | 2 x 10¹¹ | 2/3 i.t. hip 1/3 i.v |
| S70 | 24 F | Synovial sarcoma of thigh. Metastases to lungs | Operation x2, ifosfamide, doxorubicin, uromitexan, cisplatin, gemcitabine, sorafenib, etoposide. | 2 | - | 1 x 10¹¹ | 1/10 i.pl |
| | | | | | | | 6/10 i.t. lungs |
| | | | | | | | 3/10 i.v. |
| P74 | 55 M | Prostate carcinoma. Metastases to bones | LHRH-analogues, docetaxel, irradiation, orchiectomy operation. | 3 | + | 1 x 10¹¹ | i.v. |
| K75 | 64 M | Lung adenocarcinoma. Metastases in pleura and peritoneum | Operation, cisplatin, vinorelbine, erlotinib. | 1 | + | 3 x 10¹¹ | 9/16 i.p |
| | | | | | | | 4/16 i.v. |
| | | | | | | | 3/16 i.pl |
| 079 | 70 F | Ovarian carcinoma. Carcinomatosis. | Operation x3, docetaxel, carboplatin, paclitaxel, bevacizumab, gemcitabine, topotecan, talidomide, vinorelbine, doxorubicin. | 1 | + | 3 x 10¹¹ | 1/12 s.c. |
| | | | | | | | 4/12 i.t. |
| | | | | | | | 3/12 i.p. |
| | | | | | | | 4/12 i.v. |
| I80 | 38 F | Conjunctival melanoma. | Dacarbazine, interferon, paclitaxel, carboplatin, angioembolisation, irradiation. | 0 | + | 2 x 10¹¹ | 2/3 i.t. |
| | | Metastases to liver | | | | | 1/3 i.v. |
| O82 | 56 F | Ovarian carcinoma. | Paclitaxel, carboplatin, gemcitabine, topotecan, docetaxel, doxorubicine. | 2 | + | 3 x 10¹¹ | 10/31 i.v. |
| | | Carcinomatosis. | | | | | 20/31 i.t. |
| | | | | | | | 1/31 s.c. |
| H83 | 64 M | Pancreatic carcinoma | Gemcitabine | 1 | + | 4 x 10¹¹ | 2/5 i.v. |
| | | | | | | | 3/5 i.t. |
| I87 | 64 M | Melanoma Metastases to neck, parotis gland, liver. | Operation, irradiation, interferon, bevacizumab, dacarbazine, vincristine, lomustine, bleomycin | 1 | + | 2 x 10¹¹ | 2/3 i.t. |
| | | | | | | | 1/3 i.v. |
| C95 | 63 M | Rectum carcinoma. Metastases to liver. | Operation, bevacizumab, cabecitabine, irinotecan, cetuximab, oxaliplatin | 1 | - | 3 x 10¹¹ | 4/5 i.t. liver met |
| | | | | | | | 1/5 i.v. |
| H96 | 64 M | Pancreatic carcinoma. | Gemcitabine, erlotinib | 2 | + | 3 x 10¹¹ | 2/3 i.t. pancreas |
| | | | | | | | 1/3 i.v. |
| I98 | 38 F | Choroideal melanoma. Metastases to liver. | Rutenium-plate, dacarbazine, vincristine, lomustine, bleomycin, interferon, cisplatin, chemo-embolization | 1 | + | 3 x 10¹¹ | 1/2 i.t. liver met. 1/2 i.v. |
| N110 | 60 M | Head and neck carcinoma. | Operation, cisplatin, 5-fluorouracil, boron neuron capture therapy. | 2 | + | 2 x 10¹¹ | 3/10 i.v. 7/10 i.t. |
| O113 | 67 F | Ovarian carcinoma. Carcinomarsinosis. | Operation, paclitaxel, carcinomarboplatin, cisplatin, doxorubicine, gemcitabine, topotecarcinomane, vinorelbine, docetaxel. | 1 | + | 3 x 10¹¹ | ¾ i.t. pelvic tumor + met ¼ i.v. |
| S119 | 17 M | Sarcoma of limb. | Docetaxel, cisplatin, gemcitabin, ifosfamide, doxorubicine, uromitexane. | 1 | + | 4 x 10¹¹ | 2/3 i.t. limb |
| | | Metastases to lungs. | | | | | 1/3 i.v. |
| X122 | 77 F | Uterus carcinoma. | Operation, carboplatin, epirubicin, doxorubicin, brachytherapy. | 1 | - | 3 x 10¹¹ | ¾ i.t. |
| | | Peritoneal metastases. | | | | | ¼ i.v. |
| O129 | 48 F | Ovarian carcinoma. | Paclitaxel, docetaxel, carboplatin, cisplatin, gemcitabine, doxorubicin. | 0 | - | 3 x 10¹¹ | 1/4 i.v. |
| | | Carcinomarsinosis. | | | | | 1/4 i.t. |
| | | | | | | | 2/4 i.p. |
| V136 | 78 M | Bladder and prostate | Operation, irradiation, cisplatin, gemcitabine, procarbazine, vincristin, ranimustin. | 2 | + | 3 x 10¹¹ | 1/2 i.v. |
| | | carcinomas. Lymph node, bone and lung metastases. | | | | | 1/2 i.pl |
| M137 | 65 F | Mesothelioma | Carboplatin, pemetrexed. | 1 | + | 3 x 10¹¹ | 5/20 i.v. |
| | | | | | | | 13/20 i.pl |
| | | | | | | | 2/20 i.t. |
| ^{a}Performance status at the time of treatment, scale 0-5. | | | | | | | |
| ^{b}Total dose; vp=viral particles | | | | | | | |
| ^{c}i.v.=intravenous, i.p.=intraperitoneal, i.t.=intratumoral, i.pl.= intrapleura, met.=metastases | | | | | | | |
| ^{d}concurrent metronomic cyclophosphamide 50 mg/d was given orally in the absence of contraindications | | | | | | | |

**Table 8. Adverse events. All 22 patients were evaluated for adverse events (AE) according to CTCEA v.3.0 criteria for 4 weeks after Ad5/3-D24-GMCSF treatment. Grade 1 AE reported only if present in 2 or more patients. All grade 2-5 AE are reported. Numbers indicate the number of patients out of 22.**

| | | **Grade 1** | **Grade 2** | **Grade 3** | **Grade 4-5** |
|---|---|---|---|---|---|
| **Constitutional** | | | | | |
| | Chills | 3 | 1 | 0 | 0 |
| | Fatigue | 5 | 12 | 0 | 0 |
| | Fever | 13 | 6 | 0 | 0 |
| | Sweating | 1 | 1 | 0 | 0 |
| **Gastrointestinal** | | | | | |
| | Anorexia | 1 | 1 | 0 | 0 |
| | Nausea | 8 | 1 | 0 | 0 |
| | Vomiting | 4 | 0 | 0 | 0 |
| | Heartburn | 2 | 0 | 0 | 0 |
| **Haematological** | | | | | |
| | Anemia | 3 | 0 | 1 | 0 |
| | Neutropenia | 0 | 0 | 1 | 0 |
| | Thrombocytopenia | 0 | 1 | 0 | 0 |
| **Infection** | | | | | |
| | Cholecystitis | 0 | 0 | 1 | 0 |
| **Lymphatics** | | | | | |
| | Limb edema | 0 | 2 | 0 | 0 |
| **Metabolic / Laboratory** | | | | | |
| | ALT increased | 2 | 0 | 1 | 0 |
| | AST increased | 2 | 2 | 1 | 0 |
| | Hyperbilirubinemia | 2 | 0 | 1 | 0 |
| | Hypokalemia | 3 | 0 | 0 | 0 |
| | Hyperkalemia | 0 | 1 | 0 | 0 |
| | Hyponatremia | 5 | 0 | 1 | 0 |
| | Glucose inbalance | 0 | 1 | 0 | 0 |
| **Neurology and Ocular** | | | | | |
| | Dizziness | 2 | 0 | 0 | 0 |
| **Pain** | | | | | |
| | Injection site | 4 | 2 | 0 | 0 |
| | Abdominal | 4 | 6 | 0 | 0 |
| | Joints | 1 | 2 | 0 | 0 |
| | Lower extremity | 1 | 1 | 0 | 0 |
| | Back | 1 | 1 | 0 | 0 |
| | Chest wall | 2 | 0 | 0 | 0 |
| | Head ache | 1 | 2 | 0 | 0 |
| | Others | 0 | 1 | 0 | 0 |
| **Pulmonary / Upper respiratory** | | | | | |
| | Nasal dripping | 3 | 0 | 0 | 0 |
| | Hoarseness | 0 | 1 | 0 | 0 |
| | Cough | 1 | 2 | 0 | 0 |
| **Other*** | | | | | |
| | Erythrocytopenia | 5 | 0 | 0 | 0 |
| | Relative lymphocytopenia | 3 | 0 | 0 | 0 |
| | Relative lymphocytosis | 2 | 0 | 0 | 0 |
| | Leucosytosis | 3 | 0 | 0 | 0 |
| | Thrombocytosis | 3 | 0 | 0 | 0 |
| ALT , alanine aminotransferase; AST, aspartate aminotransferase; | | | | | |
| * Nor gradeable as adverse events in CTCEA v3.0 criteria | | | | | |

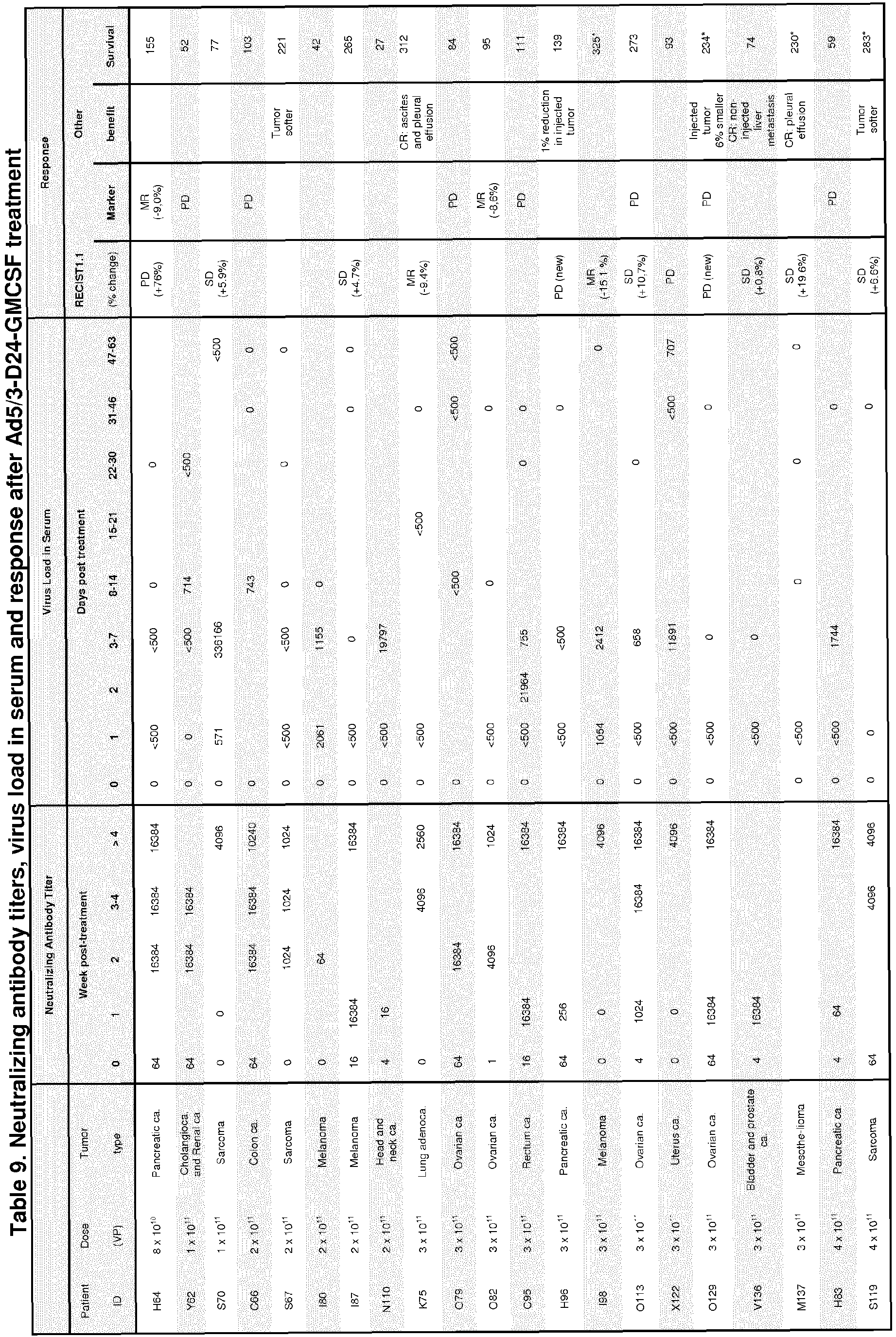

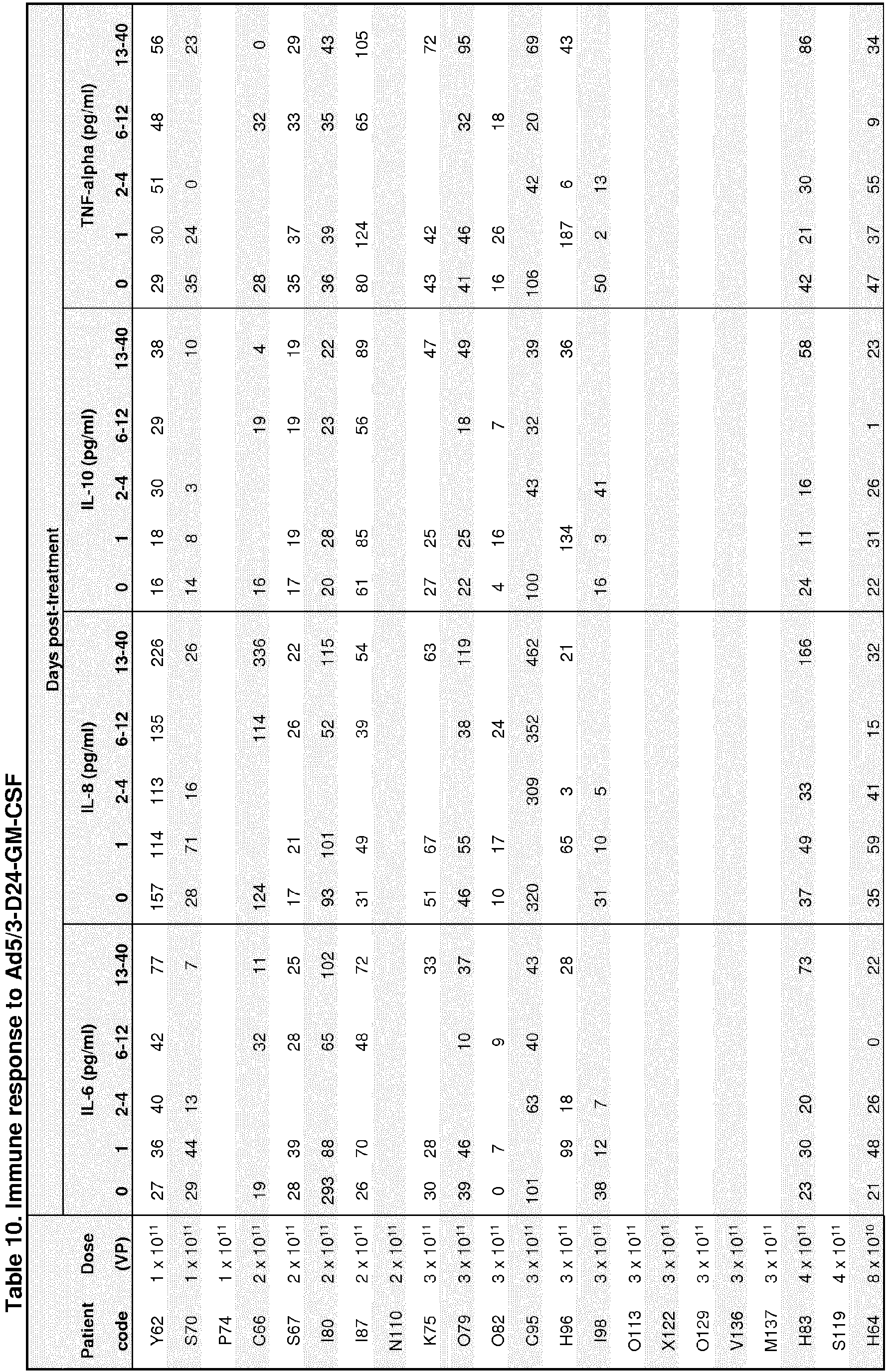

## Claims

1. An oncolytic adenoviral vector comprising an adenovirus serotype 5 (Ad5) nucleic acid backbone, a native E1 A promoter, a 24 bp deletion (D24) in the Rb binding constant region 2 of E1, a nucleic acid sequence encoding a human granulocyte-macrophage colony-stimulating factor (GM-CSF) in the place of the deleted gp19k/6.7K in the E3 region and a capsid modification, wherein the capsid modification is Ad5/3 chimerism, insertion of an integrin binding (RGD) region and/or heparin sulphate binding polylysine modification into the fiber.

2. An oncolytic adenoviral vector according to claim 1, wherein the capsid modification is Ad5/3 chimerism, wherein the knob part of the fiber is from Ad serotype 3 and the rest of the fiber is from Ad serotype 5.

3. An oncolytic adenoviral vector according to claim 1, wherein the capsid modification is an RGD-4C modification.

4. An oncolytic adenoviral vector according to any one of the previous claims comprising at least one expression cassette.

5. An oncolytic adenoviral vector according to any one of the previous claims, wherein the vector is capable of selectively replicating in cells having defects in the Rb-pathway.

6. A cell comprising the adenoviral vector according to any one of claims 1-5.

7. A pharmaceutical composition comprising the adenoviral vector according to any one of claims 1-5.

8. An oncolytic adenoviral vector according to any one of claims 1-5 or a pharmaceutical composition according to claim 7 for use as an *in situ* cancer vaccine.

9. An adenoviral vector according to any one of claims 1-5 for use in the treatment of cancer or in the manufacture of a medicament for treating cancer in a subject.

10. An adenoviral vector for use according to claim 9, wherein the cancer is selected from a group consisting of nasopharyngeal cancer, synovial cancer, hepatocellular cancer, renal cancer, cancer of connective tissues, melanoma, lung cancer, bowel cancer, colon cancer, rectal cancer, colorectal cancer, brain cancer, throat cancer, oral cancer, liver cancer, bone cancer, pancreatic cancer, choriocarcinoma, gastrinoma, pheochromocytoma, prolactinoma, T-cell leukemia/lymphoma, neuroma, von Hippel-Lindau disease, Zollinger-Ellison syndrome, adrenal cancer, anal cancer, bile duct cancer, bladder cancer, ureter cancer, brain cancer, oligodendroglioma, neuroblastoma, meningioma, spinal cord tumor, bone cancer, osteochondroma, chondrosarcoma, Ewing's sarcoma, cancer of unknown primary site, carcinoid, carcinoid of gastrointestinal tract, fibrosarcoma, breast cancer, Paget's disease, cervical cancer, colorectal cancer, rectal cancer, esophagus cancer, gall bladder cancer, head cancer, eye cancer, neck cancer, kidney cancer, Wilms' tumor, liver cancer, Kaposi's sarcoma, prostate cancer, lung cancer, testicular cancer, Hodgkin's disease, non-Hodgkin's lymphoma, oral cancer, skin cancer, mesothelioma, multiple myeloma, ovarian cancer, endocrine pancreatic cancer, glucagonoma, pancreatic cancer, parathyroid cancer, penis cancer, pituitary cancer, soft tissue sarcoma, retinoblastoma, small intestine cancer, stomach cancer, thymus cancer, thyroid cancer, trophoblastic cancer, hydatidiform mole, uterine cancer, endometrial cancer, vagina cancer, vulva cancer, acoustic neuroma, mycosis fungoides, insulinoma, carcinoid syndrome, somatostatinoma, gum cancer, heart cancer, lip cancer, meninges cancer, mouth cancer, nerve cancer, palate cancer, parotid gland cancer, peritoneum cancer, pharynx cancer, pleural cancer, salivary gland cancer, tongue cancer, tonsil cancer.

11. An adenoviral vector for use according to claim 9 or 10, wherein the subject is a human or an animal.

12. An adenoviral vector for use according to any one of claims 9-11, wherein the treatment of cancer in a subject is conducted through an intratumoral, intramuscular, intra-arterial, intravenous, intrapleural, intravesicular, intracavitary or peritoneal injection, or an oral administration.

13. An adenoviral vector for use according to claim 11, wherein the injection or the administration is conducted several times during the treatment period, or wherein an oncolytic adenoviral vector having a different fiber knob of the capsid compared to the vector of the earlier treatment is used.

14. An adenoviral vector for use according to any one of claims 9-13, wherein the treatment of cancer in a subject further comprises an administration of concurrent radiotherapy and/or concurrent chemotherapy to a subject.

15. An adenoviral vector for use according to any one of claims 9-14, wherein the treatment of cancer in a subject further comprises an administration of verapamil or another calcium channel blocker, autophagy inducing agents, temozolomide, chemotherapy or anti-CD20 therapy or other approaches for blocking of neutralizing antibodies, substances capable to downregulating regulatory T-cells in a subject, and/or cyclophosphamide to a subject.

16. A method of producing GM-CSF in a cell *in vitro,* wherein the method comprises:
a) carrying a vehicle comprising an oncolytic adenoviral vector according to any one of claims 1-5 to a cell, and
b) expressing GM-CSF of said vector in the cell.

17. A use of the oncolytic adenoviral vector according to any one of claims 1-5 for producing GM-CSF in a cell *in vitro.*

## Patentansprüche

1. Onkolytischer adenoviraler Vektor umfassend ein Nukleinsäure-Backbone von Adenovirus Serotyp 5 (Ad5), einen nativen E1A-Promoter, eine 24bp-Deletion (D24) in der Rb-bindenden konstanten Region 2 von E1, eine Nukleinsäuresequenz, die einen humanen Granulozyten-Makrophagen-Kolonie-stimulierenden Faktor (GM-CSF) an Stelle des deletierten gp19k/6.7K in der E3-Region kodiert, und eine Kapsidmodifikation, wobei die Kapsidmodifikation ein Ad5/3-Chimärismus, Einbau einer Integrin-bindenden (RGD) Region und/oder einer Heparinsulfat-bindenden Polylysinmodifikation in die Faser ist.

2. Onkolytischer adenoviraler Vektor nach Anspruch 1, wobei die Kapsidmodifikation ein Ad5/3-Chimärismus ist, wobei der Knob-Teil der Fiber vom Ad Serotyp 3 und der Rest der Fiber vom Ad Serotyp 5 ist.

3. Onkolytischer adenoviraler Vektor nach Anspruch 1, wobei die Kapsidmodifikation eine RGD-4C-Modifikation ist.

4. Onkolytischer adenoviraler Vektor nach einem der vorhergehenden Ansprüche, der wenigstens eine Expressionskassette umfasst.

5. Onkolytischer adenoviraler Vektor nach einem der vorhergehenden Ansprüche, wobei der Vektor die Fähigkeit zur selektiven Replikation in Zellen mit Defekten im Rb-Signalweg aufweist.

6. Zelle, die den adenoviralen Vektor nach einem der Ansprüche 1 bis 5 umfasst.

7. Pharmazeutische Zusammensetzung, die den adenoviralen Vektor nach einem der Ansprüche 1 bis 5 umfasst.

8. Onkolytischer adenoviraler Vektor nach einem der Ansprüche 1 bis 5 oder pharmazeutische Zusammensetzung nach Anspruch 7 zur Verwendung in einem *in*-*situ-*Krebsimpfstoff.

9. Adenoviraler Vektor nach einem der Ansprüche 1 bis 5 zur Verwendung bei der Behandlung von Krebs oder bei der Herstellung eines Medikaments zur Behandlung von Krebs bei einem Patienten.

10. Adenoviraler Vektor zur Verwendung nach Anspruch 9, wobei der Krebs gewählt ist aus einer Gruppe bestehend aus Nasenrachenkrebs, Synovialkrebs, Hepatozellularkrebs, Nierenkrebs, Bindegewebekrebs, Melanomen, Lungenkrebs, Darmkrebs, Dickdarmkrebs, Mastdarmkrebs, Kolorektalkrebs, Hirnkrebs, Kehlkopfkrebs, Mundhöhlenkrebs, Leberkrebs, Knochenkrebs, Bauchspeicheldrüsenkrebs, Choriokarzinom, Gastrinom, Phäochromozyotom, Prolaktinom, T-Zellen-Leukämie/Lymphom, Neurom, von-Hippel-Lindau-Erkrankung, Zollinger-Ellison-Syndrom, Nebennierenkrebs, Analkrebs, Gallenwegskrebs, Blasenkrebs, Harnleiterkrebs, Hirnkrebs, Oligodendrogliom, Neuroblastom, Meningiom, Rückenmarktumor, Knochenkrebs, Osteochondrom, Chondrosarkom, Ewing-Sarkom, Krebs unbekannter Primärlokalisation, Karzinoid, Karzinoid des Magen-Darm-Traktes, Fibrosarkom, Brustkrebs, Paget-Krankheit, Gebärmutterhalskrebs, Kolorektalkrebs, Mastdamkrebs, Speiseröhrenkrebs, Gallenblasenkrebs, Kopfkrebs, Augenkrebs, Halskrebs, Nierenkrebs, Wilms-Tumor, Leberkrebs, Kaposisarkom, Prostatakrebs, Lungenkrebs, Hodenkrebs, Hodgkinsche Krankheit, Non-Hodgkin-Lymphom, Mundhöhlenkrebs, Hautkrebs, Mesotheliom, multiples Myelom, Eierstockkrebs, Krebs der endokrinen Bauchspeicheldrüse, Glucagonom, Beuchspeicheldrüsenkrebs, Nebenschilddrüsenkrebs, Peniskrebs, Hypophysenkrebs, Weichgewebesarkom, Retinoblastom, Dünndarmkrebs, Magenkrebs, Thymuskrebs, Schilddrüsenkrebs, Trophoblastenkrebs, Blasenmole, Gebärmutterkrebs, Endometriumkrebs, Vaginalkrebs, Vulvakrebs, Akustikusneurom, Mycosis Fungoides, Insulinom, Karzinoidsyndrom, Somatostatinom, Zahnfleischkrebs, Herzkrebs, Lippenkrebs, Hirnhautkrebs, Mundkrebs, Nervenkrebs, Gaumenkrebs, Ohrspeicheldrüsenkrebs, Bauchfellkrebs, Pharynxkrebs, Brustfellkrebs, Speicheldrüsenkrebs, Zungenkrebs, Mandelkrebs.

11. Adenoviraler Vektor zur Verwendung nach Anspruch 9 oder 10, wobei der Patient ein Mensch oder ein Tier ist.

12. Adenoviraler Vektor zur Verwendung nach einem der Ansprüche 9 bis 11, wobei die Behandlung von Krebs bei einem Patienten über eine intratumorale, intramuskuläre, intraarterielle, intravenöse, intrapleurale, intravesikale, intrakavitäre oder peritoneale Injektion oder eine orale Verabreichung durchgeführt wird.

13. Adenoviraler Vektor zur Verwendung nach Anspruch 11, wobei die Injektion oder Verabreichung mehrere Male während des Behandlungszeitraums erfolgt oder wobei ein onkolytischer adenoviraler Vektor verwendet wird, der einen anderen Fiberknob des Kapsids aufweist als der Vektor der früheren Behandlung.

14. Adenoviraler Vektor zur Verwendung nach einem der Ansprüche 9 bis 13, wobei die Behandlung von Krebs bei einem Patienten ferner umfasst, dass einem Patienten eine gleichzeitige Radiotherapie und/oder eine gleichzeitige Chemotherapie verabreicht wird.

15. Adenoviraler Vektor zur Verwendung nach einem der Ansprüche 9 bis 14, wobei die Behandlung von Krebs bei einem Patienten ferner umfasst, dass einem Patienten Verapamil oder ein anderer Calciumkanalblocker, Autophagie-induzierende Mittel, Temozolomid, Chemotherapie oder Anti-CD20-Therapie oder andere Verfahren zur Blockierung neutralisierender Antikörper, Substanzen mit Fähigkeit zu herunterregulierenden regulatorischen T-Zellen in einem Patienten und/oder Cyclophosphamid verabreicht werden.

16. Verfahren zur *in*-vitro-Herstellung von GM-CSF in einer Zelle, wobei das Verfahren umfasst:
a) Führen eines Vehikels, das einen onkolytischen adenoviralen Vektor nach einem der Ansprüche 1 bis 5 umfasst, zu einer Zelle und
b) Expression des GM-CSF des Vektors in der Zelle.

17. Verwendung des onkolytischen adenoviralen Vektors nach einem der Ansprüche 1 bis 5 zur *in*-*vitro*-Herstellung von GM-CSF in einer Zelle.

## Revendications

1. Vecteur adénoviral oncolytique comprenant un squelette de l'acide nucléique de l'adénovirus de sérotype 5 (Ad5), un promoteur natif E1A, une délétion 24bp (D24) dans la région constante de liaison à Rb 2 de E1, une séquence d'acide nucléique codant un facteur de stimulation des colonies de granulocytes et de macrophages (GM-CSF) humain à la place du gp19k/6.7K délété dans la région E3 et une modification de la capside, ladite modification de la capside étant un chimérisme Ad5/3, insertion d'une région de liaison à intégrines (RGD) et/ou d'une modification polylysine de liaison à l'héparine sulfate dans la fibre.

2. Vecteur adénoviral oncolytique selon la revendication 1, ladite modification de la capside étant un chimérisme Ad5/3, la partie knob de la fibre étant du sérotype Ad3 et le reste de fibre étant du sérotype Ad5.

3. Vecteur adénoviral oncolytique selon la revendication 1, ladite modification de la capside étant une modification RGD-4C.

4. Vecteur adénoviral oncolytique selon l'une des revendications précédentes, comprenant au moins une cassette d'expression.

5. Vecteur adénoviral oncolytique selon l'une des revendications précédentes, ledit vecteur étant capable de réplication sélective dans des cellules ayant des anomalies dans la voie Rb.

6. Cellule comprenant le vecteur adénoviral selon l'une des revendications 1 à 5.

7. Composition pharmaceutique comprenant le vecteur adénoviral selon l'une des revendications 1 à 5.

8. Vecteur adénoviral oncolytique selon l'une des revendications 1 à 5 ou composition pharmaceutique selon la revendication 7 pour l'utilisation dans un vaccin anticancéreux *in situ.*

9. Vecteur adénoviral selon l'une des revendications 1 à 5 pour l'utilisation dans le traitement d'un cancer ou dans la fabrication d'un médicament pour le traitement d'un cancer chez un patient.

10. Vecteur adénoviral pour l'utilisation selon la revendication 9, le cancer étant choisi dans le groupe constitué par le cancer nasopharyngien, le cancer synovial, le cancer hépatocellulaire, le cancer rénal, le cancer des tissus conjonctifs, le mélanome, le cancer du poumon, le cancer de l'intestin, le cancer du côlon, le cancer rectal, le cancer colorectal, le cancer du cerveau, le cancer de la gorge, le cancer buccal, le cancer du foie, le cancer des os, le cancer pancréatique, le choriocarcinome, le gastrinome, le phéochromocyotome, le prolactinome, la leucémie à cellules T/lymphome, le neurome, la maladie de von Hippel-Lindau, le syndrome de Zollinger-Ellison, le cancer des glandes surrénales, le cancer de l'anus, le cancer de la voie biliaire, le cancer de la vessie, le cancer de l'uretère, le cancer du cerveau, l'oligodendrogliome, le neuroblastome, le méningiome, la tumeur à la moelle épinière, le cancer des os, l'ostéochondrome, le chondrosarcome, le sarcome d'Ewing, le cancer primitif inconnu, le carcinoïde, le carcinoïde du tractus gastro-intestinal, le fibrosarcome, le cancer du sein, la maladie Paget, le cancer du col de l'utérus, le cancer colorectal, le cancer rectal, le cancer de l'oesophage, le cancer de la vésicule biliaire, le cancer de la tête, le cancer de l'oeil, le cancer du cou, le cancer du rein, la tumeur de Wilms, le cancer du foie, le sarcome de Kaposi, le cancer de la prostate, le cancer du poumon, le cancer des testicules, la maladie de Hodgkin, le lymphome non hodgkinien, le cancer buccal, le cancer de la peau, le mésothéliome, le myélome multiple, le cancer de l'ovaire, le cancer du pancréas endocrine, le glucagonome, le cancer pancréatique, le cancer parathyroïdien, le cancer du pénis, le cancer de l'hypophyse, la sarcome des tissus mous, le rétinoblastome, le cancer de l'intestin grêle, le cancer de l'estomac, le cancer du thymus, le cancer de la thyroïde, le cancer trophoblastique, la môle hydatiforme, le cancer de l'utérus, le cancer de l'endomètre, le cancer du vagin, le cancer de la vulve, le neurinome de l'acoustique, la mycose fongoïde, l'insulinome, le syndrome carcinoïde, le somatostatinome, le cancer de la gencive, le cancer du coeur, le cancer de la lèvre, le cancer des méninges, le cancer de la bouche, le cancer des nerfs, le cancer du palais, le cancer de la glande parotide, le cancer du péritoine, le cancer du pharynx, le cancer de la plèvre, le cancer de la glande salivaire, le cancer de la langue, le cancer de l'amygdale.

11. Vecteur adénoviral pour l'utilisation selon la revendication 9 ou 10, ledit patient étant un humain ou un animal.

12. Vecteur adénoviral pour l'utilisation selon l'une des revendications 9 à 11, ledit traitement d'un cancer chez un patient étant effectué par une injection intratumorale, intramusculaire, intraartérielle, intraveineuse, intrapleurale, intravésiculaire, intracavitaire ou péritonéale ou par administration orale.

13. Vecteur adénoviral pour l'utilisation selon la revendication 11, ladite injection ou administration étant effectuée à plusieurs reprises pendant la période de traitement ou un vecteur adénoviral oncolytique avec un knob de fibre de la capside différent de celui du vecteur du traitement antérieur étant utilisé.

14. Vecteur adénoviral pour l'utilisation selon l'une des revendications 9 à 13, ledit traitement d'un cancer chez un patient comprenant également l'administration d'une radiothérapie concurrente et/ou d'une chimiothérapie concurrente à un patient.

15. Vecteur adénoviral pour l'utilisation selon l'une des revendications 9 à 14, ledit traitement d'un cancer chez un patient comprenant également une administration, à un patient, de vérapamil ou d'un autre bloqueur des canaux calciques, d'agents inducteurs d'autophagie, de témozolomide, d'une chimiothérapie ou d'une thérapie anti-CD20 ou d'autres approches visant l'inhibition d'anticorps neutralisants, de substances capables de cellules T régulatrices à régulation négative chez un patient et/ou de cyclophosphamide.

16. Procédé de production *in vitro* de GM-CSF dans une cellule, ledit procédé comprenant:
a) transport d'un véhicule comprenant un vecteur adénoviral oncolytique selon l'une des revendications 1 à 5 dans une cellule et
b) expression du GM-CSF dudit vecteur dans la cellule.

17. Utilisation du vecteur adénoviral oncolytique selon l'une des revendications 1 à 5 pour la production *in vitro* de GM-CSF dans une cellule.


### SEQUENCE LISTING

<110> Oncos Therapeutics Oy
<120> Adenoviral vectors and methods and uses related thereto
<130> 2090342PC
<160> 10
<170> PatentIn version 3.5
<210> 1
   <211> 21
   <212> DNA
   <213> Artificial
<220>
   <223> oligonucleotide
<400> 1
   tccggtttct atgccaaacc t 21
<210> 2
   <211> 22
   <212> DNA
   <213> Artificial sequence
<220>
   <223> oligonucleotide
<400> 2
   tcctccggtg ataatgacaa ga 22
<210> 3
   <211> 19
   <212> DNA
   <213> Artificial sequence
<220>
   <223> oligonucleotide
<400> 3
   tgatcgatcc acccagtga 19
<210> 4
   <211> 16
   <212> DNA
   <213> Artificial sequence
<220>
   <223> oligonucleotide
<400> 4
   tacctgccac gaggct 16
<210> 5
   <211> 21
   <212> DNA
   <213> Artificial sequence
<220>
   <223> oligonucleotide
<400> 5
   aaacaccacc ctccttacct g 21
<210> 6
   <211> 21
   <212> DNA
   <213> Artificial sequence
<220>
   <223> oligonucleotide
<400> 6
   tcattcatct cagcagcagt g 21
<210> 7
   <211> 35440
   <212> DNA
   <213> Artificial Sequence
<220>
   <223> Ad5/3-D24-GMCSF
<220>
   <221> misc_feature
   <222> (5754)..(5756)
   <223> n is a, c, g, or t
<220>
   <221> misc_feature
   <222> (33162)..(33162)
   <223> n is a, c, g, or t
<220>
   <221> misc_feature
   <222> (34851)..(34851)
   <223> n is a, c, g, or t
<400> 7
   taacatcatc aattatacct tccattttgg attgaagcca atatgataat gagggggtgg      60
   agtttgtgac gtggcgcggg gcgtgggaac ggggcgggtg acgtagtagt gtggcggaag     120
   tgtgatgttg caagtgtggc ggaacacatg taagcgacgg atgtggcaaa agtgacgttt     180
   ttggtgtgcg ccggtgtaca caggaagtga caattttcgc gcggttttag gcggatgttg     240
   tagtaaattt gggcgtaacc gagtaagatt tggccatttt cgcgggaaaa ctgaataaga     300
   ggaagtgaaa tctgaataat tttgtgttac tcatagcgcg taatatttgt ctagggccgc     360
   ggggactttg accgtttacg tggagactcg cccaggtgtt tttctcaggt gttttccgcg     420
   ttccgggtca aagttggcgt tttattatta tagtcagctg acgtgtagtg tatttatacc     480
   cggtgagttc ctcaagaggc cactcttgag tgccagcgag tagagttttc tcctccgagc     540
   cgctccgaca ccgggactga aaatgagaca tattatctgc cacggaggtg ttattaccga 600
   agaaatggcc gccagtcttt tggaccagct gatcgaagag gtactggctg ataatcttcc 660
   acctcctagc cattttgaac cacctaccct tcacgaactg tatgatttag acgtgacggc 720
   ccccgaagat cccaacgagg aggcggtttc gcagattttt cccgactctg taatgttggc 780
   ggtgcaggaa gggattgact tactcacttt tccgccggcg cccggttctc cggagccgcc 840
   tcacctttcc cggcagcccg agcagccgga gcagagagcc ttgggtccgg tttctatgcc 900
   aaaccttgta ccggaggtga tcgatccacc cagtgacgac gaggatgaag agggtgagga 960
   gtttgtgtta gattatgtgg agcaccccgg gcacggttgc aggtcttgtc attatcaccg 1020
   gaggaatacg ggggacccag atattatgtg ttcgctttgc tatatgagga cctgtggcat 1080
   gtttgtctac agtaagtgaa aattatgggc agtgggtgat agagtggtgg gtttggtgtg 1140
   gtaatttttt ttttaatttt tacagttttg tggtttaaag aattttgtat tgtgattttt 1200
   ttaaaaggtc ctgtgtctga acctgagcct gagcccgagc cagaaccgga gcctgcaaga 1260
   cctacccgcc gtcctaaaat ggcgcctgct atcctgagac gcccgacatc acctgtgtct 1320
   agagaatgca atagtagtac ggatagctgt gactccggtc cttctaacac acctcctgag 1380
   atacacccgg tggtcccgct gtgccccatt aaaccagttg ccgtgagagt tggtgggcgt 1440
   cgccaggctg tggaatgtat cgaggacttg cttaacgagc ctgggcaacc tttggacttg 1500
   agctgtaaac gccccaggcc ataaggtgta aacctgtgat tgcgtgtgtg gttaacgcct 1560
   ttgtttgctg aatgagttga tgtaagttta ataaagggtg agataatgtt taacttgcat 1620
   ggcgtgttaa atggggcggg gcttaaaggg tatataatgc gccgtgggct aatcttggtt 1680
   acatctgacc tcatggaggc ttgggagtgt ttggaagatt tttctgctgt gcgtaacttg 1740
   ctggaacaga gctctaacag tacctcttgg ttttggaggt ttctgtgggg ctcatcccag 1800
   gcaaagttag tctgcagaat taaggaggat tacaagtggg aatttgaaga gcttttgaaa 1860
   tcctgtggtg agctgtttga ttctttgaat ctgggtcacc aggcgctttt ccaagagaag 1920
   gtcatcaaga ctttggattt ttccacaccg gggcgcgctg cggctgctgt tgcttttttg 1980
   agttttataa aggataaatg gagcgaagaa acccatctga gcggggggta cctgctggat 2040
   tttctggcca tgcatctgtg gagagcggtt gtgagacaca agaatcgcct gctactgttg 2100
   tcttccgtcc gcccggcgat aataccgacg gaggagcagc agcagcagca ggaggaagcc 2160
   aggcggcggc ggcaggagca gagcccatgg aacccgagag ccggcctgga ccctcgggaa 2220
   tgaatgttgt acaggtggct gaactgtatc cagaactgag acgcattttg acaattacag 2280
   aggatgggca ggggctaaag ggggtaaaga gggagcgggg ggcttgtgag gctacagagg 2340
   aggctaggaa tctagctttt agcttaatga ccagacaccg tcctgagtgt attacttttc 2400
   aacagatcaa ggataattgc gctaatgagc ttgatctgct ggcgcagaag tattccatag 2460
   agcagctgac cacttactgg ctgcagccag gggatgattt tgaggaggct attagggtat 2520
   atgcaaaggt ggcacttagg ccagattgca agtacaagat cagcaaactt gtaaatatca 2580
   ggaattgttg ctacatttct gggaacgggg ccgaggtgga gatagatacg gaggataggg 2640
   tggcctttag atgtagcatg ataaatatgt ggccgggggt gcttggcatg gacggggtgg 2700
   ttattatgaa tgtaaggttt actggcccca attttagcgg tacggttttc ctggccaata 2760
   ccaaccttat cctacacggt gtaagcttct atgggtttaa caatacctgt gtggaagcct 2820
   ggaccgatgt aagggttcgg ggctgtgcct tttactgctg ctggaagggg gtggtgtgtc 2880
   gccccaaaag cagggcttca attaagaaat gcctctttga aaggtgtacc ttgggtatcc 2940
   tgtctgaggg taactccagg gtgcgccaca atgtggcctc cgactgtggt tgcttcatgc 3000
   tagtgaaaag cgtggctgtg attaagcata acatggtatg tggcaactgc gaggacaggg 3060
   cctctcagat gctgacctgc tcggacggca actgtcacct gctgaagacc attcacgtag 3120
   ccagccactc tcgcaaggcc tggccagtgt ttgagcataa catactgacc cgctgttcct 3180
   tgcatttggg taacaggagg ggggtgttcc taccttacca atgcaatttg agtcacacta 3240
   agatattgct tgagcccgag agcatgtcca aggtgaacct gaacggggtg tttgacatga 3300
   ccatgaagat ctggaaggtg ctgaggtacg atgagacccg caccaggtgc agaccctgcg 3360
   agtgtggcgg taaacatatt aggaaccagc ctgtgatgct ggatgtgacc gaggagctga 3420
   ggcccgatca cttggtgctg gcctgcaccc gcgctgagtt tggctctagc gatgaagata 3480
   cagattgagg tactgaaatg tgtgggcgtg gcttaagggt gggaaagaat atataaggtg 3540
   ggggtcttat gtagttttgt atctgttttg cagcagccgc cgccgccatg agcaccaact 3600
   cgtttgatgg aagcattgtg agctcatatt tgacaacgcg catgccccca tgggccgggg 3660
   tgcgtcagaa tgtgatgggc tccagcattg atggtcgccc cgtcctgccc gcaaactcta 3720
   ctaccttgac ctacgagacc gtgtctggaa cgccgttgga gactgcagcc tccgccgccg 3780
   cttcagccgc tgcagccacc gcccgcggga ttgtgactga ctttgctttc ctgagcccgc 3840
   ttgcaagcag tgcagcttcc cgttcatccg cccgcgatga caagttgacg gctcttttgg 3900
   cacaattgga ttctttgacc cgggaactta atgtcgtttc tcagcagctg ttggatctgc 3960
   gccagcaggt ttctgccctg aaggcttcct cccctcccaa tgcggtttaa aacataaata 4020
   aaaaaccaga ctctgtttgg atttggatca agcaagtgtc ttgctgtctt tatttagggg 4080
   ttttgcgcgc gcggtaggcc cgggaccagc ggtctcggtc gttgagggtc ctgtgtattt 4140
   tttccaggac gtggtaaagg tgactctgga tgttcagata catgggcata agcccgtctc 4200
   tggggtggag gtagcaccac tgcagagctt catgctgcgg ggtggtgttg tagatgatcc 4260
   agtcgtagca ggagcgctgg gcgtggtgcc taaaaatgtc tttcagtagc aagctgattg 4320
   ccaggggcag gcccttggtg taagtgttta caaagcggtt aagctgggat gggtgcatac 4380
   gtggggatat gagatgcatc ttggactgta tttttaggtt ggctatgttc ccagccatat 4440
   ccctccgggg attcatgttg tgcagaacca ccagcacagt gtatccggtg cacttgggaa 4500
   atttgtcatg tagcttagaa ggaaatgcgt ggaagaactt ggagacgccc ttgtgacctc 4560
   caagattttc catgcattcg tccataatga tggcaatggg cccacgggcg gcggcctggg 4620
   cgaagatatt tctgggatca ctaacgtcat agttgtgttc caggatgaga tcgtcatagg 4680
   ccatttttac aaagcgcggg cggagggtgc cagactgcgg tataatggtt ccatccggcc 4740
   caggggcgta gttaccctca cagatttgca tttcccacgc tttgagttca gatgggggga 4800
   tcatgtctac ctgcggggcg atgaagaaaa cggtttccgg ggtaggggag atcagctggg 4860
   aagaaagcag gttcctgagc agctgcgact taccgcagcc ggtgggcccg taaatcacac 4920
   ctattaccgg gtgcaactgg tagttaagag agctgcagct gccgtcatcc ctgagcaggg 4980
   gggccacttc gttaagcatg tccctgactc gcatgttttc cctgaccaaa tccgccagaa 5040
   ggcgctcgcc gcccagcgat agcagttctt gcaaggaagc aaagtttttc aacggtttga 5100
   gaccgtccgc cgtaggcatg cttttgagcg tttgaccaag cagttccagg cggtcccaca 5160
   gctcggtcac ctgctctacg gcatctcgat ccagcatatc tcctcgtttc gcgggttggg 5220
   gcggctttcg ctgtacggca gtagtcggtg ctcgtccaga cgggccaggg tcatgtcttt 5280
   ccacgggcgc agggtcctcg tcagcgtagt ctgggtcacg gtgaaggggt gcgctccggg 5340
   ctgcgcgctg gccagggtgc gcttgaggct ggtcctgctg gtgctgaagc gctgccggtc 5400
   ttcgccctgc gcgtcggcca ggtagcattt gaccatggtg tcatagtcca gcccctccgc 5460
   ggcgtggccc ttggcgcgca gcttgccctt ggaggaggcg ccgcacgagg ggcagtgcag 5520
   acttttgagg gcgtagagct tgggcgcgag aaataccgat tccggggagt aggcatccgc 5580
   gccgcaggcc ccgcagacgg tctcgcattc cacgagccag gtgagctctg gccgttcggg 5640
   gtcaaaaacc aggtttcccc catgcttttt gatgcgtttc ttacctctgg tttccatgag 5700
   ccggtgtcca cgctcggtga cgaaaaggct gtccgtgtcc ccgtatacag actnnngttt 5760
   tgagaggcct gtcctcgagc ggtgttccgc ggtcctcctc gtatagaaac tcggaccact 5820
   ctgagacaaa ggctcgcgtc caggccagca cgaaggaggc taagtgggag gggtagcggt 5880
   cgttgtccac tagggggtcc actcgctcca gggtgtgaag acacatgtcg ccctcttcgg 5940
   catcaaggaa ggtgattggt ttgtaggtgt aggccacgtg accgggtgtt cctgaagggg 6000
   ggctataaaa gggggtgggg gcgcgttcgt cctcactctc ttccgcatcg ctgtctgcga 6060
   gggccagctg ttggggtgag tactccctct gaaaagcggg catgacttct gcgctaagat 6120
   tgtcagtttc caaaaacgag gaggatttga tattcacctg gcccgcggtg atgcctttga 6180
   gggtggccgc atccatctgg tcagaaaaga caatcttttt gttgtcaagc ttggtggcaa 6240
   acgacccgta gagggcgttg gacagcaact tggcgatgga gcgcagggtt tggtttttgt 6300
   cgcgatcggc gcgctccttg gccgcgatgt ttagctgcac gtattcgcgc gcaacgcacc 6360
   gccattcggg aaagacggtg gtgcgctcgt cgggcaccag gtgcacgcgc caaccgcggt 6420
   tgtgcagggt gacaaggtca acgctggtgg ctacctctcc gcgtaggcgc tcgttggtcc 6480
   agcagaggcg gccgcccttg cgcgagcaga atggcggtag ggggtctagc tgcgtctcgt 6540
   ccggggggtc tgcgtccacg gtaaagaccc cgggcagcag gcgcgcgtcg aagtagtcta 6600
   tcttgcatcc ttgcaagtct agcgcctgct gccatgcgcg ggcggcaagc gcgcgctcgt 6660
   atgggttgag tgggggaccc catggcatgg ggtgggtgag cgcggaggcg tacatgccgc 6720
   aaatgtcgta aacgtagagg ggctctctga gtattccaag atatgtaggg tagcatcttc 6780
   caccgcggat gctggcgcgc acgtaatcgt atagttcgtg cgagggagcg aggaggtcgg 6840
   gaccgaggtt gctacgggcg ggctgctctg ctcggaagac tatctgcctg aagatggcat 6900
   gtgagttgga tgatatggtt ggacgctgga agacgttgaa gctggcgtct gtgagaccta 6960
   ccgcgtcacg cacgaaggag gcgtaggagt cgcgcagctt gttgaccagc tcggcggtga 7020
   cctgcacgtc tagggcgcag tagtccaggg tttccttgat gatgtcatac ttatcctgtc 7080
   cctttttttt ccacagctcg cggttgagga caaactcttc gcggtctttc cagtactctt 7140
   ggatcggaaa cccgtcggcc tccgaacggt aagagcctag catgtagaac tggttgacgg 7200
   cctggtaggc gcagcatccc ttttctacgg gtagcgcgta tgcctgcgcg gccttccgga 7260
   gcgaggtgtg ggtgagcgca aaggtgtccc tgaccatgac tttgaggtac tggtatttga 7320
   agtcagtgtc gtcgcatccg ccctgctccc agagcaaaaa gtccgtgcgc tttttggaac 7380
   gcggatttgg cagggcgaag gtgacatcgt tgaagagtat ctttcccgcg cgaggcataa 7440
   agttgcgtgt gatgcggaag ggtcccggca cctcggaacg gttgttaatt acctgggcgg 7500
   cgagcacgat ctcgtcaaag ccgttgatgt tgtggcccac aatgtaaagt tccaagaagc 7560
   gcgggatgcc cttgatggaa ggcaattttt taagttcctc gtaggtgagc tcttcagggg 7620
   agctgagccc gtgctctgaa agggcccagt ctgcaagatg agggttggaa gcgacgaatg 7680
   agctccacag gtcacgggcc attagcattt gcaggtggtc gcgaaaggtc ctaaactggc 7740
   gacctatggc cattttttct ggggtgatgc agtagaaggt aagcgggtct tgttcccagc 7800
   ggtcccatcc aaggttcgcg gctaggtctc gcgcggcagt cactagaggc tcatctccgc 7860
   cgaacttcat gaccagcatg aagggcacga gctgcttccc aaaggccccc atccaagtat 7920
   aggtctctac atcgtaggtg acaaagagac gctcggtgcg aggatgcgag ccgatcggga 7980
   agaactggat ctcccgccac caattggagg agtggctatt gatgtggtga aagtagaagt 8040
   ccctgcgacg ggccgaacac tcgtgctggc ttttgtaaaa acgtgcgcag tactggcagc 8100
   ggtgcacggg ctgtacatcc tgcacgaggt tgacctgacg accgcgcaca aggaagcaga 8160
   gtgggaattt gagcccctcg cctggcgggt ttggctggtg gtcttctact tcggctgctt 8220
   gtccttgacc gtctggctgc tcgaggggag ttacggtgga tcggaccacc acgccgcgcg 8280
   agcccaaagt ccagatgtcc gcgcgcggcg gtcggagctt gatgacaaca tcgcgcagat 8340
   gggagctgtc catggtctgg agctcccgcg gcgtcaggtc aggcgggagc tcctgcaggt 8400
   ttacctcgca tagacgggtc agggcgcggg ctagatccag gtgataccta atttccaggg 8460
   gctggttggt ggcggcgtcg atggcttgca agaggccgca tccccgcggc gcgactacgg 8520
   taccgcgcgg cgggcggtgg gccgcggggg tgtccttgga tgatgcatct aaaagcggtg 8580
   acgcgggcga gcccccggag gtaggggggg ctccggaccc gccgggagag ggggcagggg 8640
   cacgtcggcg ccgcgcgcgg gcaggagctg gtgctgcgcg cgtaggttgc tggcgaacgc 8700
   gacgacgcgg cggttgatct cctgaatctg gcgcctctgc gtgaagacga cgggcccggt 8760
   gagcttgagc ctgaaagaga gttcgacaga atcaatttcg gtgtcgttga cggcggcctg 8820
   gcgcaaaatc tcctgcacgt ctcctgagtt gtcttgatag gcgatctcgg ccatgaactg 8880
   ctcgatctct tcctcctgga gatctccgcg tccggctcgc tccacggtgg cggcgaggtc 8940
   gttggaaatg cgggccatga gctgcgagaa ggcgttgagg cctccctcgt tccagacgcg 9000
   gctgtagacc acgccccctt cggcatcgcg ggcgcgcatg accacctgcg cgagattgag 9060
   ctccacgtgc cgggcgaaga cggcgtagtt tcgcaggcgc tgaaagaggt agttgagggt 9120
   ggtggcggtg tgttctgcca cgaagaagta cataacccag cgtcgcaacg tggattcgtt 9180
   gatatccccc aaggcctcaa ggcgctccat ggcctcgtag aagtccacgg cgaagttgaa 9240
   aaactgggag ttgcgcgccg acacggttaa ctcctcctcc agaagacgga tgagctcggc 9300
   gacagtgtcg cgcacctcgc gctcaaaggc tacaggggcc tcttcttctt cttcaatctc 9360
   ctcttccata agggcctccc cttcttcttc ttctggcggc ggtgggggag gggggacacg 9420
   gcggcgacga cggcgcaccg ggaggcggtc gacaaagcgc tcgatcatct ccccgcggcg 9480
   acggcgcatg gtctcggtga cggcgcggcc gttctcgcgg gggcgcagtt ggaagacgcc 9540
   gcccgtcatg tcccggttat gggttggcgg ggggctgcca tgcggcaggg atacggcgct 9600
   aacgatgcat ctcaacaatt gttgtgtagg tactccgccg ccgagggacc tgagcgagtc 9660
   cgcatcgacc ggatcggaaa acctctcgag aaaggcgtct aaccagtcac agtcgcaagg 9720
   taggctgagc accgtggcgg gcggcagcgg gcggcggtcg gggttgtttc tggcggaggt 9780
   gctgctgatg atgtaattaa agtaggcggt cttgagacgg cggatggtcg acagaagcac 9840
   catgtccttg ggtccggcct gctgaatgcg caggcggtcg gccatgcccc aggcttcgtt 9900
   ttgacatcgg cgcaggtctt tgtagtagtc ttgcatgagc ctttctaccg gcacttcttc 9960
   ttctccttcc tcttgtcctg catctcttgc atctatcgct gcggcggcgg cggagtttgg 10020
   ccgtaggtgg cgccctcttc ctcccatgcg tgtgaccccg aagcccctca tcggctgaag 10080
   cagggctagg tcggcgacaa cgcgctcggc taatatggcc tgctgcacct gcgtgagggt 10140
   agactggaag tcatccatgt ccacaaagcg gtggtatgcg cccgtgttga tggtgtaagt 10200
   gcagttggcc ataacggacc agttaacggt ctggtgaccc ggctgcgaga gctcggtgta 10260
   cctgagacgc gagtaagccc tcgagtcaaa tacgtagtcg ttgcaagtcc gcaccaggta 10320
   ctggtatccc accaaaaagt gcggcggcgg ctggcggtag aggggccagc gtagggtggc 10380
   cggggctccg ggggcgagat cttccaacat aaggcgatga tatccgtaga tgtacctgga 10440
   catccaggtg atgccggcgg cggtggtgga ggcgcgcgga aagtcgcgga cgcggttcca 10500
   gatgttgcgc agcggcaaaa agtgctccat ggtcgggacg ctctggccgg tcaggcgcgc 10560
   gcaatcgttg acgctctacc gtgcaaaagg agagcctgta agcgggcact cttccgtggt 10620
   ctggtggata aattcgcaag ggtatcatgg cggacgaccg gggttcgagc cccgtatccg 10680
   gccgtccgcc gtgatccatg cggttaccgc ccgcgtgtcg aacccaggtg tgcgacgtca 10740
   gacaacgggg gagtgctcct tttggcttcc ttccaggcgc ggcggctgct gcgctagctt 10800
   ttttggccac tggccgcgcg cagcgtaagc ggttaggctg gaaagcgaaa gcattaagtg 10860
   gctcgctccc tgtagccgga gggttatttt ccaagggttg agtcgcggga cccccggttc 10920
   gagtctcgga ccggccggac tgcggcgaac gggggtttgc ctccccgtca tgcaagaccc 10980
   cgcttgcaaa ttcctccgga aacagggacg agcccctttt ttgcttttcc cagatgcatc 11040
   cggtgctgcg gcagatgcgc ccccctcctc agcagcggca agagcaagag cagcggcaga 11100
   catgcagggc accctcccct cctcctaccg cgtcaggagg ggcgacatcc gcggttgacg 11160
   cggcagcaga tggtgattac gaacccccgc ggcgccgggc ccggcactac ctggacttgg 11220
   aggagggcga gggcctggcg cggctaggag cgccctctcc tgagcggtac ccaagggtgc 11280
   agctgaagcg tgatacgcgt gaggcgtacg tgccgcggca gaacctgttt cgcgaccgcg 11340
   agggagagga gcccgaggag atgcgggatc gaaagttcca cgcagggcgc gagctgcggc 11400
   atggcctgaa tcgcgagcgg ttgctgcgcg aggaggactt tgagcccgac gcgcgaaccg 11460
   ggattagtcc cgcgcgcgca cacgtggcgg ccgccgacct ggtaaccgca tacgagcaga 11520
   cggtgaacca ggagattaac tttcaaaaaa gctttaacaa ccacgtgcgt acgcttgtgg 11580
   cgcgcgagga ggtggctata ggactgatgc atctgtggga ctttgtaagc gcgctggagc 11640
   aaaacccaaa tagcaagccg ctcatggcgc agctgttcct tatagtgcag cacagcaggg 11700
   acaacgaggc attcagggat gcgctgctaa acatagtaga gcccgagggc cgctggctgc 11760
   tcgatttgat aaacatcctg cagagcatag tggtgcagga gcgcagcttg agcctggctg 11820
   acaaggtggc cgccatcaac tattccatgc ttagcctggg caagttttac gcccgcaaga 11880
   tataccatac cccttacgtt cccatagaca aggaggtaaa gatcgagggg ttctacatgc 11940
   gcatggcgct gaaggtgctt accttgagcg acgacctggg cgtttatcgc aacgagcgca 12000
   tccacaaggc cgtgagcgtg agccggcggc gcgagctcag cgaccgcgag ctgatgcaca 12060
   gcctgcaaag ggccctggct ggcacgggca gcggcgatag agaggccgag tcctactttg 12120
   acgcgggcgc tgacctgcgc tgggccccaa gccgacgcgc cctggaggca gctggggccg 12180
   gacctgggct ggcggtggca cccgcgcgcg ctggcaacgt cggcggcgtg gaggaatatg 12240
   acgaggacga tgagtacgag ccagaggacg gcgagtacta agcggtgatg tttctgatca 12300
   gatgatgcaa gacgcaacgg acccggcggt gcgggcggcg ctgcagagcc agccgtccgg 12360
   ccttaactcc acggacgact ggcgccaggt catggaccgc atcatgtcgc tgactgcgcg 12420
   caatcctgac gcgttccggc agcagccgca ggccaaccgg ctctccgcaa ttctggaagc 12480
   ggtggtcccg gcgcgcgcaa accccacgca cgagaaggtg ctggcgatcg taaacgcgct 12540
   ggccgaaaac agggccatcc ggcccgacga ggccggcctg gtctacgacg cgctgcttca 12600
   gcgcgtggct cgttacaaca gcggcaacgt gcagaccaac ctggaccggc tggtggggga 12660
   tgtgcgcgag gccgtggcgc agcgtgagcg cgcgcagcag cagggcaacc tgggctccat 12720
   ggttgcacta aacgccttcc tgagtacaca gcccgccaac gtgccgcggg gacaggagga 12780
   ctacaccaac tttgtgagcg cactgcggct aatggtgact gagacaccgc aaagtgaggt 12840
   gtaccagtct gggccagact attttttcca gaccagtaga caaggcctgc agaccgtaaa 12900
   cctgagccag gctttcaaaa acttgcaggg gctgtggggg gtgcgggctc ccacaggcga 12960
   ccgcgcgacc gtgtctagct tgctgacgcc caactcgcgc ctgttgctgc tgctaatagc 13020
   gcccttcacg gacagtggca gcgtgtcccg ggacacatac ctaggtcact tgctgacact 13080
   gtaccgcgag gccataggtc aggcgcatgt ggacgagcat actttccagg agattacaag 13140
   tgtcagccgc gcgctggggc aggaggacac gggcagcctg gaggcaaccc taaactacct 13200
   gctgaccaac cggcggcaga agatcccctc gttgcacagt ttaaacagcg aggaggagcg 13260
   cattttgcgc tacgtgcagc agagcgtgag ccttaacctg atgcgcgacg gggtaacgcc 13320
   cagcgtggcg ctggacatga ccgcgcgcaa catggaaccg ggcatgtatg cctcaaaccg 13380
   gccgtttatc aaccgcctaa tggactactt gcatcgcgcg gccgccgtga accccgagta 13440
   tttcaccaat gccatcttga acccgcactg gctaccgccc cctggtttct acaccggggg 13500
   attcgaggtg cccgagggta acgatggatt cctctgggac gacatagacg acagcgtgtt 13560
   ttccccgcaa ccgcagaccc tgctagagtt gcaacagcgc gagcaggcag aggcggcgct 13620
   gcgaaaggaa agcttccgca ggccaagcag cttgtccgat ctaggcgctg cggccccgcg 13680
   gtcagatgct agtagcccat ttccaagctt gatagggtct cttaccagca ctcgcaccac 13740
   ccgcccgcgc ctgctgggcg aggaggagta cctaaacaac tcgctgctgc agccgcagcg 13800
   cgaaaaaaac ctgcctccgg catttcccaa caacgggata gagagcctag tggacaagat 13860
   gagtagatgg aagacgtacg cgcaggagca cagggacgtg ccaggcccgc gcccgcccac 13920
   ccgtcgtcaa aggcacgacc gtcagcgggg tctggtgtgg gaggacgatg actcggcaga 13980
   cgacagcagc gtcctggatt tgggagggag tggcaacccg tttgcgcacc ttcgccccag 14040
   gctggggaga atgttttaaa aaaaaaaaag catgatgcaa aataaaaaac tcaccaaggc 14100
   catggcaccg agcgttggtt ttcttgtatt ccccttagta tgcggcgcgc ggcgatgtat 14160
   gaggaaggtc ctcctccctc ctacgagagt gtggtgagcg cggcgccagt ggcggcggcg 14220
   ctgggttctc ccttcgatgc tcccctggac ccgccgtttg tgcctccgcg gtacctgcgg 14280
   cctaccgggg ggagaaacag catccgttac tctgagttgg cacccctatt cgacaccacc 14340
   cgtgtgtacc tggtggacaa caagtcaacg gatgtggcat ccctgaacta ccagaacgac 14400
   cacagcaact ttctgaccac ggtcattcaa aacaatgact acagcccggg ggaggcaagc 14460
   acacagacca tcaatcttga cgaccggtcg cactggggcg gcgacctgaa aaccatcctg 14520
   cataccaaca tgccaaatgt gaacgagttc atgtttacca ataagtttaa ggcgcgggtg 14580
   atggtgtcgc gcttgcctac taaggacaat caggtggagc tgaaatacga gtgggtggag 14640
   ttcacgctgc ccgagggcaa ctactccgag accatgacca tagaccttat gaacaacgcg 14700
   atcgtggagc actacttgaa agtgggcaga cagaacgggg ttctggaaag cgacatcggg 14760
   gtaaagtttg acacccgcaa cttcagactg gggtttgacc ccgtcactgg tcttgtcatg 14820
   cctggggtat atacaaacga agccttccat ccagacatca ttttgctgcc aggatgcggg 14880
   gtggacttca cccacagccg cctgagcaac ttgttgggca tccgcaagcg gcaacccttc 14940
   caggagggct ttaggatcac ctacgatgat ctggagggtg gtaacattcc cgcactgttg 15000
   gatgtggacg cctaccaggc gagcttgaaa gatgacaccg aacagggcgg gggtggcgca 15060
   ggcggcagca acagcagtgg cagcggcgcg gaagagaact ccaacgcggc agccgcggca 15120
   atgcagccgg tggaggacat gaacgatcat gccattcgcg gcgacacctt tgccacacgg 15180
   gctgaggaga agcgcgctga ggccgaagca gcggccgaag ctgccgcccc cgctgcgcaa 15240
   cccgaggtcg agaagcctca gaagaaaccg gtgatcaaac ccctgacaga ggacagcaag 15300
   aaacgcagtt acaacctaat aagcaatgac agcaccttca cccagtaccg cagctggtac 15360
   cttgcataca actacggcga ccctcagacc ggaatccgct catggaccct gctttgcact 15420
   cctgacgtaa cctgcggctc ggagcaggtc tactggtcgt tgccagacat gatgcaagac 15480
   cccgtgacct tccgctccac gcgccagatc agcaactttc cggtggtggg cgccgagctg 15540
   ttgcccgtgc actccaagag cttctacaac gaccaggccg tctactccca actcatccgc 15600
   cagtttacct ctctgaccca cgtgttcaat cgctttcccg agaaccagat tttggcgcgc 15660
   ccgccagccc ccaccatcac caccgtcagt gaaaacgttc ctgctctcac agatcacggg 15720
   acgctaccgc tgcgcaacag catcggagga gtccagcgag tgaccattac tgacgccaga 15780
   cgccgcacct gcccctacgt ttacaaggcc ctgggcatag tctcgccgcg cgtcctatcg 15840
   agccgcactt tttgagcaag catgtccatc cttatatcgc ccagcaataa cacaggctgg 15900
   ggcctgcgct tcccaagcaa gatgtttggc ggggccaaga agcgctccga ccaacaccca 15960
   gtgcgcgtgc gcgggcacta ccgcgcgccc tggggcgcgc acaaacgcgg ccgcactggg 16020
   cgcaccaccg tcgatgacgc catcgacgcg gtggtggagg aggcgcgcaa ctacacgccc 16080
   acgccgccac cagtgtccac agtggacgcg gccattcaga ccgtggtgcg cggagcccgg 16140
   cgctatgcta aaatgaagag acggcggagg cgcgtagcac gtcgccaccg ccgccgaccc 16200
   ggcactgccg cccaacgcgc ggcggcggcc ctgcttaacc gcgcacgtcg caccggccga 16260
   cgggcggcca tgcgggccgc tcgaaggctg gccgcgggta ttgtcactgt gccccccagg 16320
   tccaggcgac gagcggccgc cgcagcagcc gcggccatta gtgctatgac tcagggtcgc 16380
   aggggcaacg tgtattgggt gcgcgactcg gttagcggcc tgcgcgtgcc cgtgcgcacc 16440
   cgccccccgc gcaactagat tgcaagaaaa aactacttag actcgtactg ttgtatgtat 16500
   ccagcggcgg cggcgcgcaa cgaagctatg tccaagcgca aaatcaaaga agagatgctc 16560
   caggtcatcg cgccggagat ctatggcccc ccgaagaagg aagagcagga ttacaagccc 16620
   cgaaagctaa agcgggtcaa aaagaaaaag aaagatgatg atgatgaact tgacgacgag 16680
   gtggaactgc tgcacgctac cgcgcccagg cgacgggtac agtggaaagg tcgacgcgta 16740
   aaacgtgttt tgcgacccgg caccaccgta gtctttacgc ccggtgagcg ctccacccgc 16800
   acctacaagc gcgtgtatga tgaggtgtac ggcgacgagg acctgcttga gcaggccaac 16860
   gagcgcctcg gggagtttgc ctacggaaag cggcataagg acatgctggc gttgccgctg 16920
   gacgagggca acccaacacc tagcctaaag cccgtaacac tgcagcaggt gctgcccgcg 16980
   cttgcaccgt ccgaagaaaa gcgcggccta aagcgcgagt ctggtgactt ggcacccacc 17040
   gtgcagctga tggtacccaa gcgccagcga ctggaagatg tcttggaaaa aatgaccgtg 17100
   gaacctgggc tggagcccga ggtccgcgtg cggccaatca agcaggtggc gccgggactg 17160
   ggcgtgcaga ccgtggacgt tcagataccc actaccagta gcaccagtat tgccaccgcc 17220
   acagagggca tggagacaca aacgtccccg gttgcctcag cggtggcgga tgccgcggtg 17280
   caggcggtcg ctgcggccgc gtccaagacc tctacggagg tgcaaacgga cccgtggatg 17340
   tttcgcgttt cagccccccg gcgcccgcgc ggttcgagga agtacggcgc cgccagcgcg 17400
   ctactgcccg aatatgccct acatccttcc attgcgccta cccccggcta tcgtggctac 17460
   acctaccgcc ccagaagacg agcaactacc cgacgccgaa ccaccactgg aacccgccgc 17520
   cgccgtcgcc gtcgccagcc cgtgctggcc ccgatttccg tgcgcagggt ggctcgcgaa 17580
   ggaggcagga ccctggtgct gccaacagcg cgctaccacc ccagcatcgt ttaaaagccg 17640
   gtctttgtgg ttcttgcaga tatggccctc acctgccgcc tccgtttccc ggtgccggga 17700
   ttccgaggaa gaatgcaccg taggaggggc atggccggcc acggcctgac gggcggcatg 17760
   cgtcgtgcgc accaccggcg gcggcgcgcg tcgcaccgtc gcatgcgcgg cggtatcctg 17820
   cccctcctta ttccactgat cgccgcggcg attggcgccg tgcccggaat tgcatccgtg 17880
   gccttgcagg cgcagagaca ctgattaaaa acaagttgca tgtggaaaaa tcaaaataaa 17940
   aagtctggac tctcacgctc gcttggtcct gtaactattt tgtagaatgg aagacatcaa 18000
   ctttgcgtct ctggccccgc gacacggctc gcgcccgttc atgggaaact ggcaagatat 18060
   cggcaccagc aatatgagcg gtggcgcctt cagctggggc tcgctgtgga gcggcattaa 18120
   aaatttcggt tccaccgtta agaactatgg cagcaaggcc tggaacagca gcacaggcca 18180
   gatgctgagg gataagttga aagagcaaaa tttccaacaa aaggtggtag atggcctggc 18240
   ctctggcatt agcggggtgg tggacctggc caaccaggca gtgcaaaata agattaacag 18300
   taagcttgat ccccgccctc ccgtagagga gcctccaccg gccgtggaga cagtgtctcc 18360
   agaggggcgt ggcgaaaagc gtccgcgccc cgacagggaa gaaactctgg tgacgcaaat 18420
   agacgagcct ccctcgtacg aggaggcact aaagcaaggc ctgcccacca cccgtcccat 18480
   cgcgcccatg gctaccggag tgctgggcca gcacacaccc gtaacgctgg acctgcctcc 18540
   ccccgccgac acccagcaga aacctgtgct gccaggcccg accgccgttg ttgtaacccg 18600
   tcctagccgc gcgtccctgc gccgcgccgc cagcggtccg cgatcgttgc ggcccgtagc 18660
   cagtggcaac tggcaaagca cactgaacag catcgtgggt ctgggggtgc aatccctgaa 18720
   gcgccgacga tgcttctgaa tagctaacgt gtcgtatgtg tgtcatgtat gcgtccatgt 18780
   cgccgccaga ggagctgctg agccgccgcg cgcccgcttt ccaagatggc taccccttcg 18840
   atgatgccgc agtggtctta catgcacatc tcgggccagg acgcctcgga gtacctgagc 18900
   cccgggctgg tgcagtttgc ccgcgccacc gagacgtact tcagcctgaa taacaagttt 18960
   agaaacccca cggtggcgcc tacgcacgac gtgaccacag accggtccca gcgtttgacg 19020
   ctgcggttca tccctgtgga ccgtgaggat actgcgtact cgtacaaggc gcggttcacc 19080
   ctagctgtgg gtgataaccg tgtgctggac atggcttcca cgtactttga catccgcggc 19140
   gtgctggaca ggggccctac ttttaagccc tactctggca ctgcctacaa cgccctggct 19200
   cccaagggtg ccccaaatcc ttgcgaatgg gatgaagctg ctactgctct tgaaataaac 19260
   ctagaagaag aggacgatga caacgaagac gaagtagacg agcaagctga gcagcaaaaa 19320
   actcacgtat ttgggcaggc gccttattct ggtataaata ttacaaagga gggtattcaa 19380
   ataggtgtcg aaggtcaaac acctaaatat gccgataaaa catttcaacc tgaacctcaa 19440
   ataggagaat ctcagtggta cgaaactgaa attaatcatg cagctgggag agtccttaaa 19500
   aagactaccc caatgaaacc atgttacggt tcatatgcaa aacccacaaa tgaaaatgga 19560
   gggcaaggca ttcttgtaaa gcaacaaaat ggaaagctag aaagtcaagt ggaaatgcaa 19620
   tttttctcaa ctactgaggc gaccgcaggc aatggtgata acttgactcc taaagtggta 19680
   ttgtacagtg aagatgtaga tatagaaacc ccagacactc atatttctta catgcccact 19740
   attaaggaag gtaactcacg agaactaatg ggccaacaat ctatgcccaa caggcctaat 19800
   tacattgctt ttagggacaa ttttattggt ctaatgtatt acaacagcac gggtaatatg 19860
   ggtgttctgg cgggccaagc atcgcagttg aatgctgttg tagatttgca agacagaaac 19920
   acagagcttt cataccagct tttgcttgat tccattggtg atagaaccag gtacttttct 19980
   atgtggaatc aggctgttga cagctatgat ccagatgtta gaattattga aaatcatgga 20040
   actgaagatg aacttccaaa ttactgcttt ccactgggag gtgtgattaa tacagagact 20100
   cttaccaagg taaaacctaa aacaggtcag gaaaatggat gggaaaaaga tgctacagaa 20160
   ttttcagata aaaatgaaat aagagttgga aataattttg ccatggaaat caatctaaat 20220
   gccaacctgt ggagaaattt cctgtactcc aacatagcgc tgtatttgcc cgacaagcta 20280
   aagtacagtc cttccaacgt aaaaatttct gataacccaa acacctacga ctacatgaac 20340
   aagcgagtgg tggctcccgg gttagtggac tgctacatta accttggagc acgctggtcc 20400
   cttgactata tggacaacgt caacccattt aaccaccacc gcaatgctgg cctgcgctac 20460
   cgctcaatgt tgctgggcaa tggtcgctat gtgcccttcc acatccaggt gcctcagaag 20520
   ttctttgcca ttaaaaacct ccttctcctg ccgggctcat acacctacga gtggaacttc 20580
   aggaaggatg ttaacatggt tctgcagagc tccctaggaa atgacctaag ggttgacgga 20640
   gccagcatta agtttgatag catttgcctt tacgccacct tcttccccat ggcccacaac 20700
   accgcctcca cgcttgaggc catgcttaga aacgacacca acgaccagtc ctttaacgac 20760
   tatctctccg ccgccaacat gctctaccct atacccgcca acgctaccaa cgtgcccata 20820
   tccatcccct cccgcaactg ggcggctttc cgcggctggg ccttcacgcg ccttaagact 20880
   aaggaaaccc catcactggg ctcgggctac gacccttatt acacctactc tggctctata 20940
   ccctacctag atggaacctt ttacctcaac cacaccttta agaaggtggc cattaccttt 21000
   gactcttctg tcagctggcc tggcaatgac cgcctgctta cccccaacga gtttgaaatt 21060
   aagcgctcag ttgacgggga gggttacaac gttgcccagt gtaacatgac caaagactgg 21120
   ttcctggtac aaatgctagc taactacaac attggctacc agggcttcta tatcccagag 21180
   agctacaagg accgcatgta ctccttcttt agaaacttcc agcccatgag ccgtcaggtg 21240
   gtggatgata ctaaatacaa ggactaccaa caggtgggca tcctacacca acacaacaac 21300
   tctggatttg ttggctacct tgcccccacc atgcgcgaag gacaggccta ccctgctaac 21360
   ttcccctatc cgcttatagg caagaccgca gttgacagca ttacccagaa aaagtttctt 21420
   tgcgatcgca ccctttggcg catcccattc tccagtaact ttatgtccat gggcgcactc 21480
   acagacctgg gccaaaacct tctctacgcc aactccgccc acgcgctaga catgactttt 21540
   gaggtggatc ccatggacga gcccaccctt ctttatgttt tgtttgaagt ctttgacgtg 21600
   gtccgtgtgc accggccgca ccgcggcgtc atcgaaaccg tgtacctgcg cacgcccttc 21660
   tcggccggca acgccacaac ataaagaagc aagcaacatc aacaacagct gccgccatgg 21720
   gctccagtga gcaggaactg aaagccattg tcaaagatct tggttgtggg ccatattttt 21780
   tgggcaccta tgacaagcgc tttccaggct ttgtttctcc acacaagctc gcctgcgcca 21840
   tagtcaatac ggccggtcgc gagactgggg gcgtacactg gatggccttt gcctggaacc 21900
   cgcactcaaa aacatgctac ctctttgagc cctttggctt ttctgaccag cgactcaagc 21960
   aggtttacca gtttgagtac gagtcactcc tgcgccgtag cgccattgct tcttcccccg 22020
   accgctgtat aacgctggaa aagtccaccc aaagcgtaca ggggcccaac tcggccgcct 22080
   gtggactatt ctgctgcatg tttctccacg cctttgccaa ctggccccaa actcccatgg 22140
   atcacaaccc caccatgaac cttattaccg gggtacccaa ctccatgctc aacagtcccc 22200
   aggtacagcc caccctgcgt cgcaaccagg aacagctcta cagcttcctg gagcgccact 22260
   cgccctactt ccgcagccac agtgcgcaga ttaggagcgc cacttctttt tgtcacttga 22320
   aaaacatgta aaaataatgt actagagaca ctttcaataa aggcaaatgc ttttatttgt 22380
   acactctcgg gtgattattt acccccaccc ttgccgtctg cgccgtttaa aaatcaaagg 22440
   ggttctgccg cgcatcgcta tgcgccactg gcagggacac gttgcgatac tggtgtttag 22500
   tgctccactt aaactcaggc acaaccatcc gcggcagctc ggtgaagttt tcactccaca 22560
   ggctgcgcac catcaccaac gcgtttagca ggtcgggcgc cgatatcttg aagtcgcagt 22620
   tggggcctcc gccctgcgcg cgcgagttgc gatacacagg gttgcagcac tggaacacta 22680
   tcagcgccgg gtggtgcacg ctggccagca cgctcttgtc ggagatcaga tccgcgtcca 22740
   ggtcctccgc gttgctcagg gcgaacggag tcaactttgg tagctgcctt cccaaaaagg 22800
   gcgcgtgccc aggctttgag ttgcactcgc accgtagtgg catcaaaagg tgaccgtgcc 22860
   cggtctgggc gttaggatac agcgcctgca taaaagcctt gatctgctta aaagccacct 22920
   gagcctttgc gccttcagag aagaacatgc cgcaagactt gccggaaaac tgattggccg 22980
   gacaggccgc gtcgtgcacg cagcaccttg cgtcggtgtt ggagatctgc accacatttc 23040
   ggccccaccg gttcttcacg atcttggcct tgctagactg ctccttcagc gcgcgctgcc 23100
   cgttttcgct cgtcacatcc atttcaatca cgtgctcctt atttatcata atgcttccgt 23160
   gtagacactt aagctcgcct tcgatctcag cgcagcggtg cagccacaac gcgcagcccg 23220
   tgggctcgtg atgcttgtag gtcacctctg caaacgactg caggtacgcc tgcaggaatc 23280
   gccccatcat cgtcacaaag gtcttgttgc tggtgaaggt cagctgcaac ccgcggtgct 23340
   cctcgttcag ccaggtcttg catacggccg ccagagcttc cacttggtca ggcagtagtt 23400
   tgaagttcgc ctttagatcg ttatccacgt ggtacttgtc catcagcgcg cgcgcagcct 23460
   ccatgccctt ctcccacgca gacacgatcg gcacactcag cgggttcatc accgtaattt 23520
   cactttccgc ttcgctgggc tcttcctctt cctcttgcgt ccgcatacca cgcgccactg 23580
   ggtcgtcttc attcagccgc cgcactgtgc gcttacctcc tttgccatgc ttgattagca 23640
   ccggtgggtt gctgaaaccc accatttgta gcgccacatc ttctctttct tcctcgctgt 23700
   ccacgattac ctctggtgat ggcgggcgct cgggcttggg agaagggcgc ttctttttct 23760
   tcttgggcgc aatggccaaa tccgccgccg aggtcgatgg ccgcgggctg ggtgtgcgcg 23820
   gcaccagcgc gtcttgtgat gagtcttcct cgtcctcgga ctcgatacgc cgcctcatcc 23880
   gcttttttgg gggcgcccgg ggaggcggcg gcgacgggga cggggacgac acgtcctcca 23940
   tggttggggg acgtcgcgcc gcaccgcgtc cgcgctcggg ggtggtttcg cgctgctcct 24000
   cttcccgact ggccatttcc ttctcctata ggcagaaaaa gatcatggag tcagtcgaga 24060
   agaaggacag cctaaccgcc ccctctgagt tcgccaccac cgcctccacc gatgccgcca 24120
   acgcgcctac caccttcccc gtcgaggcac ccccgcttga ggaggaggaa gtgattatcg 24180
   agcaggaccc aggttttgta agcgaagacg acgaggaccg ctcagtacca acagaggata 24240
   aaaagcaaga ccaggacaac gcagaggcaa acgaggaaca agtcgggcgg ggggacgaaa 24300
   ggcatggcga ctacctagat gtgggagacg acgtgctgtt gaagcatctg cagcgccagt 24360
   gcgccattat ctgcgacgcg ttgcaagagc gcagcgatgt gcccctcgcc atagcggatg 24420
   tcagccttgc ctacgaacgc cacctattct caccgcgcgt accccccaaa cgccaagaaa 24480
   acggcacatg cgagcccaac ccgcgcctca acttctaccc cgtatttgcc gtgccagagg 24540
   tgcttgccac ctatcacatc tttttccaaa actgcaagat acccctatcc tgccgtgcca 24600
   accgcagccg agcggacaag cagctggcct tgcggcaggg cgctgtcata cctgatatcg 24660
   cctcgctcaa cgaagtgcca aaaatctttg agggtcttgg acgcgacgag aagcgcgcgg 24720
   caaacgctct gcaacaggaa aacagcgaaa atgaaagtca ctctggagtg ttggtggaac 24780
   tcgagggtga caacgcgcgc ctagccgtac taaaacgcag catcgaggtc acccactttg 24840
   cctacccggc acttaaccta ccccccaagg tcatgagcac agtcatgagt gagctgatcg 24900
   tgcgccgtgc gcagcccctg gagagggatg caaatttgca agaacaaaca gaggagggcc 24960
   tacccgcagt tggcgacgag cagctagcgc gctggcttca aacgcgcgag cctgccgact 25020
   tggaggagcg acgcaaacta atgatggccg cagtgctcgt taccgtggag cttgagtgca 25080
   tgcagcggtt ctttgctgac ccggagatgc agcgcaagct agaggaaaca ttgcactaca 25140
   cctttcgaca gggctacgta cgccaggcct gcaagatctc caacgtggag ctctgcaacc 25200
   tggtctccta ccttggaatt ttgcacgaaa accgccttgg gcaaaacgtg cttcattcca 25260
   cgctcaaggg cgaggcgcgc cgcgactacg tccgcgactg cgtttactta tttctatgct 25320
   acacctggca gacggccatg ggcgtttggc agcagtgctt ggaggagtgc aacctcaagg 25380
   agctgcagaa actgctaaag caaaacttga aggacctatg gacggccttc aacgagcgct 25440
   ccgtggccgc gcacctggcg gacatcattt tccccgaacg cctgcttaaa accctgcaac 25500
   agggtctgcc agacttcacc agtcaaagca tgttgcagaa ctttaggaac tttatcctag 25560
   agcgctcagg aatcttgccc gccacctgct gtgcacttcc tagcgacttt gtgcccatta 25620
   agtaccgcga atgccctccg ccgctttggg gccactgcta ccttctgcag ctagccaact 25680
   accttgccta ccactctgac ataatggaag acgtgagcgg tgacggtcta ctggagtgtc 25740
   actgtcgctg caacctatgc accccgcacc gctccctggt ttgcaattcg cagctgctta 25800
   acgaaagtca aattatcggt acctttgagc tgcagggtcc ctcgcctgac gaaaagtccg 25860
   cggctccggg gttgaaactc actccggggc tgtggacgtc ggcttacctt cgcaaatttg 25920
   tacctgagga ctaccacgcc cacgagatta ggttctacga agaccaatcc cgcccgccaa 25980
   atgcggagct taccgcctgc gtcattaccc agggccacat tcttggccaa ttgcaagcca 26040
   tcaacaaagc ccgccaagag tttctgctac gaaagggacg gggggtttac ttggaccccc 26100
   agtccggcga ggagctcaac ccaatccccc cgccgccgca gccctatcag cagcagccgc 26160
   gggcccttgc ttcccaggat ggcacccaaa aagaagctgc agctgccgcc gccacccacg 26220
   gacgaggagg aatactggga cagtcaggca gaggaggttt tggacgagga ggaggaggac 26280
   atgatggaag actgggagag cctagacgag gaagcttccg aggtcgaaga ggtgtcagac 26340
   gaaacaccgt caccctcggt cgcattcccc tcgccggcgc cccagaaatc ggcaaccggt 26400
   tccagcatgg ctacaacctc cgctcctcag gcgccgccgg cactgcccgt tcgccgaccc 26460
   aaccgtagat gggacaccac tggaaccagg gccggtaagt ccaagcagcc gccgccgtta 26520
   gcccaagagc aacaacagcg ccaaggctac cgctcatggc gcgggcacaa gaacgccata 26580
   gttgcttgct tgcaagactg tgggggcaac atctccttcg cccgccgctt tcttctctac 26640
   catcacggcg tggccttccc ccgtaacatc ctgcattact accgtcatct ctacagccca 26700
   tactgcaccg gcggcagcgg cagcggcagc aacagcagcg gccacacaga agcaaaggcg 26760
   accggatagc aagactctga caaagcccaa gaaatccaca gcggcggcag cagcaggagg 26820
   aggagcgctg cgtctggcgc ccaacgaacc cgtatcgacc cgcgagctta gaaacaggat 26880
   ttttcccact ctgtatgcta tatttcaaca gagcaggggc caagaacaag agctgaaaat 26940
   aaaaaacagg tctctgcgat ccctcacccg cagctgcctg tatcacaaaa gcgaagatca 27000
   gcttcggcgc acgctggaag acgcggaggc tctcttcagt aaatactgcg cgctgactct 27060
   taaggactag tttcgcgccc tttctcaaat ttaagcgcga aaactacgtc atctccagcg 27120
   gccacacccg gcgccagcac ctgtcgtcag cgccattatg agcaaggaaa ttcccacgcc 27180
   ctacatgtgg agttaccagc cacaaatggg acttgcggct ggagctgccc aagactactc 27240
   aacccgaata aactacatga gcgcgggacc ccacatgata tcccgggtca acggaatccg 27300
   cgcccaccga aaccgaattc tcttggaaca ggcggctatt accaccacac ctcgtaataa 27360
   ccttaatccc cgtagttggc ccgctgccct ggtgtaccag gaaagtcccg ctcccaccac 27420
   tgtggtactt cccagagacg cccaggccga agttcagatg actaactcag gggcgcagct 27480
   tgcgggcggc tttcgtcaca gggtgcggtc gcccgggcag ggtataactc acctgacaat 27540
   cagagggcga ggtattcagc tcaacgacga gtcggtgagc tcctcgcttg gtctccgtcc 27600
   ggacgggaca tttcagatcg gcggcgccgg ccgctcttca ttcacgcctc gtcaggcaat 27660
   cctaactctg cagacctcgt cctctgagcc gcgctctgga ggcattggaa ctctgcaatt 27720
   tattgaggag tttgtgccat cggtctactt taaccccttc tcgggacctc ccggccacta 27780
   tccggatcaa tttattccta actttgacgc ggtaaaggac tcggcggatg gctacgactg 27840
   aatgttaagt ggagaggcag agcaactgcg cctgaaacac ctggtccact gtcgccgcca 27900
   caagtgcttt gcccgcgact ccggtgagtt ttgctacttt gaattgcccg aggatcatat 27960
   cgagggcccg gcgcacggcg tccggcttac cgcccaggga gagcttgccc gtagcctgat 28020
   tcgggagttt acccagcgcc ccctgctagt tgagcgggac aggggaccct gtgttctcac 28080
   tgtgatttgc aactgtccta accctggatt acatcaagat ctttgttgcc atctctgtgc 28140
   tgagtataat aaatacagaa attaaaatat actggggctc ctatcgccat cctgtaaacg 28200
   ccaccgtctt cacccgccca agcaaaccaa ggcgaacctt acctggtact tttaacatct 28260
   ctccctctgt gatttacaac agtttcaacc cagacggagt gagtctacga gagaacctct 28320
   ccgagctcag ctactccatc agaaaaaaca ccaccctcct tacctgccgg gaacgtacga 28380
   tgtggctgca gagcctgctg ctcttgggca ctgtggcctg cagcatctct gcacccgccc 28440
   gctcgcccag ccccagcacg cagccctggg agcatgtgaa tgccatccag gaggcccggc 28500
   gtctcctgaa cctgagtaga gacactgctg ctgagatgaa tgaaacagta gaagtcatct 28560
   cagaaatgtt tgacctccag gagccgacct gcctacagac ccgcctggag ctgtacaagc 28620
   agggcctgcg gggcagcctc accaagctca agggcccctt gaccatgatg gccagccact 28680
   acaagcagca ctgccctcca accccggaaa cttcctgtgc aacccagact atcacctttg 28740
   aaagtttcaa agagaacctg aaggactttc tgcttgtcat cccctttgac tgctgggagc 28800
   cagtccagga gtgacaattg actctatgtg ggatatgctc cagcgctaca accttgaagt 28860
   caggcttcct ggatgtcagc atctgacttt ggccagcacc tgtcccgcgg atttgttcca 28920
   gtccaactac agcgacccac cctaacagag atgaccaaca caaccaacgc ggccgccgct 28980
   accggactta catctaccac aaatacaccc caagtttctg cctttgtcaa taactgggat 29040
   aacttgggca tgtggtggtt ctccatagcg cttatgtttg tatgccttat tattatgtgg 29100
   ctcatctgct gcctaaagcg caaacgcgcc cgaccaccca tctatagtcc catcattgtg 29160
   ctacacccaa acaatgatgg aatccataga ttggacggac tgaaacacat gttcttttct 29220
   cttacagtat gattaaatga gacatgattc ctcgagtttt tatattactg acccttgttg 29280
   cgcttttttg tgcgtgctcc acattggctg cggtttctca catcgaagta gactgcattc 29340
   cagccttcac agtctatttg ctttacggat ttgtcaccct cacgctcatc tgcagcctca 29400
   tcactgtggt catcgccttt atccagtgca ttgactgggt ctgtgtgcgc tttgcatatc 29460
   tcagacacca tccccagtac agggacagga ctatagctga gcttcttaga attctttaat 29520
   tatgaaattt actgtgactt ttctgctgat tatttgcacc ctatctgcgt tttgttcccc 29580
   gacctccaag cctcaaagac atatatcatg cagattcact cgtatatgga atattccaag 29640
   ttgctacaat gaaaaaagcg atctttccga agcctggtta tatgcaatca tctctgttat 29700
   ggtgttctgc agtaccatct tagccctagc tatatatccc taccttgaca ttggctggaa 29760
   acgaatagat gccatgaacc acccaacttt ccccgcgccc gctatgcttc cactgcaaca 29820
   agttgttgcc ggcggctttg tcccagccaa tcagcctcgc cccacttctc ccacccccac 29880
   tgaaatcagc tactttaatc taacaggagg agatgactga caccctagat ctagaaatgg 29940
   acggaattat tacagagcag cgcctgctag aaagacgcag ggcagcggcc gagcaacagc 30000
   gcatgaatca agagctccaa gacatggtta acttgcacca gtgcaaaagg ggtatctttt 30060
   gtctggtaaa gcaggccaaa gtcacctacg acagtaatac caccggacac cgccttagct 30120
   acaagttgcc aaccaagcgt cagaaattgg tggtcatggt gggagaaaag cccattacca 30180
   taactcagca ctcggtagaa accgaaggct gcattcactc accttgtcaa ggacctgagg 30240
   atctctgcac ccttattaag accctgtgcg gtctcaaaga tcttattccc tttaactaat 30300
   aaaaaaaaat aataaagcat cacttactta aaatcagtta gcaaatttct gtccagttta 30360
   ttcagcagca cctccttgcc ctcctcccag ctctggtatt gcagcttcct cctggctgca 30420
   aactttctcc acaatctaaa tggaatgtca gtttcctcct gttcctgtcc atccgcaccc 30480
   actatcttca tgttgttgca gatgaagcgc gcaagaccgt ctgaagatac cttcaacccc 30540
   gtgtatccat atgacacgga aaccggtcct ccaactgtgc cttttcttac tcctcccttt 30600
   gtatccccca atgggtttca agagagtccc cctggggtac tctctttgcg cctatccgaa 30660
   cctctagtta cctccaatgg catgcttgcg ctcaaaatgg gcaacggcct ctctctggac 30720
   gaggccggca accttacctc ccaaaatgta accactgtga gcccacctct caaaaaaacc 30780
   aagtcaaaca taaacctgga aatatctgca cccctcacag ttacctcaga agccctaact 30840
   gtggctgccg ccgcacctct aatggtcgcg ggcaacacac tcaccatgca atcacaggcc 30900
   ccgctaaccg tgcacgactc caaacttagc attgccaccc aaggacccct cacagtgtca 30960
   gaaggaaagc tagccctgca aacatcaggc cccctcacca ccaccgatag cagtaccctt 31020
   actatcactg cctcaccccc tctaactact gccactggta gcttgggcat tgacttgaaa 31080
   gagcccattt atacacaaaa tggaaaacta ggactaaagt acggggctcc tttgcatgta 31140
   acagacgacc taaacacttt gaccgtagca actggtccag gtgtgactat taataatact 31200
   tccttgcaaa ctaaagttac tggagccttg ggttttgatt cacaaggcaa tatgcaactt 31260
   aatgtagcag gaggactaag gattgattct caaaacagac gccttatact tgatgttagt 31320
   tatccgtttg atgctcaaaa ccaactaaat ctaagactag gacagggccc tctttttata 31380
   aactcagccc acaacttgga tattaactac aacaaaggcc tttacttgtt tacagcttca 31440
   aacaattcca aaaagcttga ggttaaccta agcactgcca aggggttgat gtttgacgct 31500
   acagccatag ccattaatgc aggagatggg cttgaatttg gttcacctaa tgcaccaaac 31560
   acaaatcccc tcaaaacaaa aattggccat ggcctagaat ttgattcaaa caaggctatg 31620
   gttcctaaac taggaactgg ccttagtttt gacagcacag gtgccattac agtaggaaac 31680
   aaaaataatg ataagctaac cctatggaca ggtccaaaac cagaagccaa ctgcataatt 31740
   gaatacggga aacaaaaccc agatagcaaa ctaactttaa tccttgtaaa aaatggagga 31800
   attgttaatg gatatgtaac gctaatggga gcctcagact acgttaacac cttatttaaa 31860
   aacaaaaatg tctccattaa tgtagaacta tactttgatg ccactggtca tatattacca 31920
   gactcatctt ctcttaaaac agatctagaa ctaaaataca agcaaaccgc tgactttagt 31980
   gcaagaggtt ttatgccaag tactacagcg tatccatttg tccttcctaa tgcgggaaca 32040
   cataatgaaa attatatttt tggtcaatgc tactacaaag caagcgatgg tgcccttttt 32100
   ccgttggaag ttactgttat gcttaataaa cgcctgccag atagtcgcac atcctatgtt 32160
   atgacttttt tatggtcctt gaatgctggt ctagctccag aaactactca ggcaaccctc 32220
   ataacctccc catttacctt ttcctatatt agagaagatg actaataaac tctaaagaat 32280
   cgtttgtgtt atgtttcaac gtgtttattt ttcaattgca gaaaatttca agtcattttt 32340
   cattcagtag tatagcccca ccaccacata gcttatacag atcaccgtac cttaatcaaa 32400
   ctcacagaac cctagtattc aacctgccac ctccctccca acacacagag tacacagtcc 32460
   tttctccccg gctggcctta aaaagcatca tatcatgggt aacagacata ttcttaggtg 32520
   ttatattcca cacggtttcc tgtcgagcca aacgctcatc aagtgatatt aataaactcc 32580
   ccgggcagct cacttaagtt catgtcgctg tccagctgct gagccacagg ctgctgtcca 32640
   acttgcggtt gcttaacggg cggcgaagga gaagtccacg cctacatggg gggagagtca 32700
   taatcgtgca tcaggatagg gcggtggtgc tgcagcagcg cgcgaataaa ctgctgccgc 32760
   cgccgctccg tcctgcagga atacaacatg gcagtggtct cctcagcgat gattcgcacc 32820
   gcccgcagca taaggcgctt gtcctccggg cacagcagcg caccctgatc tcacttaaat 32880
   cagcacagta actgcagcac agcaccacaa tattgttcaa aatcccacag tgcaaggcgc 32940
   tgtatccaaa gctcatggcg gggaccacag aacccacgtg gccatcatac cacaagcgca 33000
   ggtagattaa gtggcgaccc ctcataaaca cgctggacat aaacattacc tcttttggca 33060
   tgttgtaatt caccacctcc cggtaccata taaacctctg attaaacatg gcgccatcca 33120
   ccaccatcct aaaccagctg gccaaaacct gccccgccgg gntatacact gcagggaacc 33180
   gggacttgga caatgacaag tgggagagcc caggactcgt aaccatggat catcatgctc 33240
   gtcatgatat caatgttggc acaacacagg cacacgtgca tacacttcct caggattaca 33300
   agctcctccc gcgttagaac catatcccag ggaacaaccc attcctgaat cagcgtaaat 33360
   cccacactgc agggaagacc tcgcacgtaa ctcacgttgt gcattgtcaa agtgttacat 33420
   tcgggcagca gcggatgatc ctccagtatg gtagcgcggg tttctgtctc aaaaggaggt 33480
   agacgatccc tactgtacgg agtgcgccga gacaaccgag atcgtgttgg tcgtagtgtc 33540
   atgccaaatg gaacgccgga cgtagtcata tttcctgaag caaaaccagg tgcgggcgtg 33600
   acaaacagat ctgcgtctcc ggtctcgccg cttagatcgc tctgtgtagt agttgtagta 33660
   tatccactct ctcaaagcat ccaggcgccc cctggcttcg ggttctatgt aaactccttc 33720
   atgcgccgct gccctgataa catccaccac cgcagaataa gccacaccca gccaacctac 33780
   acattcgttc tgcgagtcac acacgggagg agcgggaaga gctggaagaa ccatgttttt 33840
   ttttttattc caaaagatta tccaaaacct caaaatgaag atctattaag tgaacgcgct 33900
   cccctccggt ggcgtggtca aactctacag ccaaagaaca gataatggca tttgtaagat 33960
   gttgcacaat ggcttccaaa aggcaaacgg ccctcacgtc caagtggacg taaaggctaa 34020
   acccttcagg gtgaatctcc tctataaaca ttccagcacc ttcaaccatg cccaaataat 34080
   tctcatctcg ccaccttctc aatatatctc taagcaaatc ccgaatattt aagtccgggc 34140
   cattgtaaaa aatttggctc cagagcgccc tccaccttca gcctcaagca gcgaatcatg 34200
   attgcaaaaa ttcaggttcc tcacagacct gtataagatt caaaagcgga acattaacaa 34260
   aaataccgcg atcccgtagg tcccttcgca gggccagctg aacataatcg tgcaggtctg 34320
   cacggaccag cgcggccact tccccgccag gaaccatgac aaaagaaccc acactgatta 34380
   tgacacgcat actcggagct atgctaacca gcgtagcccc gatgtaagct tgttgcatgg 34440
   gcggcgatat aaaatgcaag gtgctgctca aaaaatcagg caaagcctcg cgcaaaaaag 34500
   aaagcacatc gtagtcatgc tcatgcagat aaaggcaggt aagctccgga accaccacag 34560
   aaaaagacac catttttctc tcaaacatgt ctgcgggttt ctgcataaac acaaaataaa     34620
   ataacaaaaa aacatttaaa cattagaagc ctgtcttaca acaggaaaaa caacccttat     34680
   aagcataaga cggactacgg ccatgccggc gtgaccgtaa aaaaactggt caccgtgatt     34740
   aaaaagcacc accgacagct cctcggtcag tccggagtca taatgtaaga ctcggtaaac     34800
   acatcaggtt gattcacatc ggtcagtgtt aaaaagcgac cgaaatagcc ngggggaata     34860
   caatacccgc aggcgtagag acaacattac agcccccata ggaggtataa caaaattaat     34920
   aggagagaaa aacacataaa cacctgaaaa accctcctgc ctaggcaaaa tagcaccctc     34980
   ccgctccaga acaacataca gcgcttccac agcggcagcc ataacagtca gccttaccag     35040
   taaaaaagaa aacctattaa aaaaacacca ctcgacacgg caccagctca atcagtcaca     35100
   gtgtaaaaaa gggccaagtg cagagcgagt atatatagga ctaaaaaatg acggtaacgg     35160
   ttaaagtcca caaaaaacac ccagaaaacc gcacgcgaac ctacgcccag aaacgaaagc     35220
   caaaaaaccc acaacttcct caaatcgtca cttccgtttt cccacgttac gtcacttccc     35280
   attttaagaa aactacaatt cccaacacat acaagttact ccgccctaaa acctacgtca     35340
   cccgccccgt tcccacgccc cgcgccacgt cacaaactcc accccctcat tatcatattg     35400
   gcttcaatcc aaaataaggt atattattga tgatgttaat                           35440
<210> 8
   <211> 38303
   <212> DNA
   <213> Artificial Sequence
<220>
   <223> Ad5-D24-GMCSF
<220>
   <221> misc_feature
   <222> (36714)..(36719)
   <223> n is a, c, g, or t
<400> 8
<210> 9
   <211> 37433
   <212> DNA
   <213> Artificial Sequence
<220>
   <223> Ad5-RGD-D24-GMCSF
<220>
   <221> misc_feature
   <222> (36682)..(36682)
   <223> n is a, c, g, or t
<400> 9
   ggatccgaat tcttaattaa catcatcaat aatatacctt attttggatt gaagccaata      60
   tgataatgag ggggtggagt ttgtgacgtg gcgcggggcg tgggaacggg gcgggtgacg     120
   tagtagtgtg gcggaagtgt gatgttgcaa gtgtggcgga acacatgtaa gcgacggatg     180
   tggcaaaagt gacgtttttg gtgtgcgccg gtgtacacag gaagtgacaa ttttcgcgcg     240
   gttttaggcg gatgttgtag taaatttggg cgtaaccgag taagatttgg ccattttcgc     300
   gggaaaactg aataagagga agtgaaatct gaataatttt gtgttactca tagcgcgtaa     360
   tatttgtcta gggccgcggg gactttgacc gtttacgtgg agactcgccc aggtgttttt     420
   ctcaggtgtt ttccgcgttc cgggtcaaag ttggcgtttt attattatag tcagctgacg     480
   tgtagtgtat ttatacccgg tgagttcctc aagaggccac tcttgagtgc cagcgagtag     540
   agttttctcc tccgagccgc tccgacaccg ggactgaaaa tgagacatat tatctgccac     600
   ggaggtgtta ttaccgaaga aatggccgcc agtcttttgg accagctgat cgaagaggta     660
   ctggctgata atcttccacc tcctagccat tttgaaccac ctacccttca cgaactgtat     720
   gatttagacg tgacggcccc cgaagatccc aacgaggagg cggtttcgca gatttttccc     780
   gactctgtaa tgttggcggt gcaggaaggg attgacttac tcacttttcc gccggcgccc     840
   ggttctccgg agccgcctca cctttcccgg cagcccgagc agccggagca gagagccttg     900
   ggtccggttt ctatgccaaa ccttgtaccg gaggtgatcg atccacccag tgacgacgag     960
   gatgaagagg gtgaggagtt tgtgttagat tatgtggagc accccgggca cggttgcagg    1020
   tcttgtcatt atcaccggag gaatacgggg gacccagata ttatgtgttc gctttgctat    1080
   atgaggacct gtggcatgtt tgtctacagt aagtgaaaat tatgggcagt gggtgataga    1140
   gtggtgggtt tggtgtggta attttttttt taatttttac agttttgtgg tttaaagaat    1200
   tttgtattgt gattttttta aaaggtcctg tgtctgaacc tgagcctgag cccgagccag    1260
   aaccggagcc tgcaagacct acccgccgtc ctaaaatggc gcctgctatc ctgagacgcc    1320
   cgacatcacc tgtgtctaga gaatgcaata gtagtacgga tagctgtgac tccggtcctt    1380
   ctaacacacc tcctgagata cacccggtgg tcccgctgtg ccccattaaa ccagttgccg 1440
   tgagagttgg tgggcgtcgc caggctgtgg aatgtatcga ggacttgctt aacgagcctg 1500
   ggcaaccttt ggacttgagc tgtaaacgcc ccaggccata aggtgtaaac ctgtgattgc 1560
   gtgtgtggtt aacgcctttg tttgctgaat gagttgatgt aagtttaata aagggtgaga 1620
   taatgtttaa cttgcatggc gtgttaaatg gggcggggct taaagggtat ataatgcgcc 1680
   gtgggctaat cttggttaca tctgacctca tggaggcttg ggagtgtttg gaagattttt 1740
   ctgctgtgcg taacttgctg gaacagagct ctaacagtac ctcttggttt tggaggtttc 1800
   tgtggggctc atcccaggca aagttagtct gcagaattaa ggaggattac aagtgggaat 1860
   ttgaagagct tttgaaatcc tgtggtgagc tgtttgattc tttgaatctg ggtcaccagg 1920
   cgcttttcca agagaaggtc atcaagactt tggatttttc cacaccgggg cgcgctgcgg 1980
   ctgctgttgc ttttttgagt tttataaagg ataaatggag cgaagaaacc catctgagcg 2040
   gggggtacct gctggatttt ctggccatgc atctgtggag agcggttgtg agacacaaga 2100
   atcgcctgct actgttgtct tccgtccgcc cggcgataat accgacggag gagcagcagc 2160
   agcagcagga ggaagccagg cggcggcggc aggagcagag cccatggaac ccgagagccg 2220
   gcctggaccc tcgggaatga atgttgtaca ggtggctgaa ctgtatccag aactgagacg 2280
   cattttgaca attacagagg atgggcaggg gctaaagggg gtaaagaggg agcggggggc 2340
   ttgtgaggct acagaggagg ctaggaatct agcttttagc ttaatgacca gacaccgtcc 2400
   tgagtgtatt acttttcaac agatcaagga taattgcgct aatgagcttg atctgctggc 2460
   gcagaagtat tccatagagc agctgaccac ttactggctg cagccagggg atgattttga 2520
   ggaggctatt agggtatatg caaaggtggc acttaggcca gattgcaagt acaagatcag 2580
   caaacttgta aatatcagga attgttgcta catttctggg aacggggccg aggtggagat 2640
   agatacggag gatagggtgg cctttagatg tagcatgata aatatgtggc cgggggtgct 2700
   tggcatggac ggggtggtta ttatgaatgt aaggtttact ggccccaatt ttagcggtac 2760
   ggttttcctg gccaatacca accttatcct acacggtgta agcttctatg ggtttaacaa 2820
   tacctgtgtg gaagcctgga ccgatgtaag ggttcggggc tgtgcctttt actgctgctg 2880
   gaagggggtg gtgtgtcgcc ccaaaagcag ggcttcaatt aagaaatgcc tctttgaaag 2940
   gtgtaccttg ggtatcctgt ctgagggtaa ctccagggtg cgccacaatg tggcctccga 3000
   ctgtggttgc ttcatgctag tgaaaagcgt ggctgtgatt aagcataaca tggtatgtgg 3060
   caactgcgag gacagggcct ctcagatgct gacctgctcg gacggcaact gtcacctgct 3120
   gaagaccatt cacgtagcca gccactctcg caaggcctgg ccagtgtttg agcataacat 3180
   actgacccgc tgttccttgc atttgggtaa caggaggggg gtgttcctac cttaccaatg 3240
   caatttgagt cacactaaga tattgcttga gcccgagagc atgtccaagg tgaacctgaa 3300
   cggggtgttt gacatgacca tgaagatctg gaaggtgctg aggtacgatg agacccgcac 3360
   caggtgcaga ccctgcgagt gtggcggtaa acatattagg aaccagcctg tgatgctgga 3420
   tgtgaccgag gagctgaggc ccgatcactt ggtgctggcc tgcacccgcg ctgagtttgg 3480
   ctctagcgat gaagatacag attgaggtac tgaaatgtgt gggcgtggct taagggtggg 3540
   aaagaatata taaggtgggg gtcttatgta gttttgtatc tgttttgcag cagccgccgc 3600
   cgccatgagc accaactcgt ttgatggaag cattgtgagc tcatatttga caacgcgcat 3660
   gcccccatgg gccggggtgc gtcagaatgt gatgggctcc agcattgatg gtcgccccgt 3720
   cctgcccgca aactctacta ccttgaccta cgagaccgtg tctggaacgc cgttggagac 3780
   tgcagcctcc gccgccgctt cagccgctgc agccaccgcc cgcgggattg tgactgactt 3840
   tgctttcctg agcccgcttg caagcagtgc agcttcccgt tcatccgccc gcgatgacaa 3900
   gttgacggct cttttggcac aattggattc tttgacccgg gaacttaatg tcgtttctca 3960
   gcagctgttg gatctgcgcc agcaggtttc tgccctgaag gcttcctccc ctcccaatgc 4020
   ggtttaaaac ataaataaaa aaccagactc tgtttggatt tggatcaagc aagtgtcttg 4080
   ctgtctttat ttaggggttt tgcgcgcgcg gtaggcccgg gaccagcggt ctcggtcgtt 4140
   gagggtcctg tgtatttttt ccaggacgtg gtaaaggtga ctctggatgt tcagatacat 4200
   gggcataagc ccgtctctgg ggtggaggta gcaccactgc agagcttcat gctgcggggt 4260
   ggtgttgtag atgatccagt cgtagcagga gcgctgggcg tggtgcctaa aaatgtcttt 4320
   cagtagcaag ctgattgcca ggggcaggcc cttggtgtaa gtgtttacaa agcggttaag 4380
   ctgggatggg tgcatacgtg gggatatgag atgcatcttg gactgtattt ttaggttggc 4440
   tatgttccca gccatatccc tccggggatt catgttgtgc agaaccacca gcacagtgta 4500
   tccggtgcac ttgggaaatt tgtcatgtag cttagaagga aatgcgtgga agaacttgga 4560
   gacgcccttg tgacctccaa gattttccat gcattcgtcc ataatgatgg caatgggccc 4620
   acgggcggcg gcctgggcga agatatttct gggatcacta acgtcatagt tgtgttccag 4680
   gatgagatcg tcataggcca tttttacaaa gcgcgggcgg agggtgccag actgcggtat 4740
   aatggttcca tccggcccag gggcgtagtt accctcacag atttgcattt cccacgcttt 4800
   gagttcagat ggggggatca tgtctacctg cggggcgatg aagaaaacgg tttccggggt 4860
   aggggagatc agctgggaag aaagcaggtt cctgagcagc tgcgacttac cgcagccggt 4920
   gggcccgtaa atcacaccta ttaccgggtg caactggtag ttaagagagc tgcagctgcc 4980
   gtcatccctg agcagggggg ccacttcgtt aagcatgtcc ctgactcgca tgttttccct 5040
   gaccaaatcc gccagaaggc gctcgccgcc cagcgatagc agttcttgca aggaagcaaa 5100
   gtttttcaac ggtttgagac cgtccgccgt aggcatgctt ttgagcgttt gaccaagcag 5160
   ttccaggcgg tcccacagct cggtcacctg ctctacggca tctcgatcca gcatatctcc 5220
   tcgtttcgcg ggttggggcg gctttcgctg tacggcagta gtcggtgctc gtccagacgg 5280
   gccagggtca tgtctttcca cgggcgcagg gtcctcgtca gcgtagtctg ggtcacggtg 5340
   aaggggtgcg ctccgggctg cgcgctggcc agggtgcgct tgaggctggt cctgctggtg 5400
   ctgaagcgct gccggtcttc gccctgcgcg tcggccaggt agcatttgac catggtgtca 5460
   tagtccagcc cctccgcggc gtggcccttg gcgcgcagct tgcccttgga ggaggcgccg 5520
   cacgaggggc agtgcagact tttgagggcg tagagcttgg gcgcgagaaa taccgattcc 5580
   ggggagtagg catccgcgcc gcaggccccg cagacggtct cgcattccac gagccaggtg 5640
   agctctggcc gttcggggtc aaaaaccagg tttcccccat gctttttgat gcgtttctta 5700
   cctctggttt ccatgagccg gtgtccacgc tcggtgacga aaaggctgtc cgtgtccccg 5760
   tatacagact tgagaggcct gtcctcgagc ggtgttccgc ggtcctcctc gtatagaaac 5820
   tcggaccact ctgagacaaa ggctcgcgtc caggccagca cgaaggaggc taagtgggag 5880
   gggtagcggt cgttgtccac tagggggtcc actcgctcca gggtgtgaag acacatgtcg 5940
   ccctcttcgg catcaaggaa ggtgattggt ttgtaggtgt aggccacgtg accgggtgtt 6000
   cctgaagggg ggctataaaa gggggtgggg gcgcgttcgt cctcactctc ttccgcatcg 6060
   ctgtctgcga gggccagctg ttggggtgag tactccctct gaaaagcggg catgacttct 6120
   gcgctaagat tgtcagtttc caaaaacgag gaggatttga tattcacctg gcccgcggtg 6180
   atgcctttga gggtggccgc atccatctgg tcagaaaaga caatcttttt gttgtcaagc 6240
   ttggtggcaa acgacccgta gagggcgttg gacagcaact tggcgatgga gcgcagggtt 6300
   tggtttttgt cgcgatcggc gcgctccttg gccgcgatgt ttagctgcac gtattcgcgc 6360
   gcaacgcacc gccattcggg aaagacggtg gtgcgctcgt cgggcaccag gtgcacgcgc 6420
   caaccgcggt tgtgcagggt gacaaggtca acgctggtgg ctacctctcc gcgtaggcgc 6480
   tcgttggtcc agcagaggcg gccgcccttg cgcgagcaga atggcggtag ggggtctagc 6540
   tgcgtctcgt ccggggggtc tgcgtccacg gtaaagaccc cgggcagcag gcgcgcgtcg 6600
   aagtagtcta tcttgcatcc ttgcaagtct agcgcctgct gccatgcgcg ggcggcaagc 6660
   gcgcgctcgt atgggttgag tgggggaccc catggcatgg ggtgggtgag cgcggaggcg 6720
   tacatgccgc aaatgtcgta aacgtagagg ggctctctga gtattccaag atatgtaggg 6780
   tagcatcttc caccgcggat gctggcgcgc acgtaatcgt atagttcgtg cgagggagcg 6840
   aggaggtcgg gaccgaggtt gctacgggcg ggctgctctg ctcggaagac tatctgcctg 6900
   aagatggcat gtgagttgga tgatatggtt ggacgctgga agacgttgaa gctggcgtct 6960
   gtgagaccta ccgcgtcacg cacgaaggag gcgtaggagt cgcgcagctt gttgaccagc 7020
   tcggcggtga cctgcacgtc tagggcgcag tagtccaggg tttccttgat gatgtcatac 7080
   ttatcctgtc cctttttttt ccacagctcg cggttgagga caaactcttc gcggtctttc 7140
   cagtactctt ggatcggaaa cccgtcggcc tccgaacggt aagagcctag catgtagaac 7200
   tggttgacgg cctggtaggc gcagcatccc ttttctacgg gtagcgcgta tgcctgcgcg 7260
   gccttccgga gcgaggtgtg ggtgagcgca aaggtgtccc tgaccatgac tttgaggtac 7320
   tggtatttga agtcagtgtc gtcgcatccg ccctgctccc agagcaaaaa gtccgtgcgc 7380
   tttttggaac gcggatttgg cagggcgaag gtgacatcgt tgaagagtat ctttcccgcg 7440
   cgaggcataa agttgcgtgt gatgcggaag ggtcccggca cctcggaacg gttgttaatt 7500
   acctgggcgg cgagcacgat ctcgtcaaag ccgttgatgt tgtggcccac aatgtaaagt 7560
   tccaagaagc gcgggatgcc cttgatggaa ggcaattttt taagttcctc gtaggtgagc 7620
   tcttcagggg agctgagccc gtgctctgaa agggcccagt ctgcaagatg agggttggaa 7680
   gcgacgaatg agctccacag gtcacgggcc attagcattt gcaggtggtc gcgaaaggtc 7740
   ctaaactggc gacctatggc cattttttct ggggtgatgc agtagaaggt aagcgggtct 7800
   tgttcccagc ggtcccatcc aaggttcgcg gctaggtctc gcgcggcagt cactagaggc 7860
   tcatctccgc cgaacttcat gaccagcatg aagggcacga gctgcttccc aaaggccccc 7920
   atccaagtat aggtctctac atcgtaggtg acaaagagac gctcggtgcg aggatgcgag 7980
   ccgatcggga agaactggat ctcccgccac caattggagg agtggctatt gatgtggtga 8040
   aagtagaagt ccctgcgacg ggccgaacac tcgtgctggc ttttgtaaaa acgtgcgcag 8100
   tactggcagc ggtgcacggg ctgtacatcc tgcacgaggt tgacctgacg accgcgcaca 8160
   aggaagcaga gtgggaattt gagcccctcg cctggcgggt ttggctggtg gtcttctact 8220
   tcggctgctt gtccttgacc gtctggctgc tcgaggggag ttacggtgga tcggaccacc 8280
   acgccgcgcg agcccaaagt ccagatgtcc gcgcgcggcg gtcggagctt gatgacaaca 8340
   tcgcgcagat gggagctgtc catggtctgg agctcccgcg gcgtcaggtc aggcgggagc 8400
   tcctgcaggt ttacctcgca tagacgggtc agggcgcggg ctagatccag gtgataccta 8460
   atttccaggg gctggttggt ggcggcgtcg atggcttgca agaggccgca tccccgcggc 8520
   gcgactacgg taccgcgcgg cgggcggtgg gccgcggggg tgtccttgga tgatgcatct 8580
   aaaagcggtg acgcgggcga gcccccggag gtaggggggg ctccggaccc gccgggagag 8640
   ggggcagggg cacgtcggcg ccgcgcgcgg gcaggagctg gtgctgcgcg cgtaggttgc 8700
   tggcgaacgc gacgacgcgg cggttgatct cctgaatctg gcgcctctgc gtgaagacga 8760
   cgggcccggt gagcttgagc ctgaaagaga gttcgacaga atcaatttcg gtgtcgttga 8820
   cggcggcctg gcgcaaaatc tcctgcacgt ctcctgagtt gtcttgatag gcgatctcgg 8880
   ccatgaactg ctcgatctct tcctcctgga gatctccgcg tccggctcgc tccacggtgg 8940
   cggcgaggtc gttggaaatg cgggccatga gctgcgagaa ggcgttgagg cctccctcgt 9000
   tccagacgcg gctgtagacc acgccccctt cggcatcgcg ggcgcgcatg accacctgcg 9060
   cgagattgag ctccacgtgc cgggcgaaga cggcgtagtt tcgcaggcgc tgaaagaggt 9120
   agttgagggt ggtggcggtg tgttctgcca cgaagaagta cataacccag cgtcgcaacg 9180
   tggattcgtt gatatccccc aaggcctcaa ggcgctccat ggcctcgtag aagtccacgg 9240
   cgaagttgaa aaactgggag ttgcgcgccg acacggttaa ctcctcctcc agaagacgga 9300
   tgagctcggc gacagtgtcg cgcacctcgc gctcaaaggc tacaggggcc tcttcttctt 9360
   cttcaatctc ctcttccata agggcctccc cttcttcttc ttctggcggc ggtgggggag 9420
   gggggacacg gcggcgacga cggcgcaccg ggaggcggtc gacaaagcgc tcgatcatct 9480
   ccccgcggcg acggcgcatg gtctcggtga cggcgcggcc gttctcgcgg gggcgcagtt 9540
   ggaagacgcc gcccgtcatg tcccggttat gggttggcgg ggggctgcca tgcggcaggg 9600
   atacggcgct aacgatgcat ctcaacaatt gttgtgtagg tactccgccg ccgagggacc 9660
   tgagcgagtc cgcatcgacc ggatcggaaa acctctcgag aaaggcgtct aaccagtcac 9720
   agtcgcaagg taggctgagc accgtggcgg gcggcagcgg gcggcggtcg gggttgtttc 9780
   tggcggaggt gctgctgatg atgtaattaa agtaggcggt cttgagacgg cggatggtcg 9840
   acagaagcac catgtccttg ggtccggcct gctgaatgcg caggcggtcg gccatgcccc 9900
   aggcttcgtt ttgacatcgg cgcaggtctt tgtagtagtc ttgcatgagc ctttctaccg 9960
   gcacttcttc ttctccttcc tcttgtcctg catctcttgc atctatcgct gcggcggcgg 10020
   cggagtttgg ccgtaggtgg cgccctcttc ctcccatgcg tgtgaccccg aagcccctca 10080
   tcggctgaag cagggctagg tcggcgacaa cgcgctcggc taatatggcc tgctgcacct 10140
   gcgtgagggt agactggaag tcatccatgt ccacaaagcg gtggtatgcg cccgtgttga 10200
   tggtgtaagt gcagttggcc ataacggacc agttaacggt ctggtgaccc ggctgcgaga 10260
   gctcggtgta cctgagacgc gagtaagccc tcgagtcaaa tacgtagtcg ttgcaagtcc 10320
   gcaccaggta ctggtatccc accaaaaagt gcggcggcgg ctggcggtag aggggccagc 10380
   gtagggtggc cggggctccg ggggcgagat cttccaacat aaggcgatga tatccgtaga 10440
   tgtacctgga catccaggtg atgccggcgg cggtggtgga ggcgcgcgga aagtcgcgga 10500
   cgcggttcca gatgttgcgc agcggcaaaa agtgctccat ggtcgggacg ctctggccgg 10560
   tcaggcgcgc gcaatcgttg acgctctaga ccgtgcaaaa ggagagcctg taagcgggca 10620
   ctcttccgtg gtctggtgga taaattcgca agggtatcat ggcggacgac cggggttcga 10680
   gccccgtatc cggccgtccg ccgtgatcca tgcggttacc gcccgcgtgt cgaacccagg 10740
   tgtgcgacgt cagacaacgg gggagtgctc cttttggctt ccttccaggc gcggcggctg 10800
   ctgcgctagc ttttttggcc actggccgcg cgcagcgtaa gcggttaggc tggaaagcga 10860
   aagcattaag tggctcgctc cctgtagccg gagggttatt ttccaagggt tgagtcgcgg 10920
   gacccccggt tcgagtctcg gaccggccgg actgcggcga acgggggttt gcctccccgt 10980
   catgcaagac cccgcttgca aattcctccg gaaacaggga cgagcccctt ttttgctttt 11040
   cccagatgca tccggtgctg cggcagatgc gcccccctcc tcagcagcgg caagagcaag 11100
   agcagcggca gacatgcagg gcaccctccc ctcctcctac cgcgtcagga ggggcgacat 11160
   ccgcggttga cgcggcagca gatggtgatt acgaaccccc gcggcgccgg gcccggcact 11220
   acctggactt ggaggagggc gagggcctgg cgcggctagg agcgccctct cctgagcggt 11280
   acccaagggt gcagctgaag cgtgatacgc gtgaggcgta cgtgccgcgg cagaacctgt 11340
   ttcgcgaccg cgagggagag gagcccgagg agatgcggga tcgaaagttc cacgcagggc 11400
   gcgagctgcg gcatggcctg aatcgcgagc ggttgctgcg cgaggaggac tttgagcccg 11460
   acgcgcgaac cgggattagt cccgcgcgcg cacacgtggc ggccgccgac ctggtaaccg 11520
   catacgagca gacggtgaac caggagatta actttcaaaa aagctttaac aaccacgtgc 11580
   gtacgcttgt ggcgcgcgag gaggtggcta taggactgat gcatctgtgg gactttgtaa 11640
   gcgcgctgga gcaaaaccca aatagcaagc cgctcatggc gcagctgttc cttatagtgc 11700
   agcacagcag ggacaacgag gcattcaggg atgcgctgct aaacatagta gagcccgagg 11760
   gccgctggct gctcgatttg ataaacatcc tgcagagcat agtggtgcag gagcgcagct 11820
   tgagcctggc tgacaaggtg gccgccatca actattccat gcttagcctg ggcaagtttt 11880
   acgcccgcaa gatataccat accccttacg ttcccataga caaggaggta aagatcgagg 11940
   ggttctacat gcgcatggcg ctgaaggtgc ttaccttgag cgacgacctg ggcgtttatc 12000
   gcaacgagcg catccacaag gccgtgagcg tgagccggcg gcgcgagctc agcgaccgcg 12060
   agctgatgca cagcctgcaa agggccctgg ctggcacggg cagcggcgat agagaggccg 12120
   agtcctactt tgacgcgggc gctgacctgc gctgggcccc aagccgacgc gccctggagg 12180
   cagctggggc cggacctggg ctggcggtgg cacccgcgcg cgctggcaac gtcggcggcg 12240
   tggaggaata tgacgaggac gatgagtacg agccagagga cggcgagtac taagcggtga 12300
   tgtttctgat cagatgatgc aagacgcaac ggacccggcg gtgcgggcgg cgctgcagag 12360
   ccagccgtcc ggccttaact ccacggacga ctggcgccag gtcatggacc gcatcatgtc 12420
   gctgactgcg cgcaatcctg acgcgttccg gcagcagccg caggccaacc ggctctccgc 12480
   aattctggaa gcggtggtcc cggcgcgcgc aaaccccacg cacgagaagg tgctggcgat 12540
   cgtaaacgcg ctggccgaaa acagggccat ccggcccgac gaggccggcc tggtctacga 12600
   cgcgctgctt cagcgcgtgg ctcgttacaa cagcggcaac gtgcagacca acctggaccg 12660
   gctggtgggg gatgtgcgcg aggccgtggc gcagcgtgag cgcgcgcagc agcagggcaa 12720
   cctgggctcc atggttgcac taaacgcctt cctgagtaca cagcccgcca acgtgccgcg 12780
   gggacaggag gactacacca actttgtgag cgcactgcgg ctaatggtga ctgagacacc 12840
   gcaaagtgag gtgtaccagt ctgggccaga ctattttttc cagaccagta gacaaggcct 12900
   gcagaccgta aacctgagcc aggctttcaa aaacttgcag gggctgtggg gggtgcgggc 12960
   tcccacaggc gaccgcgcga ccgtgtctag cttgctgacg cccaactcgc gcctgttgct 13020
   gctgctaata gcgcccttca cggacagtgg cagcgtgtcc cgggacacat acctaggtca 13080
   cttgctgaca ctgtaccgcg aggccatagg tcaggcgcat gtggacgagc atactttcca 13140
   ggagattaca agtgtcagcc gcgcgctggg gcaggaggac acgggcagcc tggaggcaac 13200
   cctaaactac ctgctgacca accggcggca gaagatcccc tcgttgcaca gtttaaacag 13260
   cgaggaggag cgcattttgc gctacgtgca gcagagcgtg agccttaacc tgatgcgcga 13320
   cggggtaacg cccagcgtgg cgctggacat gaccgcgcgc aacatggaac cgggcatgta 13380
   tgcctcaaac cggccgttta tcaaccgcct aatggactac ttgcatcgcg cggccgccgt 13440
   gaaccccgag tatttcacca atgccatctt gaacccgcac tggctaccgc cccctggttt 13500
   ctacaccggg ggattcgagg tgcccgaggg taacgatgga ttcctctggg acgacataga 13560
   cgacagcgtg ttttccccgc aaccgcagac cctgctagag ttgcaacagc gcgagcaggc 13620
   agaggcggcg ctgcgaaagg aaagcttccg caggccaagc agcttgtccg atctaggcgc 13680
   tgcggccccg cggtcagatg ctagtagccc atttccaagc ttgatagggt ctcttaccag 13740
   cactcgcacc acccgcccgc gcctgctggg cgaggaggag tacctaaaca actcgctgct 13800
   gcagccgcag cgcgaaaaaa acctgcctcc ggcatttccc aacaacggga tagagagcct 13860
   agtggacaag atgagtagat ggaagacgta cgcgcaggag cacagggacg tgccaggccc 13920
   gcgcccgccc acccgtcgtc aaaggcacga ccgtcagcgg ggtctggtgt gggaggacga 13980
   tgactcggca gacgacagca gcgtcctgga tttgggaggg agtggcaacc cgtttgcgca 14040
   ccttcgcccc aggctgggga gaatgtttta aaaaaaaaaa agcatgatgc aaaataaaaa 14100
   actcaccaag gccatggcac cgagcgttgg ttttcttgta ttccccttag tatgcggcgc 14160
   gcggcgatgt atgaggaagg tcctcctccc tcctacgaga gtgtggtgag cgcggcgcca 14220
   gtggcggcgg cgctgggttc tcccttcgat gctcccctgg acccgccgtt tgtgcctccg 14280
   cggtacctgc ggcctaccgg ggggagaaac agcatccgtt actctgagtt ggcaccccta 14340
   ttcgacacca cccgtgtgta cctggtggac aacaagtcaa cggatgtggc atccctgaac 14400
   taccagaacg accacagcaa ctttctgacc acggtcattc aaaacaatga ctacagcccg 14460
   ggggaggcaa gcacacagac catcaatctt gacgaccggt cgcactgggg cggcgacctg 14520
   aaaaccatcc tgcataccaa catgccaaat gtgaacgagt tcatgtttac caataagttt 14580
   aaggcgcggg tgatggtgtc gcgcttgcct actaaggaca atcaggtgga gctgaaatac 14640
   gagtgggtgg agttcacgct gcccgagggc aactactccg agaccatgac catagacctt 14700
   atgaacaacg cgatcgtgga gcactacttg aaagtgggca gacagaacgg ggttctggaa 14760
   agcgacatcg gggtaaagtt tgacacccgc aacttcagac tggggtttga ccccgtcact 14820
   ggtcttgtca tgcctggggt atatacaaac gaagccttcc atccagacat cattttgctg 14880
   ccaggatgcg gggtggactt cacccacagc cgcctgagca acttgttggg catccgcaag 14940
   cggcaaccct tccaggaggg ctttaggatc acctacgatg atctggaggg tggtaacatt 15000
   cccgcactgt tggatgtgga cgcctaccag gcgagcttga aagatgacac cgaacagggc 15060
   gggggtggcg caggcggcag caacagcagt ggcagcggcg cggaagagaa ctccaacgcg 15120
   gcagccgcgg caatgcagcc ggtggaggac atgaacgatc atgccattcg cggcgacacc 15180
   tttgccacac gggctgagga gaagcgcgct gaggccgaag cagcggccga agctgccgcc 15240
   cccgctgcgc aacccgaggt cgagaagcct cagaagaaac cggtgatcaa acccctgaca 15300
   gaggacagca agaaacgcag ttacaaccta ataagcaatg acagcacctt cacccagtac 15360
   cgcagctggt accttgcata caactacggc gaccctcaga ccggaatccg ctcatggacc 15420
   ctgctttgca ctcctgacgt aacctgcggc tcggagcagg tctactggtc gttgccagac 15480
   atgatgcaag accccgtgac cttccgctcc acgcgccaga tcagcaactt tccggtggtg 15540
   ggcgccgagc tgttgcccgt gcactccaag agcttctaca acgaccaggc cgtctactcc 15600
   caactcatcc gccagtttac ctctctgacc cacgtgttca atcgctttcc cgagaaccag 15660
   attttggcgc gcccgccagc ccccaccatc accaccgtca gtgaaaacgt tcctgctctc 15720
   acagatcacg ggacgctacc gctgcgcaac agcatcggag gagtccagcg agtgaccatt 15780
   actgacgcca gacgccgcac ctgcccctac gtttacaagg ccctgggcat agtctcgccg 15840
   cgcgtcctat cgagccgcac tttttgagca agcatgtcca tccttatatc gcccagcaat 15900
   aacacaggct ggggcctgcg cttcccaagc aagatgtttg gcggggccaa gaagcgctcc 15960
   gaccaacacc cagtgcgcgt gcgcgggcac taccgcgcgc cctggggcgc gcacaaacgc 16020
   ggccgcactg ggcgcaccac cgtcgatgac gccatcgacg cggtggtgga ggaggcgcgc 16080
   aactacacgc ccacgccgcc accagtgtcc acagtggacg cggccattca gaccgtggtg 16140
   cgcggagccc ggcgctatgc taaaatgaag agacggcgga ggcgcgtagc acgtcgccac 16200
   cgccgccgac ccggcactgc cgcccaacgc gcggcggcgg ccctgcttaa ccgcgcacgt 16260
   cgcaccggcc gacgggcggc catgcgggcc gctcgaaggc tggccgcggg tattgtcact 16320
   gtgcccccca ggtccaggcg acgagcggcc gccgcagcag ccgcggccat tagtgctatg 16380
   actcagggtc gcaggggcaa cgtgtattgg gtgcgcgact cggttagcgg cctgcgcgtg 16440
   cccgtgcgca cccgcccccc gcgcaactag attgcaagaa aaaactactt agactcgtac 16500
   tgttgtatgt atccagcggc ggcggcgcgc aacgaagcta tgtccaagcg caaaatcaaa 16560
   gaagagatgc tccaggtcat cgcgccggag atctatggcc ccccgaagaa ggaagagcag 16620
   gattacaagc cccgaaagct aaagcgggtc aaaaagaaaa agaaagatga tgatgatgaa 16680
   cttgacgacg aggtggaact gctgcacgct accgcgccca ggcgacgggt acagtggaaa 16740
   ggtcgacgcg taaaacgtgt tttgcgaccc ggcaccaccg tagtctttac gcccggtgag 16800
   cgctccaccc gcacctacaa gcgcgtgtat gatgaggtgt acggcgacga ggacctgctt 16860
   gagcaggcca acgagcgcct cggggagttt gcctacggaa agcggcataa ggacatgctg 16920
   gcgttgccgc tggacgaggg caacccaaca cctagcctaa agcccgtaac actgcagcag 16980
   gtgctgcccg cgcttgcacc gtccgaagaa aagcgcggcc taaagcgcga gtctggtgac 17040
   ttggcaccca ccgtgcagct gatggtaccc aagcgccagc gactggaaga tgtcttggaa 17100
   aaaatgaccg tggaacctgg gctggagccc gaggtccgcg tgcggccaat caagcaggtg 17160
   gcgccgggac tgggcgtgca gaccgtggac gttcagatac ccactaccag tagcaccagt 17220
   attgccaccg ccacagaggg catggagaca caaacgtccc cggttgcctc agcggtggcg 17280
   gatgccgcgg tgcaggcggt cgctgcggcc gcgtccaaga cctctacgga ggtgcaaacg 17340
   gacccgtgga tgtttcgcgt ttcagccccc cggcgcccgc gcggttcgag gaagtacggc 17400
   gccgccagcg cgctactgcc cgaatatgcc ctacatcctt ccattgcgcc tacccccggc 17460
   tatcgtggct acacctaccg ccccagaaga cgagcaacta cccgacgccg aaccaccact 17520
   ggaacccgcc gccgccgtcg ccgtcgccag cccgtgctgg ccccgatttc cgtgcgcagg 17580
   gtggctcgcg aaggaggcag gaccctggtg ctgccaacag cgcgctacca ccccagcatc 17640
   gtttaaaagc cggtctttgt ggttcttgca gatatggccc tcacctgccg cctccgtttc 17700
   ccggtgccgg gattccgagg aagaatgcac cgtaggaggg gcatggccgg ccacggcctg 17760
   acgggcggca tgcgtcgtgc gcaccaccgg cggcggcgcg cgtcgcaccg tcgcatgcgc 17820
   ggcggtatcc tgcccctcct tattccactg atcgccgcgg cgattggcgc cgtgcccgga 17880
   attgcatccg tggccttgca ggcgcagaga cactgattaa aaacaagttg catgtggaaa 17940
   aatcaaaata aaaagtctgg actctcacgc tcgcttggtc ctgtaactat tttgtagaat 18000
   ggaagacatc aactttgcgt ctctggcccc gcgacacggc tcgcgcccgt tcatgggaaa 18060
   ctggcaagat atcggcacca gcaatatgag cggtggcgcc ttcagctggg gctcgctgtg 18120
   gagcggcatt aaaaatttcg gttccaccgt taagaactat ggcagcaagg cctggaacag 18180
   cagcacaggc cagatgctga gggataagtt gaaagagcaa aatttccaac aaaaggtggt 18240
   agatggcctg gcctctggca ttagcggggt ggtggacctg gccaaccagg cagtgcaaaa 18300
   taagattaac agtaagcttg atccccgccc tcccgtagag gagcctccac cggccgtgga 18360
   gacagtgtct ccagaggggc gtggcgaaaa gcgtccgcgc cccgacaggg aagaaactct 18420
   ggtgacgcaa atagacgagc ctccctcgta cgaggaggca ctaaagcaag gcctgcccac 18480
   cacccgtccc atcgcgccca tggctaccgg agtgctgggc cagcacacac ccgtaacgct 18540
   ggacctgcct ccccccgccg acacccagca gaaacctgtg ctgccaggcc cgaccgccgt 18600
   tgttgtaacc cgtcctagcc gcgcgtccct gcgccgcgcc gccagcggtc cgcgatcgtt 18660
   gcggcccgta gccagtggca actggcaaag cacactgaac agcatcgtgg gtctgggggt 18720
   gcaatccctg aagcgccgac gatgcttctg aatagctaac gtgtcgtatg tgtgtcatgt 18780
   atgcgtccat gtcgccgcca gaggagctgc tgagccgccg cgcgcccgct ttccaagatg 18840
   gctacccctt cgatgatgcc gcagtggtct tacatgcaca tctcgggcca ggacgcctcg 18900
   gagtacctga gccccgggct ggtgcagttt gcccgcgcca ccgagacgta cttcagcctg 18960
   aataacaagt ttagaaaccc cacggtggcg cctacgcacg acgtgaccac agaccggtcc 19020
   cagcgtttga cgctgcggtt catccctgtg gaccgtgagg atactgcgta ctcgtacaag 19080
   gcgcggttca ccctagctgt gggtgataac cgtgtgctgg acatggcttc cacgtacttt 19140
   gacatccgcg gcgtgctgga caggggccct acttttaagc cctactctgg cactgcctac 19200
   aacgccctgg ctcccaaggg tgccccaaat ccttgcgaat gggatgaagc tgctactgct 19260
   cttgaaataa acctagaaga agaggacgat gacaacgaag acgaagtaga cgagcaagct 19320
   gagcagcaaa aaactcacgt atttgggcag gcgccttatt ctggtataaa tattacaaag 19380
   gagggtattc aaataggtgt cgaaggtcaa acacctaaat atgccgataa aacatttcaa 19440
   cctgaacctc aaataggaga atctcagtgg tacgaaactg aaattaatca tgcagctggg 19500
   agagtcctta aaaagactac cccaatgaaa ccatgttacg gttcatatgc aaaacccaca 19560
   aatgaaaatg gagggcaagg cattcttgta aagcaacaaa atggaaagct agaaagtcaa 19620
   gtggaaatgc aatttttctc aactactgag gcgaccgcag gcaatggtga taacttgact 19680
   cctaaagtgg tattgtacag tgaagatgta gatatagaaa ccccagacac tcatatttct 19740
   tacatgccca ctattaagga aggtaactca cgagaactaa tgggccaaca atctatgccc 19800
   aacaggccta attacattgc ttttagggac aattttattg gtctaatgta ttacaacagc 19860
   acgggtaata tgggtgttct ggcgggccaa gcatcgcagt tgaatgctgt tgtagatttg 19920
   caagacagaa acacagagct ttcataccag cttttgcttg attccattgg tgatagaacc 19980
   aggtactttt ctatgtggaa tcaggctgtt gacagctatg atccagatgt tagaattatt 20040
   gaaaatcatg gaactgaaga tgaacttcca aattactgct ttccactggg aggtgtgatt 20100
   aatacagaga ctcttaccaa ggtaaaacct aaaacaggtc aggaaaatgg atgggaaaaa 20160
   gatgctacag aattttcaga taaaaatgaa ataagagttg gaaataattt tgccatggaa 20220
   atcaatctaa atgccaacct gtggagaaat ttcctgtact ccaacatagc gctgtatttg 20280
   cccgacaagc taaagtacag tccttccaac gtaaaaattt ctgataaccc aaacacctac 20340
   gactacatga acaagcgagt ggtggctccc gggttagtgg actgctacat taaccttgga 20400
   gcacgctggt cccttgacta tatggacaac gtcaacccat ttaaccacca ccgcaatgct 20460
   ggcctgcgct accgctcaat gttgctgggc aatggtcgct atgtgccctt ccacatccag 20520
   gtgcctcaga agttctttgc cattaaaaac ctccttctcc tgccgggctc atacacctac 20580
   gagtggaact tcaggaagga tgttaacatg gttctgcaga gctccctagg aaatgaccta 20640
   agggttgacg gagccagcat taagtttgat agcatttgcc tttacgccac cttcttcccc 20700
   atggcccaca acaccgcctc cacgcttgag gccatgctta gaaacgacac caacgaccag 20760
   tcctttaacg actatctctc cgccgccaac atgctctacc ctatacccgc caacgctacc 20820
   aacgtgccca tatccatccc ctcccgcaac tgggcggctt tccgcggctg ggccttcacg 20880
   cgccttaaga ctaaggaaac cccatcactg ggctcgggct acgaccctta ttacacctac 20940
   tctggctcta taccctacct agatggaacc ttttacctca accacacctt taagaaggtg 21000
   gccattacct ttgactcttc tgtcagctgg cctggcaatg accgcctgct tacccccaac 21060
   gagtttgaaa ttaagcgctc agttgacggg gagggttaca acgttgccca gtgtaacatg 21120
   accaaagact ggttcctggt acaaatgcta gctaactaca acattggcta ccagggcttc 21180
   tatatcccag agagctacaa ggaccgcatg tactccttct ttagaaactt ccagcccatg 21240
   agccgtcagg tggtggatga tactaaatac aaggactacc aacaggtggg catcctacac 21300
   caacacaaca actctggatt tgttggctac cttgccccca ccatgcgcga aggacaggcc 21360
   taccctgcta acttccccta tccgcttata ggcaagaccg cagttgacag cattacccag 21420
   aaaaagtttc tttgcgatcg caccctttgg cgcatcccat tctccagtaa ctttatgtcc 21480
   atgggcgcac tcacagacct gggccaaaac cttctctacg ccaactccgc ccacgcgcta 21540
   gacatgactt ttgaggtgga tcccatggac gagcccaccc ttctttatgt tttgtttgaa 21600
   gtctttgacg tggtccgtgt gcaccggccg caccgcggcg tcatcgaaac cgtgtacctg 21660
   cgcacgccct tctcggccgg caacgccaca acataaagaa gcaagcaaca tcaacaacag 21720
   ctgccgccat gggctccagt gagcaggaac tgaaagccat tgtcaaagat cttggttgtg 21780
   ggccatattt tttgggcacc tatgacaagc gctttccagg ctttgtttct ccacacaagc 21840
   tcgcctgcgc catagtcaat acggccggtc gcgagactgg gggcgtacac tggatggcct 21900
   ttgcctggaa cccgcactca aaaacatgct acctctttga gccctttggc ttttctgacc 21960
   agcgactcaa gcaggtttac cagtttgagt acgagtcact cctgcgccgt agcgccattg 22020
   cttcttcccc cgaccgctgt ataacgctgg aaaagtccac ccaaagcgta caggggccca 22080
   actcggccgc ctgtggacta ttctgctgca tgtttctcca cgcctttgcc aactggcccc 22140
   aaactcccat ggatcacaac cccaccatga accttattac cggggtaccc aactccatgc 22200
   tcaacagtcc ccaggtacag cccaccctgc gtcgcaacca ggaacagctc tacagcttcc 22260
   tggagcgcca ctcgccctac ttccgcagcc acagtgcgca gattaggagc gccacttctt 22320
   tttgtcactt gaaaaacatg taaaaataat gtactagaga cactttcaat aaaggcaaat 22380
   gcttttattt gtacactctc gggtgattat ttacccccac ccttgccgtc tgcgccgttt 22440
   aaaaatcaaa ggggttctgc cgcgcatcgc tatgcgccac tggcagggac acgttgcgat 22500
   actggtgttt agtgctccac ttaaactcag gcacaaccat ccgcggcagc tcggtgaagt 22560
   tttcactcca caggctgcgc accatcacca acgcgtttag caggtcgggc gccgatatct 22620
   tgaagtcgca gttggggcct ccgccctgcg cgcgcgagtt gcgatacaca gggttgcagc 22680
   actggaacac tatcagcgcc gggtggtgca cgctggccag cacgctcttg tcggagatca 22740
   gatccgcgtc caggtcctcc gcgttgctca gggcgaacgg agtcaacttt ggtagctgcc 22800
   ttcccaaaaa gggcgcgtgc ccaggctttg agttgcactc gcaccgtagt ggcatcaaaa 22860
   ggtgaccgtg cccggtctgg gcgttaggat acagcgcctg cataaaagcc ttgatctgct 22920
   taaaagccac ctgagccttt gcgccttcag agaagaacat gccgcaagac ttgccggaaa 22980
   actgattggc cggacaggcc gcgtcgtgca cgcagcacct tgcgtcggtg ttggagatct 23040
   gcaccacatt tcggccccac cggttcttca cgatcttggc cttgctagac tgctccttca 23100
   gcgcgcgctg cccgttttcg ctcgtcacat ccatttcaat cacgtgctcc ttatttatca 23160
   taatgcttcc gtgtagacac ttaagctcgc cttcgatctc agcgcagcgg tgcagccaca 23220
   acgcgcagcc cgtgggctcg tgatgcttgt aggtcacctc tgcaaacgac tgcaggtacg 23280
   cctgcaggaa tcgccccatc atcgtcacaa aggtcttgtt gctggtgaag gtcagctgca 23340
   acccgcggtg ctcctcgttc agccaggtct tgcatacggc cgccagagct tccacttggt 23400
   caggcagtag tttgaagttc gcctttagat cgttatccac gtggtacttg tccatcagcg 23460
   cgcgcgcagc ctccatgccc ttctcccacg cagacacgat cggcacactc agcgggttca 23520
   tcaccgtaat ttcactttcc gcttcgctgg gctcttcctc ttcctcttgc gtccgcatac 23580
   cacgcgccac tgggtcgtct tcattcagcc gccgcactgt gcgcttacct cctttgccat 23640
   gcttgattag caccggtggg ttgctgaaac ccaccatttg tagcgccaca tcttctcttt 23700
   cttcctcgct gtccacgatt acctctggtg atggcgggcg ctcgggcttg ggagaagggc 23760
   gcttcttttt cttcttgggc gcaatggcca aatccgccgc cgaggtcgat ggccgcgggc 23820
   tgggtgtgcg cggcaccagc gcgtcttgtg atgagtcttc ctcgtcctcg gactcgatac 23880
   gccgcctcat ccgctttttt gggggcgccc ggggaggcgg cggcgacggg gacggggacg 23940
   acacgtcctc catggttggg ggacgtcgcg ccgcaccgcg tccgcgctcg gggtggtttt 24000
   cgcgctgctc ctcttcccga ctggccattt ccttctccta taggcagaaa aagatcatgg 24060
   agtcagtcga gaagaaggac agcctaaccg ccccctctga gttcgccacc accgcctcca 24120
   ccgatgccgc caacgcgcct accaccttcc ccgtcgaggc acccccgctt gaggaggagg 24180
   aagtgattat cgagcaggac ccaggttttg taagcgaaga cgacgaggac cgctcagtac 24240
   caacagagga taaaaagcaa gaccaggaca acgcagaggc aaacgaggaa caagtcgggc 24300
   ggggggacga aaggcatggc gactacctag atgtgggaga cgacgtgctg ttgaagcatc 24360
   tgcagcgcca gtgcgccatt atctgcgacg cgttgcaaga gcgcagcgat gtgcccctcg 24420
   ccatagcgga tgtcagcctt gcctacgaac gccacctatt ctcaccgcgc gtacccccca 24480
   aacgccaaga aaacggcaca tgcgagccca acccgcgcct caacttctac cccgtatttg 24540
   ccgtgccaga ggtgcttgcc acctatcaca tctttttcca aaactgcaag atacccctat 24600
   cctgccgtgc caaccgcagc cgagcggaca agcagctggc cttgcggcag ggcgctgtca 24660
   tacctgatat cgcctcgctc aacgaagtgc caaaaatctt tgagggtctt ggacgcgacg 24720
   agaagcgcgc ggcaaacgct ctgcaacagg aaaacagcga aaatgaaagt cactctggag 24780
   tgttggtgga actcgagggt gacaacgcgc gcctagccgt actaaaacgc agcatcgagg 24840
   tcacccactt tgcctacccg gcacttaacc taccccccaa ggtcatgagc acagtcatga 24900
   gtgagctgat cgtgcgccgt gcgcagcccc tggagaggga tgcaaatttg caagaacaaa 24960
   cagaggaggg cctacccgca gttggcgacg agcagctagc gcgctggctt caaacgcgcg 25020
   agcctgccga cttggaggag cgacgcaaac taatgatggc cgcagtgctc gttaccgtgg 25080
   agcttgagtg catgcagcgg ttctttgctg acccggagat gcagcgcaag ctagaggaaa 25140
   cattgcacta cacctttcga cagggctacg tacgccaggc ctgcaagatc tccaacgtgg 25200
   agctctgcaa cctggtctcc taccttggaa ttttgcacga aaaccgcctt gggcaaaacg 25260
   tgcttcattc cacgctcaag ggcgaggcgc gccgcgacta cgtccgcgac tgcgtttact 25320
   tatttctatg ctacacctgg cagacggcca tgggcgtttg gcagcagtgc ttggaggagt 25380
   gcaacctcaa ggagctgcag aaactgctaa agcaaaactt gaaggaccta tggacggcct 25440
   tcaacgagcg ctccgtggcc gcgcacctgg cggacatcat tttccccgaa cgcctgctta 25500
   aaaccctgca acagggtctg ccagacttca ccagtcaaag catgttgcag aactttagga 25560
   actttatcct agagcgctca ggaatcttgc ccgccacctg ctgtgcactt cctagcgact 25620
   ttgtgcccat taagtaccgc gaatgccctc cgccgctttg gggccactgc taccttctgc 25680
   agctagccaa ctaccttgcc taccactctg acataatgga agacgtgagc ggtgacggtc 25740
   tactggagtg tcactgtcgc tgcaacctat gcaccccgca ccgctccctg gtttgcaatt 25800
   cgcagctgct taacgaaagt caaattatcg gtacctttga gctgcagggt ccctcgcctg 25860
   acgaaaagtc cgcggctccg gggttgaaac tcactccggg gctgtggacg tcggcttacc 25920
   ttcgcaaatt tgtacctgag gactaccacg cccacgagat taggttctac gaagaccaat 25980
   cccgcccgcc aaatgcggag cttaccgcct gcgtcattac ccagggccac attcttggcc 26040
   aattgcaagc catcaacaaa gcccgccaag agtttctgct acgaaaggga cggggggttt 26100
   acttggaccc ccagtccggc gaggagctca acccaatccc cccgccgccg cagccctatc 26160
   agcagcagcc gcgggccctt gcttcccagg atggcaccca aaaagaagct gcagctgccg 26220
   ccgccaccca cggacgagga ggaatactgg gacagtcagg cagaggaggt tttggacgag 26280
   gaggaggagg acatgatgga agactgggag agcctagacg aggaagcttc cgaggtcgaa 26340
   gaggtgtcag acgaaacacc gtcaccctcg gtcgcattcc cctcgccggc gccccagaaa 26400
   tcggcaaccg gttccagcat ggctacaacc tccgctcctc aggcgccgcc ggcactgccc 26460
   gttcgccgac ccaaccgtag atgggacacc actggaacca gggccggtaa gtccaagcag 26520
   ccgccgccgt tagcccaaga gcaacaacag cgccaaggct accgctcatg gcgcgggcac 26580
   aagaacgcca tagttgcttg cttgcaagac tgtgggggca acatctcctt cgcccgccgc 26640
   tttcttctct accatcacgg cgtggccttc ccccgtaaca tcctgcatta ctaccgtcat 26700
   ctctacagcc catactgcac cggcggcagc ggcagcggca gcaacagcag cggccacaca 26760
   gaagcaaagg cgaccggata gcaagactct gacaaagccc aagaaatcca cagcggcggc 26820
   agcagcagga ggaggagcgc tgcgtctggc gcccaacgaa cccgtatcga cccgcgagct 26880
   tagaaacagg atttttccca ctctgtatgc tatatttcaa cagagcaggg gccaagaaca 26940
   agagctgaaa ataaaaaaca ggtctctgcg atccctcacc cgcagctgcc tgtatcacaa 27000
   aagcgaagat cagcttcggc gcacgctgga agacgcggag gctctcttca gtaaatactg 27060
   cgcgctgact cttaaggact agtttcgcgc cctttctcaa atttaagcgc gaaaactacg 27120
   tcatctccag cggccacacc cggcgccagc acctgtcgtc agcgccatta tgagcaagga 27180
   aattcccacg ccctacatgt ggagttacca gccacaaatg ggacttgcgg ctggagctgc 27240
   ccaagactac tcaacccgaa taaactacat gagcgcggga ccccacatga tatcccgggt 27300
   caacggaatc cgcgcccacc gaaaccgaat tctcttggaa caggcggcta ttaccaccac 27360
   acctcgtaat aaccttaatc cccgtagttg gcccgctgcc ctggtgtacc aggaaagtcc 27420
   cgctcccacc actgtggtac ttcccagaga cgcccaggcc gaagttcaga tgactaactc 27480
   aggggcgcag cttgcgggcg gctttcgtca cagggtgcgg tcgcccgggc agggtataac 27540
   tcacctgaca atcagagggc gaggtattca gctcaacgac gagtcggtga gctcctcgct 27600
   tggtctccgt ccggacggga catttcagat cggcggcgcc ggccgctctt cattcacgcc 27660
   tcgtcaggca atcctaactc tgcagacctc gtcctctgag ccgcgctctg gaggcattgg 27720
   aactctgcaa tttattgagg agtttgtgcc atcggtctac tttaacccct tctcgggacc 27780
   tcccggccac tatccggatc aatttattcc taactttgac gcggtaaagg actcggcgga 27840
   tggctacgac tgaatgttaa gtggagaggc agagcaactg cgcctgaaac acctggtcca 27900
   ctgtcgccgc cacaagtgct ttgcccgcga ctccggtgag ttttgctact ttgaattgcc 27960
   cgaggatcat atcgagggcc cggcgcacgg cgtccggctt accgcccagg gagagcttgc 28020
   ccgtagcctg attcgggagt ttacccagcg ccccctgcta gttgagcggg acaggggacc 28080
   ctgtgttctc actgtgattt gcaactgtcc taaccctgga ttacatcaag atctttgttg 28140
   ccatctctgt gctgagtata ataaatacag aaattaaaat atactggggc tcctatcgcc 28200
   atcctgtaaa cgccaccgtc ttcacccgcc caagcaaacc aaggcgaacc ttacctggta 28260
   cttttaacat ctctccctct gtgatttaca acagtttcaa cccagacgga gtgagtctac 28320
   gagagaacct ctccgagctc agctactcca tcagaaaaaa caccaccctc cttacctgcc 28380
   gggaacgtac gatgtggctg cagagcctgc tgctcttggg cactgtggcc tgcagcatct 28440
   ctgcacccgc ccgctcgccc agccccagca cgcagccctg ggagcatgtg aatgccatcc 28500
   aggaggcccg gcgtctcctg aacctgagta gagacactgc tgctgagatg aatgaaacag 28560
   tagaagtcat ctcagaaatg tttgacctcc aggagccgac ctgcctacag acccgcctgg 28620
   agctgtacaa gcagggcctg cggggcagcc tcaccaagct caagggcccc ttgaccatga 28680
   tggccagcca ctacaagcag cactgccctc caaccccgga aacttcctgt gcaacccaga 28740
   ctatcacctt tgaaagtttc aaagagaacc tgaaggactt tctgcttgtc atcccctttg 28800
   actgctggga gccagtccag gagtgacaat tgactctatg tgggatatgc tccagcgcta 28860
   caaccttgaa gtcaggcttc ctggatgtca gcatctgact ttggccagca cctgtcccgc 28920
   ggatttgttc cagtccaact acagcgaccc accctaacag agatgaccaa cacaaccaac 28980
   gcggccgccg ctaccggact tacatctacc acaaatacac cccaagtttc tgcctttgtc 29040
   aataactggg ataacttggg catgtggtgg ttctccatag cgcttatgtt tgtatgcctt 29100
   attattatgt ggctcatctg ctgcctaaag cgcaaacgcg cccgaccacc catctatagt 29160
   cccatcattg tgctacaccc aaacaatgat ggaatccata gattggacgg actgaaacac 29220
   atgttctttt ctcttacagt atgattaaat gagacatgat tcctcgagtt tttatattac 29280
   tgacccttgt tgcgcttttt tgtgcgtgct ccacattggc tgcggtttct cacatcgaag 29340
   tagactgcat tccagccttc acagtctatt tgctttacgg atttgtcacc ctcacgctca 29400
   tctgcagcct catcactgtg gtcatcgcct ttatccagtg cattgactgg gtctgtgtgc 29460
   gctttgcata tctcagacac catccccagt acagggacag gactatagct gagcttctta 29520
   gaattcttta attatgaaat ttactgtgac ttttctgctg attatttgca ccctatctgc 29580
   gttttgttcc ccgacctcca agcctcaaag acatatatca tgcagattca ctcgtatatg 29640
   gaatattcca agttgctaca atgaaaaaag cgatctttcc gaagcctggt tatatgcaat 29700
   catctctgtt atggtgttct gcagtaccat cttagcccta gctatatatc cctaccttga 29760
   cattggctgg aaacgaatag atgccatgaa ccacccaact ttccccgcgc ccgctatgct 29820
   tccactgcaa caagttgttg ccggcggctt tgtcccagcc aatcagcctc gccccacttc 29880
   tcccaccccc actgaaatca gctactttaa tctaacagga ggagatgact gacaccctag 29940
   atctagaaat ggacggaatt attacagagc agcgcctgct agaaagacgc agggcagcgg 30000
   ccgagcaaca gcgcatgaat caagagctcc aagacatggt taacttgcac cagtgcaaaa 30060
   ggggtatctt ttgtctggta aagcaggcca aagtcaccta cgacagtaat accaccggac 30120
   accgccttag ctacaagttg ccaaccaagc gtcagaaatt ggtggtcatg gtgggagaaa 30180
   agcccattac cataactcag cactcggtag aaaccgaagg ctgcattcac tcaccttgtc 30240
   aaggacctga ggatctctgc acccttatta agaccctgtg cggtctcaaa gatcttattc 30300
   cctttaacta ataaaaaaaa ataataaagc atcacttact taaaatcagt tagcaaattt 30360
   ctgtccagtt tattcagcag cacctccttg ccctcctccc agctctggta ttgcagcttc 30420
   ctcctggctg caaactttct ccacaatcta aatggaatgt cagtttcctc ctgttcctgt 30480
   ccatccgcac ccactatctt catgttgttg cagatgaagc gcgcaagacc gtctgaagat 30540
   accttcaacc ccgtgtatcc atatgacacg gaaaccggtc ctccaactgt gccttttctt 30600
   actcctccct ttgtatcccc caatgggttt caagagagtc cccctggggt actctctttg 30660
   cgcctatccg aacctctagt tacctccaat ggcatgcttg cgctcaaaat gggcaacggc 30720
   ctctctctgg acgaggccgg caaccttacc tcccaaaatg taaccactgt gagcccacct 30780
   ctcaaaaaaa ccaagtcaaa cataaacctg gaaatatctg cacccctcac agttacctca 30840
   gaagccctaa ctgtggctgc cgccgcacct ctaatggtcg cgggcaacac actcaccatg 30900
   caatcacagg ccccgctaac cgtgcacgac tccaaactta gcattgccac ccaaggaccc 30960
   ctcacagtgt cagaaggaaa gctagccctg caaacatcag gccccctcac caccaccgat 31020
   agcagtaccc ttactatcac tgcctcaccc cctctaacta ctgccactgg tagcttgggc 31080
   attgacttga aagagcccat ttatacacaa aatggaaaac taggactaaa gtacggggct 31140
   cctttgcatg taacagacga cctaaacact ttgaccgtag caactggtcc aggtgtgact 31200
   attaataata cttccttgca aactaaagtt actggagcct tgggttttga ttcacaaggc 31260
   aatatgcaac ttaatgtagc aggaggacta aggattgatt ctcaaaacag acgccttata 31320
   cttgatgtta gttatccgtt tgatgctcaa aaccaactaa atctaagact aggacagggc 31380
   cctcttttta taaactcagc ccacaacttg gatattaact acaacaaagg cctttacttg 31440
   tttacagctt caaacaattc caaaaagctt gaggttaacc taagcactgc caaggggttg 31500
   atgtttgacg ctacagccat agccattaat gcaggagatg ggcttgaatt tggttcacct 31560
   aatgcaccaa acacaaatcc cctcaaaaca aaaattggcc atggcctaga atttgattca 31620
   aacaaggcta tggttcctaa actaggaact ggccttagtt ttgacagcac aggtgccatt 31680
   acagtaggaa acaaaaataa tgataagcta actttgtgga ccacaccagc tccatctcct 31740
   aactgtagac taaatgcaga gaaagatgct aaactcactt tggtcttaac aaaatgtggc 31800
   agtcaaatac ttgctacagt ttcagttttg gctgttaaag gcagtttggc tccaatatct 31860
   ggaacagttc aaagtgctca tcttattata agatttgacg aaaatggagt gctactaaac 31920
   aattccttcc tggacccaga atattggaac tttagaaatg gagatcttac tgaaggcaca 31980
   gcctatacaa acgctgttgg atttatgcct aacctatcag cttatccaaa atctcacggt 32040
   aaaactgcca aaagtaacat tgtcagtcaa gtttacttaa acggagacaa aactaaacct 32100
   gtaacactaa cgatcacact aaacggtaca caggaaacag gagacacaac ttgtgactgc 32160
   cgcggagact gtttctgccc atctgcatac tctatgtcat tttcatggga ctggtctggc 32220
   cacaactaca ttaatgaaat atttgccaca tcctcttaca ctttttcata cattgcccaa 32280
   gaataaagaa tcgtttgtgt tatgtttcaa cgtgtttatt tttcaattgc agaaaatttc 32340
   aagtcatttt tcattcagta gtatagcccc accaccacat agcttataca gatcaccgta 32400
   ccttaatcaa actcacagaa ccctagtatt caacctgcca cctccctccc aacacacaga 32460
   gtacacagtc ctttctcccc ggctggcctt aaaaagcatc atatcatggg taacagacat 32520
   attcttaggt gttatattcc acacggtttc ctgtcgagcc aaacgctcat cagtgatatt 32580
   aataaactcc ccgggcagct cacttaagtt catgtcgctg tccagctgct gagccacagg 32640
   ctgctgtcca acttgcggtt gcttaacggg cggcgaagga gaagtccacg cctacatggg 32700
   ggtagagtca taatcgtgca tcaggatagg gcggtggtgc tgcagcagcg cgcgaataaa 32760
   ctgctgccgc cgccgctccg tcctgcagga atacaacatg gcagtggtct cctcagcgat 32820
   gattcgcacc gcccgcagca taaggcgcct tgtcctccgg gcacagcagc gcaccctgat 32880
   ctcacttaaa tcagcacagt aactgcagca cagcaccaca atattgttca aaatcccaca 32940
   gtgcaaggcg ctgtatccaa agctcatggc ggggaccaca gaacccacgt ggccatcata 33000
   ccacaagcgc aggtagatta agtggcgacc cctcataaac acgctggaca taaacattac 33060
   ctcttttggc atgttgtaat tcaccacctc ccggtaccat ataaacctct gattaaacat 33120
   ggcgccatcc accaccatcc taaaccagct ggccaaaacc tgcccgccgg ctatacactg 33180
   cagggaaccg ggactggaac aatgacagtg gagagcccag gactcgtaac catggatcat 33240
   catgctcgtc atgatatcaa tgttggcaca acacaggcac acgtgcatac acttcctcag 33300
   gattacaagc tcctcccgcg ttagaaccat atcccaggga acaacccatt cctgaatcag 33360
   cgtaaatccc acactgcagg gaagacctcg cacgtaactc acgttgtgca ttgtcaaagt 33420
   gttacattcg ggcagcagcg gatgatcctc cagtatggta gcgcgggttt ctgtctcaaa 33480
   aggaggtaga cgatccctac tgtacggagt gcgccgagac aaccgagatc gtgttggtcg 33540
   tagtgtcatg ccaaatggaa cgccggacgt agtcatattt cctgaagcaa aaccaggtgc 33600
   gggcgtgaca aacagatctg cgtctccggt ctcgccgctt agatcgctct gtgtagtagt 33660
   tgtagtatat ccactctctc aaagcatcca ggcgccccct ggcttcgggt tctatgtaaa 33720
   ctccttcatg cgccgctgcc ctgataacat ccaccaccgc agaataagcc acacccagcc 33780
   aacctacaca ttcgttctgc gagtcacaca cgggaggagc gggaagagct ggaagaacca 33840
   tgtttttttt tttattccaa aagattatcc aaaacctcaa aatgaagatc tattaagtga 33900
   acgcgctccc ctccggtggc gtggtcaaac tctacagcca aagaacagat aatggcattt 33960
   gtaagatgtt gcacaatggc ttccaaaagg caaacggccc tcacgtccaa gtggacgtaa 34020
   aggctaaacc cttcagggtg aatctcctct ataaacattc cagcaccttc aaccatgccc 34080
   aaataattct catctcgcca ccttctcaat atatctctaa gcaaatcccg aatattaagt 34140
   ccggccattg taaaaatctg ctccagagcg ccctccacct tcagcctcaa gcagcgaatc 34200
   atgattgcaa aaattcaggt tcctcacaga cctgtataag attcaaaagc ggaacattaa 34260
   caaaaatacc gcgatcccgt aggtcccttc gcagggccag ctgaacataa tcgtgcaggt 34320
   ctgcacggac cagcgcggcc acttccccgc caggaaccat gacaaaagaa cccacactga 34380
   ttatgacacg catactcgga gctatgctaa ccagcgtagc cccgatgtaa gcttgttgca 34440
   tgggcggcga tataaaatgc aaggtgctgc tcaaaaaatc aggcaaagcc tcgcgcaaaa 34500
   aagaaagcac atcgtagtca tgctcatgca gataaaggca ggtaagctcc ggaaccacca 34560
   cagaaaaaga caccattttt ctctcaaaca tgtctgcggg tttctgcata aacacaaaat 34620
   aaaataacaa aaaaacattt aaacattaga agcctgtctt acaacaggaa aaacaaccct 34680
   tataagcata agacggacta cggccatgcc ggcgtgaccg taaaaaaact ggtcaccgtg 34740
   attaaaaagc accaccgaca gctcctcggt catgtccgga gtcataatgt aagactcggt 34800
   aaacacatca ggttgattca catcggtcag tgctaaaaag cgaccgaaat agcccggggg 34860
   aatacatacc cgcaggcgta gagacaacat tacagccccc ataggaggta taacaaaatt 34920
   aataggagag aaaaacacat aaacacctga aaaaccctcc tgcctaggca aaatagcacc 34980
   ctcccgctcc agaacaacat acagcgcttc cacagcggca gccataacag tcagccttac 35040
   cagtaaaaaa gaaaacctat taaaaaaaca ccactcgaca cggcaccagc tcaatcagtc 35100
   acagtgtaaa aaagggccaa gtgcagagcg agtatatata ggactaaaaa atgacgtaac 35160
   ggttaaagtc cacaaaaaac acccagaaaa ccgcacgcga acctacgccc agaaacgaaa 35220
   gccaaaaaac ccacaacttc ctcaaatcgt cacttccgtt ttcccacgtt acgtcacttc 35280
   ccattttaag aaaactacaa ttcccaacac atacaagtta ctccgcccta aaacctacgt 35340
   cacccgcccc gttcccacgc cccgcgccac gtcacaaact ccaccccctc attatcatat 35400
   tggcttcaat ccaaaataag gtatattatt gatgatgtta attaagaatt cggatctgcg 35460
   acgcgaggct ggatggcctt ccccattatg attcttctcg cttccggcgg catcgggatg 35520
   cccgcgttgc aggccatgct gtccaggcag gtagatgacg accatcaggg acagcttcac 35580
   ggccagcaaa aggccaggaa ccgtaaaaag gccgcgttgc tggcgttttt ccataggctc 35640
   cgcccccctg acgagcatca caaaaatcga cgctcaagtc agaggtggcg aaacccgaca 35700
   ggactataaa gataccaggc gtttccccct ggaagctccc tcgtgcgctc tcctgttccg 35760
   accctgccgc ttaccggata cctgtccgcc tttctccctt cgggaagcgt ggcgctttct 35820
   caatgctcac gctgtaggta tctcagttcg gtgtaggtcg ttcgctccaa gctgggctgt 35880
   gtgcacgaac cccccgttca gcccgaccgc tgcgccttat ccggtaacta tcgtcttgag 35940
   tccaacccgg taagacacga cttatcgcca ctggcagcag ccactggtaa caggattagc 36000
   agagcgaggt atgtaggcgg tgctacagag ttcttgaagt ggtggcctaa ctacggctac 36060
   actagaagga cagtatttgg tatctgcgct ctgctgaagc cagttacctt cggaaaaaga 36120
   gttggtagct cttgatccgg caaacaaacc accgctggta gcggtggttt ttttgtttgc 36180
   aagcagcaga ttacgcgcag aaaaaaagga tctcaagaag atcctttgat cttttctacg 36240
   gggtctgacg ctcagtggaa cgaaaactca cgttaaggga ttttggtcat gagattatca 36300
   aaaaggatct tcacctagat ccttttaaat caatctaaag tatatatgag taaacttggt 36360
   ctgacagtta ccaatgctta atcagtgagg cacctatctc agcgatctgt ctatttcgtt 36420
   catccatagt tgcctgactc cccgtcgtgt agataactac gatacgggag ggcttaccat 36480
   ctggccccag tgctgcaatg ataccgcgag acccacgctc accggctcca gatttatcag 36540
   caataaacca gccagccgga agggccgagc gcagaagtgg tcctgcaact ttatccgcct 36600
   ccatccagtc tattaattgt tgccgggaag ctagagtaag tagttcgcca gttaatagtt 36660
   tgcgcaacgt tgttgccatt gntgcaggca tcgtggtgtc acgctcgtcg tttggtatgg 36720
   cttcattcag ctccggttcc caacgatcaa ggcgagttac atgatccccc atgttgtgca 36780
   aaaaagcggt tagctccttc ggtcctccga tcgttgtcag aagtaagttg gccgcagtgt 36840
   tatcactcat ggttatggca gcactgcata attctcttac tgtcatgcca tccgtaagat 36900
   gcttttctgt gactggtgag tactcaacca agtcattctg agaatagtgt atgcggcgac 36960
   cgagttgctc ttgcccggcg tcaacacggg ataataccgc gccacatagc agaactttaa 37020
   aagtgctcat cattggaaaa cgttcttcgg ggcgaaaact ctcaaggatc ttaccgctgt 37080
   tgagatccag ttcgatgtaa cccactcgtg cacccaactg atcttcagca tcttttactt 37140
   tcaccagcgt ttctgggtga gcaaaaacag gaaggcaaaa tgccgcaaaa aagggaataa 37200
   gggcgacacg gaaatgttga atactcatac tcttcctttt tcaatattat tgaagcattt 37260
   atcagggtta ttgtctcatg agcggataca tatttgaatg tatttagaaa aataaacaaa    37320
   taggggttcc gcgcacattt ccccgaaaag tgccacctga cgtctaagaa accattatta    37380
   tcatgacatt aacctataaa aataggcgta tcacgaggcc ctttcgtctt caa           37433
<210> 10
   <211> 38366
   <212> DNA
   <213> Artificial Sequence
<220>
   <223> Ad5-pK7-D24-GMCSF
<220>
   <221> misc_feature
   <222> (4)..(6)
   <223> n is a, c, g, or t
<220>
   <221> misc_feature
   <222> (35479)..(35481)
   <223> n is a, c, g, or t
<220>
   <221> misc_feature
   <222> (36782)..(36787)
   <223> n is a, c, g, or t
<220>
   <221> misc_feature
   <222> (38325)..(38361)
   <223> n is a, c, g, or t
<400> 10
   taannntccc ttccagctct ctgccccttt tggattgaag ccaatatgat aatgaggggg        60
   tggagtttgt gacgtggcgc gggcgtggga acggggcggg tgacgtagta gtgtggcgga       120
   agtgtgatgt tgcaagtgtg gcggaacaca tgtaagcgac ggatgtggca aaagtgacgt       180
   ttttggtgtg cgccggtgta cacaggaagt gacaattttc gcgcggtttt aggcggatgt       240
   tgtagtaaat ttgggcgtaa ccgagtaaga tttggccatt ttcgcgggaa aactgaataa       300
   gaggaagtga aatctgaata attttgtgtt actcatagcg cgtaatattt gtctagggcc       360
   gcggggactt tgaccgttta cgtggagact cgcccaggtg tttttctcag gtgttttccg       420
   cgttccgggt caaagttggc gttttattat tatagtcagc tgacgtgtag tgtatttata       480
   cccggtgagt tcctcaagag gccactcttg agtgccagcg agtagagttt tctcctccga       540
   gccgctccga caccgggact gaaaatgaga catattatct gccacggagg tgttattacc       600
   gaagaaatgg ccgccagtct tttggaccag ctgatcgaag aggtactggc tgataatctt       660
   ccacctccta gccattttga accacctacc cttcacgaac tgtatgattt agacgtgacg       720
   gcccccgaag atcccaacga ggaggcggtt tcgcagattt ttcccgactc tgtaatgttg       780
   gcggtgcagg aagggattga cttactcact tttccgccgg cgcccggttc tccggagccg 840
   cctcaccttt cccggcagcc cgagcagccg gagcagagag ccttgggtcc ggtttctatg 900
   ccaaaccttg taccggaggt gatcgatcca cccagtgacg acgaggatga agagggtgag 960
   gagtttgtgt tagattatgt ggagcacccc gggcacggtt gcaggtcttg tcattatcac 1020
   cggaggaata cgggggaccc agatattatg tgttcgcttt gctatatgag gacctgtggc 1080
   atgtttgtct acagtaagtg aaaattatgg gcagtgggtg atagagtggt gggtttggtg 1140
   tggtaatttt ttttttaatt tttacagttt tgtggtttaa agaattttgt attgtgattt 1200
   ttttaaaagg tcctgtgtct gaacctgagc ctgagcccga gccagaaccg gagcctgcaa 1260
   gacctacccg ccgtcctaaa atggcgcctg ctatcctgag acgcccgaca tcacctgtgt 1320
   ctagagaatg caatagtagt acggatagct gtgactccgg tccttctaac acacctcctg 1380
   agatacaccc ggtggtcccg ctgtgcccca ttaaaccagt tgccgtgaga gttggtgggc 1440
   gtcgccaggc tgtggaatgt atcgaggact tgcttaacga gcctgggcaa cctttggact 1500
   tgagctgtaa acgccccagg ccataaggtg taaacctgtg attgcgtgtg tggttaacgc 1560
   ctttgtttgc tgaatgagtt gatgtaagtt taataaaggg tgagataatg tttaacttgc 1620
   atggcgtgtt aaatggggcg gggcttaaag ggtatataat gcgccgtggg ctaatcttgg 1680
   ttacatctga cctcatggag gcttgggagt gtttggaaga tttttctgct gtgcgtaact 1740
   tgctggaaca gagctctaac agtacctctt ggttttggag gtttctgtgg ggctcatccc 1800
   aggcaaagtt agtctgcaga attaaggagg attacaagtg ggaatttgaa gagcttttga 1860
   aatcctgtgg tgagctgttt gattctttga atctgggtca ccaggcgctt ttccaagaga 1920
   aggtcatcaa gactttggat ttttccacac cggggcgcgc tgcggctgct gttgcttttt 1980
   tgagttttat aaaggataaa tggagcgaag aaacccatct gagcgggggg tacctgctgg 2040
   attttctggc catgcatctg tggagagcgg ttgtgagaca caagaatcgc ctgctactgt 2100
   tgtcttccgt ccgcccggcg ataataccga cggaggagca gcagcagcag caggaggaag 2160
   ccaggcggcg gcggcaggag cagagcccat ggaacccgag agccggcctg gaccctcggg 2220
   aatgaatgtt gtacaggtgg ctgaactgta tccagaactg agacgcattt tgacaattac 2280
   agaggatggg caggggctaa agggggtaaa gagggagcgg ggggcttgtg aggctacaga 2340
   ggaggctagg aatctagctt ttagcttaat gaccagacac cgtcctgagt gtattacttt 2400
   tcaacagatc aaggataatt gcgctaatga gcttgatctg ctggcgcaga agtattccat 2460
   agagcagctg accacttact ggctgcagcc aggggatgat tttgaggagg ctattagggt 2520
   atatgcaaag gtggcactta ggccagattg caagtacaag atcagcaaac ttgtaaatat 2580
   caggaattgt tgctacattt ctgggaacgg ggccgaggtg gagatagata cggaggatag 2640
   ggtggccttt agatgtagca tgataaatat gtggccgggg gtgcttggca tggacggggt 2700
   ggttattatg aatgtaaggt ttactggccc caattttagc ggtacggttt tcctggccaa 2760
   taccaacctt atcctacacg gtgtaagctt ctatgggttt aacaatacct gtgtggaagc 2820
   ctggaccgat gtaagggttc ggggctgtgc cttttactgc tgctggaagg gggtggtgtg 2880
   tcgccccaaa agcagggctt caattaagaa atgcctcttt gaaaggtgta ccttgggtat 2940
   cctgtctgag ggtaactcca gggtgcgcca caatgtggcc tccgactgtg gttgcttcat 3000
   gctagtgaaa agcgtggctg tgattaagca taacatggta tgtggcaact gcgaggacag 3060
   ggcctctcag atgctgacct gctcggacgg caactgtcac ctgctgaaga ccattcacgt 3120
   agccagccac tctcgcaagg cctggccagt gtttgagcat aacatactga cccgctgttc 3180
   cttgcatttg ggtaacagga ggggggtgtt cctaccttac caatgcaatt tgagtcacac 3240
   taagatattg cttgagcccg agagcatgtc caaggtgaac ctgaacgggg tgtttgacat 3300
   gaccatgaag atctggaagg tgctgaggta cgatgagacc cgcaccaggt gcagaccctg 3360
   cgagtgtggc ggtaaacata ttaggaacca gcctgtgatg ctggatgtga ccgaggagct 3420
   gaggcccgat cacttggtgc tggcctgcac ccgcgctgag tttggctcta gcgatgaaga 3480
   tacagattga ggtactgaaa tgtgtgggcg tggcttaagg gtgggaaaga atatataagg 3540
   tgggggtctt atgtagtttt gtatctgttt tgcagcagcc gccgccgcca tgagcaccaa 3600
   ctcgtttgat ggaagcattg tgagctcata tttgacaacg cgcatgcccc catgggccgg 3660
   ggtgcgtcag aatgtgatgg gctccagcat tgatggtcgc cccgtcctgc ccgcaaactc 3720
   tactaccttg acctacgaga ccgtgtctgg aacgccgttg gagactgcag cctccgccgc 3780
   cgcttcagcc gctgcagcca ccgcccgcgg gattgtgact gactttgctt tcctgagccc 3840
   gcttgcaagc agtgcagctt cccgttcatc cgcccgcgat gacaagttga cggctctttt 3900
   ggcacaattg gattctttga cccgggaact taatgtcgtt tctcagcagc tgttggatct 3960
   gcgccagcag gtttctgccc tgaaggcttc ctcccctccc aatgcggttt aaaacataaa 4020
   taaaaaacca gactctgttt ggatttggat caagcaagtg tcttgctgtc tttatttagg 4080
   ggttttgcgc gcgcggtagg cccgggacca gcggtctcgg tcgttgaggg tcctgtgtat 4140
   tttttccagg acgtggtaaa ggtgactctg gatgttcaga tacatgggca taagcccgtc 4200
   tctggggtgg aggtagcacc actgcagagc ttcatgctgc ggggtggtgt tgtagatgat 4260
   ccagtcgtag caggagcgct gggcgtggtg cctaaaaatg tctttcagta gcaagctgat 4320
   tgccaggggc aggcccttgg tgtaagtgtt tacaaagcgg ttaagctggg atgggtgcat 4380
   acgtggggat atgagatgca tcttggactg tatttttagg ttggctatgt tcccagccat 4440
   atccctccgg ggattcatgt tgtgcagaac caccagcaca gtgtatccgg tgcacttggg 4500
   aaatttgtca tgtagcttag aaggaaatgc gtggaagaac ttggagacgc ccttgtgacc 4560
   tccaagattt tccatgcatt cgtccataat gatggcaatg ggcccacggg cggcggcctg 4620
   ggcgaagata tttctgggat cactaacgtc atagttgtgt tccaggatga gatcgtcata 4680
   ggccattttt acaaagcgcg ggcggagggt gccagactgc ggtataatgg ttccatccgg 4740
   cccaggggcg tagttaccct cacagatttg catttcccac gctttgagtt cagatggggg 4800
   gatcatgtct acctgcgggg cgatgaagaa aacggtttcc ggggtagggg agatcagctg 4860
   ggaagaaagc aggttcctga gcagctgcga cttaccgcag ccggtgggcc cgtaaatcac 4920
   acctattacc gggtgcaact ggtagttaag agagctgcag ctgccgtcat ccctgagcag 4980
   gggggccact tcgttaagca tgtccctgac tcgcatgttt tccctgacca aatccgccag 5040
   aaggcgctcg ccgcccagcg atagcagttc ttgcaaggaa gcaaagtttt tcaacggttt 5100
   gagaccgtcc gccgtaggca tgcttttgag cgtttgacca agcagttcca ggcggtccca 5160
   cagctcggtc acctgctcta cggcatctcg atccagcata tctcctcgtt tcgcgggttg 5220
   gggcggcttt cgctgtacgg cagtagtcgg tgctcgtcca gacgggccag ggtcatgtct 5280
   ttccacgggc gcagggtcct cgtcagcgta gtctgggtca cggtgaaggg gtgcgctccg 5340
   ggctgcgcgc tggccagggt gcgcttgagg ctggtcctgc tggtgctgaa gcgctgccgg 5400
   tcttcgccct gcgcgtcggc caggtagcat ttgaccatgg tgtcatagtc cagcccctcc 5460
   gcggcgtggc ccttggcgcg cagcttgccc ttggaggagg cgccgcacga ggggcagtgc 5520
   agacttttga gggcgtagag cttgggcgcg agaaataccg attccgggga gtaggcatcc 5580
   gcgccgcagg ccccgcagac ggtctcgcat tccacgagcc aggtgagctc tggccgttcg 5640
   gggtcaaaaa ccaggtttcc cccatgcttt ttgatgcgtt tcttacctct ggtttccatg 5700
   agccggtgtc cacgctcggt gacgaaaagg ctgtccgtgt ccccgtatac agacttgaga 5760
   ggcctgtcct cgagcggtgt tccgcggtcc tcctcgtata gaaactcgga ccactctgag 5820
   acaaaggctc gcgtccaggc cagcacgaag gaggctaagt gggaggggta gcggtcgttg 5880
   tccactaggg ggtccactcg ctccagggtg tgaagacaca tgtcgccctc ttcggcatca 5940
   aggaaggtga ttggtttgta ggtgtaggcc acgtgaccgg gtgttcctga aggggggcta 6000
   taaaaggggg tgggggcgcg ttcgtcctca ctctcttccg catcgctgtc tgcgagggcc 6060
   agctgttggg gtgagtactc cctctgaaaa gcgggcatga cttctgcgct aagattgtca 6120
   gtttccaaaa acgaggagga tttgatattc acctggcccg cggtgatgcc tttgagggtg 6180
   gccgcatcca tctggtcaga aaagacaatc tttttgttgt caagcttggt ggcaaacgac 6240
   ccgtagaggg cgttggacag caacttggcg atggagcgca gggtttggtt tttgtcgcga 6300
   tcggcgcgct ccttggccgc gatgtttagc tgcacgtatt cgcgcgcaac gcaccgccat 6360
   tcgggaaaga cggtggtgcg ctcgtcgggc accaggtgca cgcgccaacc gcggttgtgc 6420
   agggtgacaa ggtcaacgct ggtggctacc tctccgcgta ggcgctcgtt ggtccagcag 6480
   aggcggccgc ccttgcgcga gcagaatggc ggtagggggt ctagctgcgt ctcgtccggg 6540
   gggtctgcgt ccacggtaaa gaccccgggc agcaggcgcg cgtcgaagta gtctatcttg 6600
   catccttgca agtctagcgc ctgctgccat gcgcgggcgg caagcgcgcg ctcgtatggg 6660
   ttgagtgggg gaccccatgg catggggtgg gtgagcgcgg aggcgtacat gccgcaaatg 6720
   tcgtaaacgt agaggggctc tctgagtatt ccaagatatg tagggtagca tcttccaccg 6780
   cggatgctgg cgcgcacgta atcgtatagt tcgtgcgagg gagcgaggag gtcgggaccg 6840
   aggttgctac gggcgggctg ctctgctcgg aagactatct gcctgaagat ggcatgtgag 6900
   ttggatgata tggttggacg ctggaagacg ttgaagctgg cgtctgtgag acctaccgcg 6960
   tcacgcacga aggaggcgta ggagtcgcgc agcttgttga ccagctcggc ggtgacctgc 7020
   acgtctaggg cgcagtagtc cagggtttcc ttgatgatgt catacttatc ctgtcccttt 7080
   tttttccaca gctcgcggtt gaggacaaac tcttcgcggt ctttccagta ctcttggatc 7140
   ggaaacccgt cggcctccga acggtaagag cctagcatgt agaactggtt gacggcctgg 7200
   taggcgcagc atcccttttc tacgggtagc gcgtatgcct gcgcggcctt ccggagcgag 7260
   gtgtgggtga gcgcaaaggt gtccctgacc atgactttga ggtactggta tttgaagtca 7320
   gtgtcgtcgc atccgccctg ctcccagagc aaaaagtccg tgcgcttttt ggaacgcgga 7380
   tttggcaggg cgaaggtgac atcgttgaag agtatctttc ccgcgcgagg cataaagttg 7440
   cgtgtgatgc ggaagggtcc cggcacctcg gaacggttgt taattacctg ggcggcgagc 7500
   acgatctcgt caaagccgtt gatgttgtgg cccacaatgt aaagttccaa gaagcgcggg 7560
   atgcccttga tggaaggcaa ttttttaagt tcctcgtagg tgagctcttc aggggagctg 7620
   agcccgtgct ctgaaagggc ccagtctgca agatgagggt tggaagcgac gaatgagctc 7680
   cacaggtcac gggccattag catttgcagg tggtcgcgaa aggtcctaaa ctggcgacct 7740
   atggccattt tttctggggt gatgcagtag aaggtaagcg ggtcttgttc ccagcggtcc 7800
   catccaaggt tcgcggctag gtctcgcgcg gcagtcacta gaggctcatc tccgccgaac 7860
   ttcatgacca gcatgaaggg cacgagctgc ttcccaaagg cccccatcca agtataggtc 7920
   tctacatcgt aggtgacaaa gagacgctcg gtgcgaggat gcgagccgat cgggaagaac 7980
   tggatctccc gccaccaatt ggaggagtgg ctattgatgt ggtgaaagta gaagtccctg 8040
   cgacgggccg aacactcgtg ctggcttttg taaaaacgtg cgcagtactg gcagcggtgc 8100
   acgggctgta catcctgcac gaggttgacc tgacgaccgc gcacaaggaa gcagagtggg 8160
   aatttgagcc cctcgcctgg cgggtttggc tggtggtctt ctacttcggc tgcttgtcct 8220
   tgaccgtctg gctgctcgag gggagttacg gtggatcgga ccaccacgcc gcgcgagccc 8280
   aaagtccaga tgtccgcgcg cggcggtcgg agcttgatga caacatcgcg cagatgggag 8340
   ctgtccatgg tctggagctc ccgcggcgtc aggtcaggcg ggagctcctg caggtttacc 8400
   tcgcatagac gggtcagggc gcgggctaga tccaggtgat acctaatttc caggggctgg 8460
   ttggtggcgg cgtcgatggc ttgcaagagg ccgcatcccc gcggcgcgac tacggtaccg 8520
   cgcggcgggc ggtgggccgc gggggtgtcc ttggatgatg catctaaaag cggtgacgcg 8580
   ggcgagcccc cggaggtagg gggggctccg gacccgccgg gagagggggc aggggcacgt 8640
   cggcgccgcg cgcgggcagg agctggtgct gcgcgcgtag gttgctggcg aacgcgacga 8700
   cgcggcggtt gatctcctga atctggcgcc tctgcgtgaa gacgacgggc ccggtgagct 8760
   tgagcctgaa agagagttcg acagaatcaa tttcggtgtc gttgacggcg gcctggcgca 8820
   aaatctcctg cacgtctcct gagttgtctt gataggcgat ctcggccatg aactgctcga 8880
   tctcttcctc ctggagatct ccgcgtccgg ctcgctccac ggtggcggcg aggtcgttgg 8940
   aaatgcgggc catgagctgc gagaaggcgt tgaggcctcc ctcgttccag acgcggctgt 9000
   agaccacgcc cccttcggca tcgcgggcgc gcatgaccac ctgcgcgaga ttgagctcca 9060
   cgtgccgggc gaagacggcg tagtttcgca ggcgctgaaa gaggtagttg agggtggtgg 9120
   cggtgtgttc tgccacgaag aagtacataa cccagcgtcg caacgtggat tcgttgatat 9180
   cccccaaggc ctcaaggcgc tccatggcct cgtagaagtc cacggcgaag ttgaaaaact 9240
   gggagttgcg cgccgacacg gttaactcct cctccagaag acggatgagc tcggcgacag 9300
   tgtcgcgcac ctcgcgctca aaggctacag gggcctcttc ttcttcttca atctcctctt 9360
   ccataagggc ctccccttct tcttcttctg gcggcggtgg gggagggggg acacggcggc 9420
   gacgacggcg caccgggagg cggtcgacaa agcgctcgat catctccccg cggcgacggc 9480
   gcatggtctc ggtgacggcg cggccgttct cgcgggggcg cagttggaag acgccgcccg 9540
   tcatgtcccg gttatgggtt ggcggggggc tgccatgcgg cagggatacg gcgctaacga 9600
   tgcatctcaa caattgttgt gtaggtactc cgccgccgag ggacctgagc gagtccgcat 9660
   cgaccggatc ggaaaacctc tcgagaaagg cgtctaacca gtcacagtcg caaggtaggc 9720
   tgagcaccgt ggcgggcggc agcgggcggc ggtcggggtt gtttctggcg gaggtgctgc 9780
   tgatgatgta attaaagtag gcggtcttga gacggcggat ggtcgacaga agcaccatgt 9840
   ccttgggtcc ggcctgctga atgcgcaggc ggtcggccat gccccaggct tcgttttgac 9900
   atcggcgcag gtctttgtag tagtcttgca tgagcctttc taccggcact tcttcttctc 9960
   cttcctcttg tcctgcatct cttgcatcta tcgctgcggc ggcggcggag tttggccgta 10020
   ggtggcgccc tcttcctccc atgcgtgtga ccccgaagcc cctcatcggc tgaagcaggg 10080
   ctaggtcggc gacaacgcgc tcggctaata tggcctgctg cacctgcgtg agggtagact 10140
   ggaagtcatc catgtccaca aagcggtggt atgcgcccgt gttgatggtg taagtgcagt 10200
   tggccataac ggaccagtta acggtctggt gacccggctg cgagagctcg gtgtacctga 10260
   gacgcgagta agccctcgag tcaaatacgt agtcgttgca agtccgcacc aggtactggt 10320
   atcccaccaa aaagtgcggc ggcggctggc ggtagagggg ccagcgtagg gtggccgggg 10380
   ctccgggggc gagatcttcc aacataaggc gatgatatcc gtagatgtac ctggacatcc 10440
   aggtgatgcc ggcggcggtg gtggaggcgc gcggaaagtc gcggacgcgg ttccagatgt 10500
   tgcgcagcgg caaaaagtgc tccatggtcg ggacgctctg gccggtcagg cgcgcgcaat 10560
   cgttgacgct ctagaccgtg caaaaggaga gcctgtaagc gggcactctt ccgtggtctg 10620
   gtggataaat tcgcaagggt atcatggcgg acgaccgggg ttcgagcccc gtatccggcc 10680
   gtccgccgtg atccatgcgg ttaccgcccg cgtgtcgaac ccaggtgtgc gacgtcagac 10740
   aacgggggag tgctcctttt ggcttccttc caggcgcggc ggctgctgcg ctagcttttt 10800
   tggccactgg ccgcgcgcag cgtaagcggt taggctggaa agcgaaagca ttaagtggct 10860
   cgctccctgt agccggaggg ttattttcca agggttgagt cgcgggaccc ccggttcgag 10920
   tctcggaccg gccggactgc ggcgaacggg ggtttgcctc cccgtcatgc aagaccccgc 10980
   ttgcaaattc ctccggaaac agggacgagc cccttttttg cttttcccag atgcatccgg 11040
   tgctgcggca gatgcgcccc cctcctcagc agcggcaaga gcaagagcag cggcagacat 11100
   gcagggcacc ctcccctcct cctaccgcgt caggaggggc gacatccgcg gttgacgcgg 11160
   cagcagatgg tgattacgaa cccccgcggc gccgggcccg gcactacctg gacttggagg 11220
   agggcgaggg cctggcgcgg ctaggagcgc cctctcctga gcggtaccca agggtgcagc 11280
   tgaagcgtga tacgcgtgag gcgtacgtgc cgcggcagaa cctgtttcgc gaccgcgagg 11340
   gagaggagcc cgaggagatg cgggatcgaa agttccacgc agggcgcgag ctgcggcatg 11400
   gcctgaatcg cgagcggttg ctgcgcgagg aggactttga gcccgacgcg cgaaccggga 11460
   ttagtcccgc gcgcgcacac gtggcggccg ccgacctggt aaccgcatac gagcagacgg 11520
   tgaaccagga gattaacttt caaaaaagct ttaacaacca cgtgcgtacg cttgtggcgc 11580
   gcgaggaggt ggctatagga ctgatgcatc tgtgggactt tgtaagcgcg ctggagcaaa 11640
   acccaaatag caagccgctc atggcgcagc tgttccttat agtgcagcac agcagggaca 11700
   acgaggcatt cagggatgcg ctgctaaaca tagtagagcc cgagggccgc tggctgctcg 11760
   atttgataaa catcctgcag agcatagtgg tgcaggagcg cagcttgagc ctggctgaca 11820
   aggtggccgc catcaactat tccatgctta gcctgggcaa gttttacgcc cgcaagatat 11880
   accatacccc ttacgttccc atagacaagg aggtaaagat cgaggggttc tacatgcgca 11940
   tggcgctgaa ggtgcttacc ttgagcgacg acctgggcgt ttatcgcaac gagcgcatcc 12000
   acaaggccgt gagcgtgagc cggcggcgcg agctcagcga ccgcgagctg atgcacagcc 12060
   tgcaaagggc cctggctggc acgggcagcg gcgatagaga ggccgagtcc tactttgacg 12120
   cgggcgctga cctgcgctgg gccccaagcc gacgcgccct ggaggcagct ggggccggac 12180
   ctgggctggc ggtggcaccc gcgcgcgctg gcaacgtcgg cggcgtggag gaatatgacg 12240
   aggacgatga gtacgagcca gaggacggcg agtactaagc ggtgatgttt ctgatcagat 12300
   gatgcaagac gcaacggacc cggcggtgcg ggcggcgctg cagagccagc cgtccggcct 12360
   taactccacg gacgactggc gccaggtcat ggaccgcatc atgtcgctga ctgcgcgcaa 12420
   tcctgacgcg ttccggcagc agccgcaggc caaccggctc tccgcaattc tggaagcggt 12480
   ggtcccggcg cgcgcaaacc ccacgcacga gaaggtgctg gcgatcgtaa acgcgctggc 12540
   cgaaaacagg gccatccggc ccgacgaggc cggcctggtc tacgacgcgc tgcttcagcg 12600
   cgtggctcgt tacaacagcg gcaacgtgca gaccaacctg gaccggctgg tgggggatgt 12660
   gcgcgaggcc gtggcgcagc gtgagcgcgc gcagcagcag ggcaacctgg gctccatggt 12720
   tgcactaaac gccttcctga gtacacagcc cgccaacgtg ccgcggggac aggaggacta 12780
   caccaacttt gtgagcgcac tgcggctaat ggtgactgag acaccgcaaa gtgaggtgta 12840
   ccagtctggg ccagactatt ttttccagac cagtagacaa ggcctgcaga ccgtaaacct 12900
   gagccaggct ttcaaaaact tgcaggggct gtggggggtg cgggctccca caggcgaccg 12960
   cgcgaccgtg tctagcttgc tgacgcccaa ctcgcgcctg ttgctgctgc taatagcgcc 13020
   cttcacggac agtggcagcg tgtcccggga cacataccta ggtcacttgc tgacactgta 13080
   ccgcgaggcc ataggtcagg cgcatgtgga cgagcatact ttccaggaga ttacaagtgt 13140
   cagccgcgcg ctggggcagg aggacacggg cagcctggag gcaaccctaa actacctgct 13200
   gaccaaccgg cggcagaaga tcccctcgtt gcacagttta aacagcgagg aggagcgcat 13260
   tttgcgctac gtgcagcaga gcgtgagcct taacctgatg cgcgacgggg taacgcccag 13320
   cgtggcgctg gacatgaccg cgcgcaacat ggaaccgggc atgtatgcct caaaccggcc 13380
   gtttatcaac cgcctaatgg actacttgca tcgcgcggcc gccgtgaacc ccgagtattt 13440
   caccaatgcc atcttgaacc cgcactggct accgccccct ggtttctaca ccgggggatt 13500
   cgaggtgccc gagggtaacg atggattcct ctgggacgac atagacgaca gcgtgttttc 13560
   cccgcaaccg cagaccctgc tagagttgca acagcgcgag caggcagagg cggcgctgcg 13620
   aaaggaaagc ttccgcaggc caagcagctt gtccgatcta ggcgctgcgg ccccgcggtc 13680
   agatgctagt agcccatttc caagcttgat agggtctctt accagcactc gcaccacccg 13740
   cccgcgcctg ctgggcgagg aggagtacct aaacaactcg ctgctgcagc cgcagcgcga 13800
   aaaaaacctg cctccggcat ttcccaacaa cgggatagag agcctagtgg acaagatgag 13860
   tagatggaag acgtacgcgc aggagcacag ggacgtgcca ggcccgcgcc cgcccacccg 13920
   tcgtcaaagg cacgaccgtc agcggggtct ggtgtgggag gacgatgact cggcagacga 13980
   cagcagcgtc ctggatttgg gagggagtgg caacccgttt gcgcaccttc gccccaggct 14040
   ggggagaatg ttttaaaaaa aaaaaagcat gatgcaaaat aaaaaactca ccaaggccat 14100
   ggcaccgagc gttggttttc ttgtattccc cttagtatgc ggcgcgcggc gatgtatgag 14160
   gaaggtcctc ctccctccta cgagagtgtg gtgagcgcgg cgccagtggc ggcggcgctg 14220
   ggttctccct tcgatgctcc cctggacccg ccgtttgtgc ctccgcggta cctgcggcct 14280
   accgggggga gaaacagcat ccgttactct gagttggcac ccctattcga caccacccgt 14340
   gtgtacctgg tggacaacaa gtcaacggat gtggcatccc tgaactacca gaacgaccac 14400
   agcaactttc tgaccacggt cattcaaaac aatgactaca gcccggggga ggcaagcaca 14460
   cagaccatca atcttgacga ccggtcgcac tggggcggcg acctgaaaac catcctgcat 14520
   accaacatgc caaatgtgaa cgagttcatg tttaccaata agtttaaggc gcgggtgatg 14580
   gtgtcgcgct tgcctactaa ggacaatcag gtggagctga aatacgagtg ggtggagttc 14640
   acgctgcccg agggcaacta ctccgagacc atgaccatag accttatgaa caacgcgatc 14700
   gtggagcact acttgaaagt gggcagacag aacggggttc tggaaagcga catcggggta 14760
   aagtttgaca cccgcaactt cagactgggg tttgaccccg tcactggtct tgtcatgcct 14820
   ggggtatata caaacgaagc cttccatcca gacatcattt tgctgccagg atgcggggtg 14880
   gacttcaccc acagccgcct gagcaacttg ttgggcatcc gcaagcggca acccttccag 14940
   gagggcttta ggatcaccta cgatgatctg gagggtggta acattcccgc actgttggat 15000
   gtggacgcct accaggcgag cttgaaagat gacaccgaac agggcggggg tggcgcaggc 15060
   ggcagcaaca gcagtggcag cggcgcggaa gagaactcca acgcggcagc cgcggcaatg 15120
   cagccggtgg aggacatgaa cgatcatgcc attcgcggcg acacctttgc cacacgggct 15180
   gaggagaagc gcgctgaggc cgaagcagcg gccgaagctg ccgcccccgc tgcgcaaccc 15240
   gaggtcgaga agcctcagaa gaaaccggtg atcaaacccc tgacagagga cagcaagaaa 15300
   cgcagttaca acctaataag caatgacagc accttcaccc agtaccgcag ctggtacctt 15360
   gcatacaact acggcgaccc tcagaccgga atccgctcat ggaccctgct ttgcactcct 15420
   gacgtaacct gcggctcgga gcaggtctac tggtcgttgc cagacatgat gcaagacccc 15480
   gtgaccttcc gctccacgcg ccagatcagc aactttccgg tggtgggcgc cgagctgttg 15540
   cccgtgcact ccaagagctt ctacaacgac caggccgtct actcccaact catccgccag 15600
   tttacctctc tgacccacgt gttcaatcgc tttcccgaga accagatttt ggcgcgcccg 15660
   ccagccccca ccatcaccac cgtcagtgaa aacgttcctg ctctcacaga tcacgggacg 15720
   ctaccgctgc gcaacagcat cggaggagtc cagcgagtga ccattactga cgccagacgc 15780
   cgcacctgcc cctacgttta caaggccctg ggcatagtct cgccgcgcgt cctatcgagc 15840
   cgcacttttt gagcaagcat gtccatcctt atatcgccca gcaataacac aggctggggc 15900
   ctgcgcttcc caagcaagat gtttggcggg gccaagaagc gctccgacca acacccagtg 15960
   cgcgtgcgcg ggcactaccg cgcgccctgg ggcgcgcaca aacgcggccg cactgggcgc 16020
   accaccgtcg atgacgccat cgacgcggtg gtggaggagg cgcgcaacta cacgcccacg 16080
   ccgccaccag tgtccacagt ggacgcggcc attcagaccg tggtgcgcgg agcccggcgc 16140
   tatgctaaaa tgaagagacg gcggaggcgc gtagcacgtc gccaccgccg ccgacccggc 16200
   actgccgccc aacgcgcggc ggcggccctg cttaaccgcg cacgtcgcac cggccgacgg 16260
   gcggccatgc gggccgctcg aaggctggcc gcgggtattg tcactgtgcc ccccaggtcc 16320
   aggcgacgag cggccgccgc agcagccgcg gccattagtg ctatgactca gggtcgcagg 16380
   ggcaacgtgt attgggtgcg cgactcggtt agcggcctgc gcgtgcccgt gcgcacccgc 16440
   cccccgcgca actagattgc aagaaaaaac tacttagact cgtactgttg tatgtatcca 16500
   gcggcggcgg cgcgcaacga agctatgtcc aagcgcaaaa tcaaagaaga gatgctccag 16560
   gtcatcgcgc cggagatcta tggccccccg aagaaggaag agcaggatta caagccccga 16620
   aagctaaagc gggtcaaaaa gaaaaagaaa gatgatgatg atgaacttga cgacgaggtg 16680
   gaactgctgc acgctaccgc gcccaggcga cgggtacagt ggaaaggtcg acgcgtaaaa 16740
   cgtgttttgc gacccggcac caccgtagtc tttacgcccg gtgagcgctc cacccgcacc 16800
   tacaagcgcg tgtatgatga ggtgtacggc gacgaggacc tgcttgagca ggccaacgag 16860
   cgcctcgggg agtttgccta cggaaagcgg cataaggaca tgctggcgtt gccgctggac 16920
   gagggcaacc caacacctag cctaaagccc gtaacactgc agcaggtgct gcccgcgctt 16980
   gcaccgtccg aagaaaagcg cggcctaaag cgcgagtctg gtgacttggc acccaccgtg 17040
   cagctgatgg tacccaagcg ccagcgactg gaagatgtct tggaaaaaat gaccgtggaa 17100
   cctgggctgg agcccgaggt ccgcgtgcgg ccaatcaagc aggtggcgcc gggactgggc 17160
   gtgcagaccg tggacgttca gatacccact accagtagca ccagtattgc caccgccaca 17220
   gagggcatgg agacacaaac gtccccggtt gcctcagcgg tggcggatgc cgcggtgcag 17280
   gcggtcgctg cggccgcgtc caagacctct acggaggtgc aaacggaccc gtggatgttt 17340
   cgcgtttcag ccccccggcg cccgcgcggt tcgaggaagt acggcgccgc cagcgcgcta 17400
   ctgcccgaat atgccctaca tccttccatt gcgcctaccc ccggctatcg tggctacacc 17460
   taccgcccca gaagacgagc aactacccga cgccgaacca ccactggaac ccgccgccgc 17520
   cgtcgccgtc gccagcccgt gctggccccg atttccgtgc gcagggtggc tcgcgaagga 17580
   ggcaggaccc tggtgctgcc aacagcgcgc taccacccca gcatcgttta aaagccggtc 17640
   tttgtggttc ttgcagatat ggccctcacc tgccgcctcc gtttcccggt gccgggattc 17700
   cgaggaagaa tgcaccgtag gaggggcatg gccggccacg gcctgacggg cggcatgcgt 17760
   cgtgcgcacc accggcggcg gcgcgcgtcg caccgtcgca tgcgcggcgg tatcctgccc 17820
   ctccttattc cactgatcgc cgcggcgatt ggcgccgtgc ccggaattgc atccgtggcc 17880
   ttgcaggcgc agagacactg attaaaaaca agttgcatgt ggaaaaatca aaataaaaag 17940
   tctggactct cacgctcgct tggtcctgta actattttgt agaatggaag acatcaactt 18000
   tgcgtctctg gccccgcgac acggctcgcg cccgttcatg ggaaactggc aagatatcgg 18060
   caccagcaat atgagcggtg gcgccttcag ctggggctcg ctgtggagcg gcattaaaaa 18120
   tttcggttcc accgttaaga actatggcag caaggcctgg aacagcagca caggccagat 18180
   gctgagggat aagttgaaag agcaaaattt ccaacaaaag gtggtagatg gcctggcctc 18240
   tggcattagc ggggtggtgg acctggccaa ccaggcagtg caaaataaga ttaacagtaa 18300
   gcttgatccc cgccctcccg tagaggagcc tccaccggcc gtggagacag tgtctccaga 18360
   ggggcgtggc gaaaagcgtc cgcgccccga cagggaagaa actctggtga cgcaaataga 18420
   cgagcctccc tcgtacgagg aggcactaaa gcaaggcctg cccaccaccc gtcccatcgc 18480
   gcccatggct accggagtgc tgggccagca cacacccgta acgctggacc tgcctccccc 18540
   cgccgacacc cagcagaaac ctgtgctgcc aggcccgacc gccgttgttg taacccgtcc 18600
   tagccgcgcg tccctgcgcc gcgccgccag cggtccgcga tcgttgcggc ccgtagccag 18660
   tggcaactgg caaagcacac tgaacagcat cgtgggtctg ggggtgcaat ccctgaagcg 18720
   ccgacgatgc ttctgaatag ctaacgtgtc gtatgtgtgt catgtatgcg tccatgtcgc 18780
   cgccagagga gctgctgagc cgccgcgcgc ccgctttcca agatggctac cccttcgatg 18840
   atgccgcagt ggtcttacat gcacatctcg ggccaggacg cctcggagta cctgagcccc 18900
   gggctggtgc agtttgcccg cgccaccgag acgtacttca gcctgaataa caagtttaga 18960
   aaccccacgg tggcgcctac gcacgacgtg accacagacc ggtcccagcg tttgacgctg 19020
   cggttcatcc ctgtggaccg tgaggatact gcgtactcgt acaaggcgcg gttcacccta 19080
   gctgtgggtg ataaccgtgt gctggacatg gcttccacgt actttgacat ccgcggcgtg 19140
   ctggacaggg gccctacttt taagccctac tctggcactg cctacaacgc cctggctccc 19200
   aagggtgccc caaatccttg cgaatgggat gaagctgcta ctgctcttga aataaaccta 19260
   gaagaagagg acgatgacaa cgaagacgaa gtagacgagc aagctgagca gcaaaaaact 19320
   cacgtatttg ggcaggcgcc ttattctggt ataaatatta caaaggaggg tattcaaata 19380
   ggtgtcgaag gtcaaacacc taaatatgcc gataaaacat ttcaacctga acctcaaata 19440
   ggagaatctc agtggtacga aactgaaatt aatcatgcag ctgggagagt ccttaaaaag 19500
   actaccccaa tgaaaccatg ttacggttca tatgcaaaac ccacaaatga aaatggaggg 19560
   caaggcattc ttgtaaagca acaaaatgga aagctagaaa gtcaagtgga aatgcaattt 19620
   ttctcaacta ctgaggcgac cgcaggcaat ggtgataact tgactcctaa agtggtattg 19680
   tacagtgaag atgtagatat agaaacccca gacactcata tttcttacat gcccactatt 19740
   aaggaaggta actcacgaga actaatgggc caacaatcta tgcccaacag gcctaattac 19800
   attgctttta gggacaattt tattggtcta atgtattaca acagcacggg taatatgggt 19860
   gttctggcgg gccaagcatc gcagttgaat gctgttgtag atttgcaaga cagaaacaca 19920
   gagctttcat accagctttt gcttgattcc attggtgata gaaccaggta cttttctatg 19980
   tggaatcagg ctgttgacag ctatgatcca gatgttagaa ttattgaaaa tcatggaact 20040
   gaagatgaac ttccaaatta ctgctttcca ctgggaggtg tgattaatac agagactctt 20100
   accaaggtaa aacctaaaac aggtcaggaa aatggatggg aaaaagatgc tacagaattt 20160
   tcagataaaa atgaaataag agttggaaat aattttgcca tggaaatcaa tctaaatgcc 20220
   aacctgtgga gaaatttcct gtactccaac atagcgctgt atttgcccga caagctaaag 20280
   tacagtcctt ccaacgtaaa aatttctgat aacccaaaca cctacgacta catgaacaag 20340
   cgagtggtgg ctcccgggtt agtggactgc tacattaacc ttggagcacg ctggtccctt 20400
   gactatatgg acaacgtcaa cccatttaac caccaccgca atgctggcct gcgctaccgc 20460
   tcaatgttgc tgggcaatgg tcgctatgtg cccttccaca tccaggtgcc tcagaagttc 20520
   tttgccatta aaaacctcct tctcctgccg ggctcataca cctacgagtg gaacttcagg 20580
   aaggatgtta acatggttct gcagagctcc ctaggaaatg acctaagggt tgacggagcc 20640
   agcattaagt ttgatagcat ttgcctttac gccaccttct tccccatggc ccacaacacc 20700
   gcctccacgc ttgaggccat gcttagaaac gacaccaacg accagtcctt taacgactat 20760
   ctctccgccg ccaacatgct ctaccctata cccgccaacg ctaccaacgt gcccatatcc 20820
   atcccctccc gcaactgggc ggctttccgc ggctgggcct tcacgcgcct taagactaag 20880
   gaaaccccat cactgggctc gggctacgac ccttattaca cctactctgg ctctataccc 20940
   tacctagatg gaacctttta cctcaaccac acctttaaga aggtggccat tacctttgac 21000
   tcttctgtca gctggcctgg caatgaccgc ctgcttaccc ccaacgagtt tgaaattaag 21060
   cgctcagttg acggggaggg ttacaacgtt gcccagtgta acatgaccaa agactggttc 21120
   ctggtacaaa tgctagctaa ctacaacatt ggctaccagg gcttctatat cccagagagc 21180
   tacaaggacc gcatgtactc cttctttaga aacttccagc ccatgagccg tcaggtggtg 21240
   gatgatacta aatacaagga ctaccaacag gtgggcatcc tacaccaaca caacaactct 21300
   ggatttgttg gctaccttgc ccccaccatg cgcgaaggac aggcctaccc tgctaacttc 21360
   ccctatccgc ttataggcaa gaccgcagtt gacagcatta cccagaaaaa gtttctttgc 21420
   gatcgcaccc tttggcgcat cccattctcc agtaacttta tgtccatggg cgcactcaca 21480
   gacctgggcc aaaaccttct ctacgccaac tccgcccacg cgctagacat gacttttgag 21540
   gtggatccca tggacgagcc cacccttctt tatgttttgt ttgaagtctt tgacgtggtc 21600
   cgtgtgcacc ggccgcaccg cggcgtcatc gaaaccgtgt acctgcgcac gcccttctcg 21660
   gccggcaacg ccacaacata aagaagcaag caacatcaac aacagctgcc gccatgggct 21720
   ccagtgagca ggaactgaaa gccattgtca aagatcttgg ttgtgggcca tattttttgg 21780
   gcacctatga caagcgcttt ccaggctttg tttctccaca caagctcgcc tgcgccatag 21840
   tcaatacggc cggtcgcgag actgggggcg tacactggat ggcctttgcc tggaacccgc 21900
   actcaaaaac atgctacctc tttgagccct ttggcttttc tgaccagcga ctcaagcagg 21960
   tttaccagtt tgagtacgag tcactcctgc gccgtagcgc cattgcttct tcccccgacc 22020
   gctgtataac gctggaaaag tccacccaaa gcgtacaggg gcccaactcg gccgcctgtg 22080
   gactattctg ctgcatgttt ctccacgcct ttgccaactg gccccaaact cccatggatc 22140
   acaaccccac catgaacctt attaccgggg tacccaactc catgctcaac agtccccagg 22200
   tacagcccac cctgcgtcgc aaccaggaac agctctacag cttcctggag cgccactcgc 22260
   cctacttccg cagccacagt gcgcagatta ggagcgccac ttctttttgt cacttgaaaa 22320
   acatgtaaaa ataatgtact agagacactt tcaataaagg caaatgcttt tatttgtaca 22380
   ctctcgggtg attatttacc cccacccttg ccgtctgcgc cgtttaaaaa tcaaaggggt 22440
   tctgccgcgc atcgctatgc gccactggca gggacacgtt gcgatactgg tgtttagtgc 22500
   tccacttaaa ctcaggcaca accatccgcg gcagctcggt gaagttttca ctccacaggc 22560
   tgcgcaccat caccaacgcg tttagcaggt cgggcgccga tatcttgaag tcgcagttgg 22620
   ggcctccgcc ctgcgcgcgc gagttgcgat acacagggtt gcagcactgg aacactatca 22680
   gcgccgggtg gtgcacgctg gccagcacgc tcttgtcgga gatcagatcc gcgtccaggt 22740
   cctccgcgtt gctcagggcg aacggagtca actttggtag ctgccttccc aaaaagggcg 22800
   cgtgcccagg ctttgagttg cactcgcacc gtagtggcat caaaaggtga ccgtgcccgg 22860
   tctgggcgtt aggatacagc gcctgcataa aagccttgat ctgcttaaaa gccacctgag 22920
   cctttgcgcc ttcagagaag aacatgccgc aagacttgcc ggaaaactga ttggccggac 22980
   aggccgcgtc gtgcacgcag caccttgcgt cggtgttgga gatctgcacc acatttcggc 23040
   cccaccggtt cttcacgatc ttggccttgc tagactgctc cttcagcgcg cgctgcccgt 23100
   tttcgctcgt cacatccatt tcaatcacgt gctccttatt tatcataatg cttccgtgta 23160
   gacacttaag ctcgccttcg atctcagcgc agcggtgcag ccacaacgcg cagcccgtgg 23220
   gctcgtgatg cttgtaggtc acctctgcaa acgactgcag gtacgcctgc aggaatcgcc 23280
   ccatcatcgt cacaaaggtc ttgttgctgg tgaaggtcag ctgcaacccg cggtgctcct 23340
   cgttcagcca ggtcttgcat acggccgcca gagcttccac ttggtcaggc agtagtttga 23400
   agttcgcctt tagatcgtta tccacgtggt acttgtccat cagcgcgcgc gcagcctcca 23460
   tgcccttctc ccacgcagac acgatcggca cactcagcgg gttcatcacc gtaatttcac 23520
   tttccgcttc gctgggctct tcctcttcct cttgcgtccg cataccacgc gccactgggt 23580
   cgtcttcatt cagccgccgc actgtgcgct tacctccttt gccatgcttg attagcaccg 23640
   gtgggttgct gaaacccacc atttgtagcg ccacatcttc tctttcttcc tcgctgtcca 23700
   cgattacctc tggtgatggc gggcgctcgg gcttgggaga agggcgcttc tttttcttct 23760
   tgggcgcaat ggccaaatcc gccgccgagg tcgatggccg cgggctgggt gtgcgcggca 23820
   ccagcgcgtc ttgtgatgag tcttcctcgt cctcggactc gatacgccgc ctcatccgct 23880
   tttttggggg cgcccgggga ggcggcggcg acggggacgg ggacgacacg tcctccatgg 23940
   ttgggggacg tcgcgccgca ccgcgtccgc gctcgggggt ggtttcgcgc tgctcctctt 24000
   cccgactggc catttccttc tcctataggc agaaaaagat catggagtca gtcgagaaga 24060
   aggacagcct aaccgccccc tctgagttcg ccaccaccgc ctccaccgat gccgccaacg 24120
   cgcctaccac cttccccgtc gaggcacccc cgcttgagga ggaggaagtg attatcgagc 24180
   aggacccagg ttttgtaagc gaagacgacg aggaccgctc agtaccaaca gaggataaaa 24240
   agcaagacca ggacaacgca gaggcaaacg aggaacaagt cgggcggggg gacgaaaggc 24300
   atggcgacta cctagatgtg ggagacgacg tgctgttgaa gcatctgcag cgccagtgcg 24360
   ccattatctg cgacgcgttg caagagcgca gcgatgtgcc cctcgccata gcggatgtca 24420
   gccttgccta cgaacgccac ctattctcac cgcgcgtacc ccccaaacgc caagaaaacg 24480
   gcacatgcga gcccaacccg cgcctcaact tctaccccgt atttgccgtg ccagaggtgc 24540
   ttgccaccta tcacatcttt ttccaaaact gcaagatacc cctatcctgc cgtgccaacc 24600
   gcagccgagc ggacaagcag ctggccttgc ggcagggcgc tgtcatacct gatatcgcct 24660
   cgctcaacga agtgccaaaa atctttgagg gtcttggacg cgacgagaag cgcgcggcaa 24720
   acgctctgca acaggaaaac agcgaaaatg aaagtcactc tggagtgttg gtggaactcg 24780
   agggtgacaa cgcgcgccta gccgtactaa aacgcagcat cgaggtcacc cactttgcct 24840
   acccggcact taacctaccc cccaaggtca tgagcacagt catgagtgag ctgatcgtgc 24900
   gccgtgcgca gcccctggag agggatgcaa atttgcaaga acaaacagag gagggcctac 24960
   ccgcagttgg cgacgagcag ctagcgcgct ggcttcaaac gcgcgagcct gccgacttgg 25020
   aggagcgacg caaactaatg atggccgcag tgctcgttac cgtggagctt gagtgcatgc 25080
   agcggttctt tgctgacccg gagatgcagc gcaagctaga ggaaacattg cactacacct 25140
   ttcgacaggg ctacgtacgc caggcctgca agatctccaa cgtggagctc tgcaacctgg 25200
   tctcctacct tggaattttg cacgaaaacc gccttgggca aaacgtgctt cattccacgc 25260
   tcaagggcga ggcgcgccgc gactacgtcc gcgactgcgt ttacttattt ctatgctaca 25320
   cctggcagac ggccatgggc gtttggcagc agtgcttgga ggagtgcaac ctcaaggagc 25380
   tgcagaaact gctaaagcaa aacttgaagg acctatggac ggccttcaac gagcgctccg 25440
   tggccgcgca cctggcggac atcattttcc ccgaacgcct gcttaaaacc ctgcaacagg 25500
   gtctgccaga cttcaccagt caaagcatgt tgcagaactt taggaacttt atcctagagc 25560
   gctcaggaat cttgcccgcc acctgctgtg cacttcctag cgactttgtg cccattaagt 25620
   accgcgaatg ccctccgccg ctttggggcc actgctacct tctgcagcta gccaactacc 25680
   ttgcctacca ctctgacata atggaagacg tgagcggtga cggtctactg gagtgtcact 25740
   gtcgctgcaa cctatgcacc ccgcaccgct ccctggtttg caattcgcag ctgcttaacg 25800
   aaagtcaaat tatcggtacc tttgagctgc agggtccctc gcctgacgaa aagtccgcgg 25860
   ctccggggtt gaaactcact ccggggctgt ggacgtcggc ttaccttcgc aaatttgtac 25920
   ctgaggacta ccacgcccac gagattaggt tctacgaaga ccaatcccgc ccgccaaatg 25980
   cggagcttac cgcctgcgtc attacccagg gccacattct tggccaattg caagccatca 26040
   acaaagcccg ccaagagttt ctgctacgaa agggacgggg ggtttacttg gacccccagt 26100
   ccggcgagga gctcaaccca atccccccgc cgccgcagcc ctatcagcag cagccgcggg 26160
   cccttgcttc ccaggatggc acccaaaaag aagctgcagc tgccgccgcc acccacggac 26220
   gaggaggaat actgggacag tcaggcagag gaggttttgg acgaggagga ggaggacatg 26280
   atggaagact gggagagcct agacgaggaa gcttccgagg tcgaagaggt gtcagacgaa 26340
   acaccgtcac cctcggtcgc attcccctcg ccggcgcccc agaaatcggc aaccggttcc 26400
   agcatggcta caacctccgc tcctcaggcg ccgccggcac tgcccgttcg ccgacccaac 26460
   cgtagatggg acaccactgg aaccagggcc ggtaagtcca agcagccgcc gccgttagcc 26520
   caagagcaac aacagcgcca aggctaccgc tcatggcgcg ggcacaagaa cgccatagtt 26580
   gcttgcttgc aagactgtgg gggcaacatc tccttcgccc gccgctttct tctctaccat 26640
   cacggcgtgg ccttcccccg taacatcctg cattactacc gtcatctcta cagcccatac 26700
   tgcaccggcg gcagcggcag cggcagcaac agcagcggcc acacagaagc aaaggcgacc 26760
   ggatagcaag actctgacaa agcccaagaa atccacagcg gcggcagcag caggaggagg 26820
   agcgctgcgt ctggcgccca acgaacccgt atcgacccgc gagcttagaa acaggatttt 26880
   tcccactctg tatgctatat ttcaacagag caggggccaa gaacaagagc tgaaaataaa 26940
   aaacaggtct ctgcgatccc tcacccgcag ctgcctgtat cacaaaagcg aagatcagct 27000
   tcggcgcacg ctggaagacg cggaggctct cttcagtaaa tactgcgcgc tgactcttaa 27060
   ggactagttt cgcgcccttt ctcaaattta agcgcgaaaa ctacgtcatc tccagcggcc 27120
   acacccggcg ccagcacctg tcgtcagcgc cattatgagc aaggaaattc ccacgcccta 27180
   catgtggagt taccagccac aaatgggact tgcggctgga gctgcccaag actactcaac 27240
   ccgaataaac tacatgagcg cgggacccca catgatatcc cgggtcaacg gaatccgcgc 27300
   ccaccgaaac cgaattctct tggaacaggc ggctattacc accacacctc gtaataacct 27360
   taatccccgt agttggcccg ctgccctggt gtaccaggaa agtcccgctc ccaccactgt 27420
   ggtacttccc agagacgccc aggccgaagt tcagatgact aactcagggg cgcagcttgc 27480
   gggcggcttt cgtcacaggg tgcggtcgcc cgggcagggt ataactcacc tgacaatcag 27540
   agggcgaggt attcagctca acgacgagtc ggtgagctcc tcgcttggtc tccgtccgga 27600
   cgggacattt cagatcggcg gcgccggccg ctcttcattc acgcctcgtc aggcaatcct 27660
   aactctgcag acctcgtcct ctgagccgcg ctctggaggc attggaactc tgcaatttat 27720
   tgaggagttt gtgccatcgg tctactttaa ccccttctcg ggacctcccg gccactatcc 27780
   ggatcaattt attcctaact ttgacgcggt aaaggactcg gcggatggct acgactgaat 27840
   gttaagtgga gaggcagagc aactgcgcct gaaacacctg gtccactgtc gccgccacaa 27900
   gtgctttgcc cgcgactccg gtgagttttg ctactttgaa ttgcccgagg atcatatcga 27960
   gggcccggcg cacggcgtcc ggcttaccgc ccagggagag cttgcccgta gcctgattcg 28020
   ggagtttacc cagcgccccc tgctagttga gcgggacagg ggaccctgtg ttctcactgt 28080
   gatttgcaac tgtcctaacc ctggattaca tcaagatctt tgttgccatc tctgtgctga 28140
   gtataataaa tacagaaatt aaaatatact ggggctccta tcgccatcct gtaaacgcca 28200
   ccgtcttcac ccgcccaagc aaaccaaggc gaaccttacc tggtactttt aacatctctc 28260
   cctctgtgat ttacaacagt ttcaacccag acggagtgag tctacgagag aacctctccg 28320
   agctcagcta ctccatcaga aaaaacacca ccctccttac ctgccgggaa cgtacgatgt 28380
   ggctgcagag cctgctgctc ttgggcactg tggcctgcag catctctgca cccgcccgct 28440
   cgcccagccc cagcacgcag ccctgggagc atgtgaatgc catccaggag gcccggcgtc 28500
   tcctgaacct gagtagagac actgctgctg agatgaatga aacagtagaa gtcatctcag 28560
   aaatgtttga cctccaggag ccgacctgcc tacagacccg cctggagctg tacaagcagg 28620
   gcctgcgggg cagcctcacc aagctcaagg gccccttgac catgatggcc agccactaca 28680
   agcagcactg ccctccaacc ccggaaactt cctgtgcaac ccagactatc acctttgaaa 28740
   gtttcaaaga gaacctgaag gactttctgc ttgtcatccc ctttgactgc tgggagccag 28800
   tccaggagtg acaattgact ctatgtggga tatgctccag cgctacaacc ttgaagtcag 28860
   gcttcctgga tgtcagcatc tgactttggc cagcacctgt cccgcggatt tgttccagtc 28920
   caactacagc gacccaccct aacagagatg accaacacaa ccaacgcggc cgccgctacc 28980
   ggacttacat ctaccacaaa tacaccccaa gtttctgcct ttgtcaataa ctgggataac 29040
   ttgggcatgt ggtggttctc catagcgctt atgtttgtat gccttattat tatgtggctc 29100
   atctgctgcc taaagcgcaa acgcgcccga ccacccatct atagtcccat cattgtgcta 29160
   cacccaaaca atgatggaat ccatagattg gacggactga aacacatgtt cttttctctt 29220
   acagtatgat taaatgagac atgattcctc gagtttttat attactgacc cttgttgcgc 29280
   ttttttgtgc gtgctccaca ttggctgcgg tttctcacat cgaagtagac tgcattccag 29340
   ccttcacagt ctatttgctt tacggatttg tcaccctcac gctcatctgc agcctcatca 29400
   ctgtggtcat cgcctttatc cagtgcattg actgggtctg tgtgcgcttt gcatatctca 29460
   gacaccatcc ccagtacagg gacaggacta tagctgagct tcttagaatt ctttaattat 29520
   gaaatttact gtgacttttc tgctgattat ttgcacccta tctgcgtttt gttccccgac 29580
   ctccaagcct caaagacata tatcatgcag attcactcgt atatggaata ttccaagttg 29640
   ctacaatgaa aaaagcgatc tttccgaagc ctggttatat gcaatcatct ctgttatggt 29700
   gttctgcagt accatcttag ccctagctat atatccctac cttgacattg gctggaaacg 29760
   aatagatgcc atgaaccacc caactttccc cgcgcccgct atgcttccac tgcaacaagt 29820
   tgttgccggc ggctttgtcc cagccaatca gcctcgcccc acttctccca cccccactga 29880
   aatcagctac tttaatctaa caggaggaga tgactgacac cctagatcta gaaatggacg 29940
   gaattattac agagcagcgc ctgctagaaa gacgcagggc agcggccgag caacagcgca 30000
   tgaatcaaga gctccaagac atggttaact tgcaccagtg caaaaggggt atcttttgtc 30060
   tggtaaagca ggccaaagtc acctacgaca gtaataccac cggacaccgc cttagctaca 30120
   agttgccaac caagcgtcag aaattggtgg tcatggtggg agaaaagccc attaccataa 30180
   ctcagcactc ggtagaaacc gaaggctgca ttcactcacc ttgtcaagga cctgaggatc 30240
   tctgcaccct tattaagacc ctgtgcggtc tcaaagatct tattcccttt aactaataaa 30300
   aaaaaataat aaagcatcac ttacttaaaa tcagttagca aatttctgtc cagtttattc 30360
   agcagcacct ccttgccctc ctcccagctc tggtattgca gcttcctcct ggctgcaaac 30420
   tttctccaca atctaaatgg aatgtcagtt tcctcctgtt cctgtccatc cgcacccact 30480
   atcttcatgt tgttgcagat gaagcgcgca agaccgtctg aagatacctt caaccccgtg 30540
   tatccatacc catttaaatg ggtgacacgg aaaccggtcc tccaactgtg ccttttctta 30600
   ctcctccctt tgtatccccc aatgggtttc aagagagtcc ccctggggta ctctctttgc 30660
   gcctatccga acctctagtt acctccaatg gcatgcttgc gctcaaaatg ggcaacggcc 30720
   tctctctgga cgaggccggc aaccttacct cccaaaatgt aaccactgtg agcccacctc 30780
   tcaaaaaaac caagtcaaac ataaacctgg aaatatctgc acccctcaca gttacctcag 30840
   aagccctaac tgtggctgcc gccgcacctc taatggtcgc gggcaacaca ctcaccatgc 30900
   aatcacaggc cccgctaacc gtgcacgact ccaaacttag cattgccacc caaggacccc 30960
   tcacagtgtc agaaggaaag ctagccctgc aaacatcagg ccccctcacc accaccgata 31020
   gcagtaccct tactatcact gcctcacccc ctctaactac tgccactggt agcttgggca 31080
   ttgacttgaa agagcccatt tatacacaaa atggaaaact aggactaaag tacggggctc 31140
   ctttgcatgt aacagacgac ctaaacactt tgaccgtagc aactggtcca ggtgtgacta 31200
   ttaataatac ttccttgcaa actaaagtta ctggagcctt gggttttgat tcacaaggca 31260
   atatgcaact taatgtagca ggaggactaa ggattgattc tcaaaacaga cgccttatac 31320
   ttgatgttag ttatccgttt gatgctcaaa accaactaaa tctaagacta ggacagggcc 31380
   ctctttttat aaactcagcc cacaacttgg atattaacta caacaaaggc ctttacttgt 31440
   ttacagcttc aaacaattcc aaaaagcttg aggttaacct aagcactgcc aaggggttga 31500
   tgtttgacgc tacagccata gccattaatg caggagatgg gcttgaattt ggttcaccta 31560
   atgcaccaaa cacaaatccc ctcaaaacaa aaattggcca tggcctagaa tttgattcaa 31620
   acaaggctat ggttcctaaa ctaggaactg gccttagttt tgacagcaca ggtgccatta 31680
   cagtaggaaa caaaaataat gataagctaa ctttgtggac cacaccagct ccatctccta 31740
   actgtagact aaatgcagag aaagatgcta aactcacttt ggtcttaaca aaatgtggca 31800
   gtcaaatact tgctacagtt tcagttttgg ctgttaaagg cagtttggct ccaatatctg 31860
   gaacagttca aagtgctcat cttattataa gatttgacga aaatggagtg ctactaaaca 31920
   attccttcct ggacccagaa tattggaact ttagaaatgg agatcttact gaaggcacag 31980
   cctatacaaa cgctgttgga tttatgccta acctatcagc ttatccaaaa tctcacggta 32040
   aaactgccaa aagtaacatt gtcagtcaag tttacttaaa cggagacaaa actaaacctg 32100
   taacactaac cattacacta aacggtacac aggaaacagg agacacaact ccaagtgcat 32160
   actctatgtc attttcatgg gactggtctg gccacaacta cattaatgaa atatttgcca 32220
   catcctctta cactttttca tacattgcgc aagaaggatc aggatcaggt tcagggagtg 32280
   gctctaaaaa gaagaaaaag aagaagtaat aaagtaatcg tttgtgttat gtttcaacgt 32340
   gtttattttt caattgcaga aaatttcaag tcatttttca ttcagtagta tagccccacc 32400
   accacatagc ttatacagat caccgtacct taatcaaact cacagaaccc tagtattcaa 32460
   cctgccacct ccctcccaac acacagagta cacagtcctt tctccccggc tggccttaaa 32520
   aagcatcata tcatgggtaa cagacatatt cttaggtgtt atattccaca cggtttcctg 32580
   tcgagccaaa cgctcatcag tgatattaat aaactccccg ggcagctcac ttaagttcat 32640
   gtcgctgtcc agctgctgag ccacaggctg ctgtccaact tgcggttgct taacgggcgg 32700
   cgaaggagaa gtccacgcct acatgggggt agagtcataa tcgtgcatca ggatagggcg 32760
   gtggtgctgc agcagcgcgc gaataaactg ctgccgccgc cgctccgtcc tgcaggaata 32820
   caacatggca gtggtctcct cagcgatgat tcgcaccgcc cgcagcataa ggcgccttgt 32880
   cctccgggca cagcagcgca ccctgatctc acttaaatca gcacagtaac tgcagcacag 32940
   caccacaata ttgttcaaaa tcccacagtg caaggcgctg tatccaaagc tcatggcggg 33000
   gaccacagaa cccacgtggc catcatacca caagcgcagg tagattaagt ggcgacccct 33060
   cataaacacg ctggacataa acattacctc ttttggcatg ttgtaattca ccacctcccg 33120
   gtaccatata aacctctgat taaacatggc gccatccacc accatcctaa accagctggc 33180
   caaaacctgc ccgccggcta tacactgcag ggaaccggga ctggaacaat gacagtggag 33240
   agcccaggac tcgtaaccat ggatcatcat gctcgtcatg atatcaatgt tggcacaaca 33300
   caggcacacg tgcatacact tcctcaggat tacaagctcc tcccgcgtta gaaccatatc 33360
   ccagggaaca acccattcct gaatcagcgt aaatcccaca ctgcagggaa gacctcgcac 33420
   gtaactcacg ttgtgcattg tcaaagtgtt acattcgggc agcagcggat gatcctccag 33480
   tatggtagcg cgggtttctg tctcaaaagg aggtagacga tccctactgt acggagtgcg 33540
   ccgagacaac cgagatcgtg ttggtcgtag tgtcatgcca aatggaacgc cggacgtagt 33600
   catatttcct gaagcaaaac caggtgcggg cgtgacaaac agatctgcgt ctccggtctc 33660
   gccgcttaga tcgctctgtg tagtagttgt agtatatcca ctctctcaaa gcatccaggc 33720
   gccccctggc ttcgggttct atgtaaactc cttcatgcgc cgctgccctg ataacatcca 33780
   ccaccgcaga ataagccaca cccagccaac ctacacattc gttctgcgag tcacacacgg 33840
   gaggagcggg aagagctgga agaaccatgt tttttttttt attccaaaag attatccaaa 33900
   acctcaaaat gaagatctat taagtgaacg cgctcccctc cggtggcgtg gtcaaactct 33960
   acagccaaag aacagataat ggcatttgta agatgttgca caatggcttc caaaaggcaa 34020
   acggccctca cgtccaagtg gacgtaaagg ctaaaccctt cagggtgaat ctcctctata 34080
   aacattccag caccttcaac catgcccaaa taattctcat ctcgccacct tctcaatata 34140
   tctctaagca aatcccgaat attaagtccg gccattgtaa aaatctgctc cagagcgccc 34200
   tccaccttca gcctcaagca gcgaatcatg attgcaaaaa ttcaggttcc tcacagacct 34260
   gtataagatt caaaagcgga acattaacaa aaataccgcg atcccgtagg tcccttcgca 34320
   gggccagctg aacataatcg tgcaggtctg cacggaccag cgcggccact tccccgccag 34380
   gaaccatgac aaaagaaccc acactgatta tgacacgcat actcggagct atgctaacca 34440
   gcgtagcccc gatgtaagct tgttgcatgg gcggcgatat aaaatgcaag gtgctgctca 34500
   aaaaatcagg caaagcctcg cgcaaaaaag aaagcacatc gtagtcatgc tcatgcagat 34560
   aaaggcaggt aagctccgga accaccacag aaaaagacac catttttctc tcaaacatgt 34620
   ctgcgggttt ctgcataaac acaaaataaa ataacaaaaa aacatttaaa cattagaagc 34680
   ctgtcttaca acaggaaaaa caacccttat aagcataaga cggactacgg ccatgccggc 34740
   gtgaccgtaa aaaaactggt caccgtgatt aaaaagcacc accgacagct cctcggtcat 34800
   gtccggagtc ataatgtaag actcggtaaa cacatcaggt tgattcacat cggtcagtgc 34860
   taaaaagcga ccgaaatagc ccgggggaat acatacccgc aggcgtagag acaacattac 34920
   agcccccata ggaggtataa caaaattaat aggagagaaa aacacataaa cacctgaaaa 34980
   accctcctgc ctaggcaaaa tagcaccctc ccgctccaga acaacataca gcgcttccac 35040
   agcggcagcc ataacagtca gccttaccag taaaaaagaa aacctattaa aaaaacacca 35100
   ctcgacacgg caccagctca atcagtcaca gtgtaaaaaa gggccaagtg cagagcgagt 35160
   atatatagga ctaaaaaatg acgtaacggt taaagtccac aaaaaacacc cagaaaaccg 35220
   cacgcgaacc tacgcccaga aacgaaagcc aaaaaaccca caacttcctc aaatcgtcac 35280
   ttccgttttc ccacgttacg tcacttccca ttttaagaaa actacaattc ccaacacata 35340
   caagttactc cgccctaaaa cctacgtcac ccgccccgtt cccacgcccc gcgccacgtc 35400
   acaaactcca ccccctcatt atcatattgg cttcaatcca aaataaggta tattattgat 35460
   gatgttaatt aaggatccnn ncggtgtgaa ataccgcaca gatgcgtaag gagaaaatac 35520
   cgcatcaggc gctcttccgc ttcctcgctc actgactcgc tgcgctcggt cgttcggctg 35580
   cggcgagcgg tatcagctca ctcaaaggcg gtaatacggt tatccacaga atcaggggat 35640
   aacgcaggaa agaacatgtg agcaaaaggc cagcaaaagg ccaggaaccg taaaaaggcc 35700
   gcgttgctgg cgtttttcca taggctccgc ccccctgacg agcatcacaa aaatcgacgc 35760
   tcaagtcaga ggtggcgaaa cccgacagga ctataaagat accaggcgtt tccccctgga 35820
   agctccctcg tgcgctctcc tgttccgacc ctgccgctta ccggatacct gtccgccttt 35880
   ctcccttcgg gaagcgtggc gctttctcat agctcacgct gtaggtatct cagttcggtg 35940
   taggtcgttc gctccaagct gggctgtgtg cacgaacccc ccgttcagcc cgaccgctgc 36000
   gccttatccg gtaactatcg tcttgagtcc aacccggtaa gacacgactt atcgccactg 36060
   gcagcagcca ctggtaacag gattagcaga gcgaggtatg taggcggtgc tacagagttc 36120
   ttgaagtggt ggcctaacta cggctacact agaaggacag tatttggtat ctgcgctctg 36180
   ctgaagccag ttaccttcgg aaaaagagtt ggtagctctt gatccggcaa acaaaccacc 36240
   gctggtagcg gtggtttttt tgtttgcaag cagcagatta cgcgcagaaa aaaaggatct 36300
   caagaagatc ctttgatctt ttctacgggg tctgacgctc agtggaacga aaactcacgt 36360
   taagggattt tggtcatgag attatcaaaa aggatcttca cctagatcct tttaaattaa 36420
   aaatgaagtt ttaaatcaat ctaaagtata tatgagtaaa cttggtctga cagttaccaa 36480
   tgcttaatca gtgaggcacc tatctcagcg atctgtctat ttcgttcatc catagttgcc 36540
   tgactccccg tcgtgtagat aactacgata cgggagggct taccatctgg ccccagtgct 36600
   gcaatgatac cgcgagaccc acgctcaccg gctccagatt tatcagcaat aaaccagcca 36660
   gccggaaggg ccgagcgcag aagtggtcct gcaactttat ccgcctccat ccagtctatt 36720
   aattgttgcc gggaagctag agtaagtagt tcgccagtta atagtttgcg caacgttgtt   36780
   gnnnnnnaaa aaggatcttc acctagatcc ttttcacgta gaaagccagt ccgcagaaac   36840
   ggtgctgacc ccggatgaat gtcagctact gggctatctg gacaagggaa aacgcaagcg   36900
   caaagagaaa gcaggtagct tgcagtgggc ttacatggcg atagctagac tgggcggttt   36960
   tatggacagc aagcgaaccg gaattgccag ctggggcgcc ctctggtaag gttgggaagc   37020
   cctgcaaagt aaactggatg gctttctcgc cgccaaggat ctgatggcgc aggggatcaa   37080
   gctctgatca agagacagga tgaggatcgt ttcgcatgat tgaacaagat ggattgcacg   37140
   caggttctcc ggccgcttgg gtggagaggc tattcggcta tgactgggca caacagacaa   37200
   tcggctgctc tgatgccgcc gtgttccggc tgtcagcgca ggggcgcccg gttctttttg   37260
   tcaagaccga cctgtccggt gccctgaatg aactgcaaga cgaggcagcg cggctatcgt   37320
   ggctggccac gacgggcgtt ccttgcgcag ctgtgctcga cgttgtcact gaagcgggaa   37380
   gggactggct gctattgggc gaagtgccgg ggcaggatct cctgtcatct caccttgctc   37440
   ctgccgagaa agtatccatc atggctgatg caatgcggcg gctgcatacg cttgatccgg   37500
   ctacctgccc attcgaccac caagcgaaac atcgcatcga gcgagcacgt actcggatgg   37560
   aagccggtct tgtcgatcag gatgatctgg acgaagagca tcaggggctc gcgccagccg   37620
   aactgttcgc caggctcaag gcgagcatgc ccgacggcga ggatctcgtc gtgacccatg   37680
   gcgatgcctg cttgccgaat atcatggtgg aaaatggccg cttttctgga ttcatcgact   37740
   gtggccggct gggtgtggcg gaccgctatc aggacatagc gttggctacc cgtgatattg   37800
   ctgaagagct tggcggcgaa tgggctgacc gcttcctcgt gctttacggt atcgccgctc   37860
   ccgattcgca gcgcatcgcc ttctatcgcc ttcttgacga gttcttctga attttgttaa   37920
   aatttttgtt aaatcagctc attttttaac caataggccg aaatcggcaa catcccttat   37980
   aaatcaaaag aatagaccgc gatagggttg agtgttgttc cagtttggaa caagagtcca   38040
   ctattaaaga acgtggactc caacgtcaaa gggcgaaaaa ccgtctatca gggcgatggc   38100
   ccactacgtg aaccatcacc caaatcaagt tttttgcggt cgaggtgccg taaagctcta   38160
   aatcggaacc ctaaagggag cccccgattt agagcttgac ggggaaagcc ggcgaacgtg   38220
   gcgagaaagg aagggaagaa agcgaaagga gcgggcgcta gggcgctggc aagtgtagcg   38280
   gtcacgctgc gcgtaaccac cacacccgcg cgcttaatgc gccgnnnnnn nnnnnnnnnn   38340
   nnnnnnnnnn nnnnnnnnnn nttaat                                        38366